# EUROPEAN PATENT APPLICATION

(11) **EP 1 916 239 A1**
(43) Date of publication of application: **30.04.2008**
(21) Application number: 06782591.9
(22) Date of filing: 03.08.2006
(51) Int. Cl.: C07D 213/64, A61P 1/16

(54) **PYRIDONE COMPOUND**

(30) Priority: 10.08.2005 JP 2005231995
(71) Applicant: BANYU PHARMACEUTICAL CO., LTD., Tokyo 102-8667 (JP)
(72) Inventor: ANDO, Makoto, Tsukuba-shi Ibaraki 300-2611 (JP); SEKINO, Etsuko, Tsukuba-shi Ibaraki 300-2611 (JP); HAGA, Yuji, Tsukuba-shi Ibaraki 300-2611 (JP); OTAKE, Norikazu, Tsukuba-shi Ibaraki 300-2611 (JP); MORIYA, Minoru, Tsukuba-shi Ibaraki 300-2611 (JP)
(74) Representative: Buchan, Gavin MacNicol
(86) International application number: PCT/JP2006/315775
(87) International publication number: WO 2007/018248

(57) **Abstract**

An active ingredient is a compound represented by the general formula [I]: 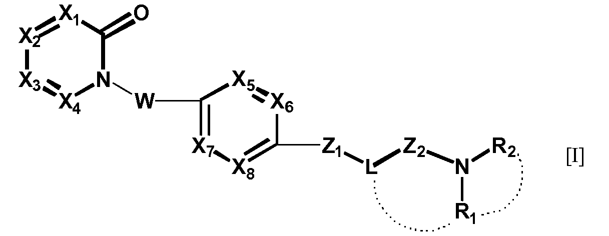 [wherein R₁ and R₂ represent a lower alkyl group, a C₃₋₆ cycloalkyl group or the like, X₁ and X₂ represent methine, an Ar-Y₁-Y₂-Y₃-substituted methine or the like, however either of them is an Ar-Y₁-Y₂-Y₃-substituted methine, X₃ to X₈ represent methine, -N- or the like, Y₁ and Y₃ represent a single bond, -O- or the like, Y₂ represent a single bond, a lower alkylene group or the like, W represent -(O)-(CH₂)n-(O)- or the like, n represents an integer of 1 to 4, L and Z₂ represent a single bond or a methylene group, Z₁ represents a single bond, a C₁₋₄ alkylene group or the like, and Ar represents an aromatic carbocyclic group or the like]. The compound acts as a melanin-concentrating hormone receptor antagonist and useful as a therapeutic agent for obesity or the like.

## Description

### TECHNICAL FIELD

The present invention relates to a novel pyridone compound. The compound acts as a melanin concentrating hormone receptor antagonist, and is useful as a preventive, treating or remedial agent for various circular system diseases, nervous system diseases, metabolic diseases, genital diseases, respiratory diseases, digestive diseases, etc.

### BACKGROUND ART

Melanin concentrating hormone (hereafter referred to as "MCH") is a cyclic peptide hormone/neuro-peptide, which was for the first time isolated by Kawauchi, et al., in 1983 from sermon hypophysis. [Nature, Vol. 305, 321 (1983)]. The hormone is known to functionally antagonize for melanin cell stimulating hormone in fishes, to cause concentration of melanin granules in melanophore and participate in body color change [International Review of Cytology, Vol. 126, 1 (1991); Trends in Endocrinology and Metabolism, Vol. 5, 120 (1994)]. Also in mammals, MCH-containing neuron cells are localized in the hypothalamus lateral field and uncertain zone, but their nerve fibers are projecting over a very wide scope in the brain [see The Journal of Comparative Neurology, Vol. 319, 218 (1992)], and MCH is considered to preside over various central functions in living bodies.

Hypothalamus lateral field is known of old as feeding center, and furthermore, recently molecular biological and pharmacological knowledges suggesting participation of MCH in controlling energetic homeostasis are being much accumulated. That is, it has been reported that expression of mRNA, which is an MCH precursor, is accelerated in the brains of ob/ob mice, db/db mice, A^{y}/a mice, Zucker fatty rats which are model animals of hereditary obesity, and in the brains of fasting mice [see Nature, Vol. 380, 243 (1996); Diabetes, Vol. 47, 294 (1998); Biochemical and Biophysical Research Communications, Vol. 268, 88 (2000); Molecular Brain Research, Vol. 92, 43 (2001)].

Acute ventricular administration of MCH to rats was observed to induce accelerated feeding activity [Nature, Vol. 380, 243 (1996)] and chronic administration invites obesity accompanied by polyphagy [see Proceedings of the National Academy of Sciences of the United States of America, Vol. 99, 3240 (2002)]. Moreover, MCH precursor gene-deficient mice show reduced food ingestion or rise in oxygen consumption per body weight compared to wild type mice. Their low body weight due to decrease in body fat was observed [see Nature, Vol. 396, 670 (1998)].

On the contrary, transgenic mice which express excessive MCH precursor develop obesity accompanied by polyphagy and insulin resistance [The Journal of Clinical Investigation, Vol. 107, 379 (2001)]. Consequently, it is suggested that MCH is an important factor for developing obesity and participates in diseases induced by metabolic disorders or respiratory diseases for which obesity is one risk factor. Besides, MCH is known to participate also in anxiety-causing action, epilepsy, memory and learning, diuretic action, sodium/potassium excretory action, oxytocin secreting action, reproduction and reproductive function [see Peptides, Vol. 17, 171 (1996); Peptides, Vol. 18, 1095 (1997); Peptides, Vol. 15, 757 (1994); Journal of Neuroendocrinology, Vol. 8, 57 (1996); Critical Reviews in Neurobiology, Vol. 8, 221 (1994)].

MCH causes versatile pharmacological actions through MCH receptors which are present mainly in the central nervous system. As receptors of MCH, at least two types of type 1 receptors (MCH-1R or SLC-1) and type 2 receptors (MCH-2R or SLT) are known [see Nature, Vol. 400, 261 (1999); Nature, Vol. 400, 265 (1999); Biochemical and Biophysical Research Communications, Vol. 261, 622 (1999); Nature Cell Biology, Vol. 1, 267 (1999); FEBS Letters, Vol. 457, 522 (1999); Biochemical and Biophysical Research Communications, Vol. 283, 1013 (2001); The Journal of Biological Chemistry, Vol. 276, 20125 (2001); Proceedings of the National Academy of Sciences of the United States of America, Vol. 98, 7564 (2001); Proceedings of the National Academy of Sciences of the United States of America, Vol. 98, 7576 (2001); The Journal of Biological Chemistry, Vol. 276, 34664 (2001); Molecular Pharmacology, Vol. 60, 632 (2001)].

Of those, the pharmacological action observed on rodents is induced mainly via MCH-1R [see Genomics, Vol. 79, 785 (2002)]. Because MCH-1R gene-deficient mice chronically administered with MCH do not develop polyphagy or obesity, it is known that controlling of energy metabolism by MCH is induced via MCH-1R. Furthermore, the deficiency of MCH-1R is known to promote the activity amount of mice [see Proceedings of the National Academy of Sciences of the United States of America, Vol. 99, 3240 (2002)], and its participation in central diseases accompanied by behavioral disorders, for example, attention-deficit hyperactivity disorder, schizophrenia, depression and the like also is strongly suggested [see Molecular Medicine Today, Vol. 6, 43 (2000); Trends in Neuroscience, Vol. 24, 527 (2001)].

It is also reported that an autoantibody to MCH-1R is present in serum of vitiligo vulgaris patients [see The Journal of Clinical Investigation, Vol. 109, 923 (2002)]. Furthermore, expression of MCH-1R in certain species of cancer cells was reported, and in vivo expression sites of MCH and MCH-1R also suggest MCH's participation in cancer, sleep and vigil, drug dependence and digestive disorders [see Biochemical and Biophysical Research Communications, Vol. 289, 44 (2001); Neuroendocrinology, Vol. 61, 348 (1995); Endocrinology, Vol. 137, 561 (1996); The Journal of Comparative Neurology, Vol. 435, 26 (2001)].

Functions of MCH are expressed upon it binding to MCH receptors. Therefore, when its binding to MCH receptor is inhibited, then expression of MCH action can be inhibited. In consequence, substances which are antagonists for binding of MCH with its receptor are useful as preventive, treating or remedial agents for those various diseases in which MCH participates, for example, metabolic disorders such as obesity, diabetes, hormone disorder, hyperlipidemia, gout, fatty liver; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation et al.

As compounds having an MCH receptor antagonistic effect, for example, various compounds are disclosed in International Patent Publication WO01/21577, WO01/82925, WO02/06245, WO02/02744. However, they do not have a pyridone ring.

WO02/81454 (Patent Reference 1) discloses a pyridone derivative having an anti-hyperglycemia activity. However, concrete compounds in the reference have a single bond in the site of -Y₁-Y₂-Y₃- in the formula of the present invention, and in addition, they have a single bond also in the sites of Z₁, L and Z₂; but in the present invention, Y₁, Y₂, Y₃, Z₁, L and Z₂ are not all single bonds at the same time, and therefore the present invention differs from Patent Reference 1. Further, the reference does not describe an MCH receptor antagonistic effect.

WO03/68230 (Patent Reference 2) discloses a pyridone derivative having a P38MAP kinase activity. However, concrete compounds disclosed in the reference have a methylene group as the -W- moiety in the formula of the present invention, therefore differing from the present invention in which the main chain of the linker has at least 2 atoms; and further, the reference does not describe an MCH receptor antagonistic effect.
Patent Reference 1: International Patent Publication WO02/81454
Patent Reference 2: International Patent Publication WO03/68230

### DISCLOSURE OF THE INVENTION

The present inventors have assiduously studied compounds having an MCH receptor antagonistic effect, and as a result, have found that a pyridone compound, in which an aromatic 6-membered ring bonds to the N atom of the pyridone ring via a linker having a main chain with at least 2 atoms and a specific amino group bonds to para-position of the aromatic 6-membered ring via linker, has an MCH receptor antagonistic effect and is effective for prevention or remedy of various MCH receptor-associated diseases, and have completed the present invention.

Specifically, the invention provides:
(1) a pyridone compound of a formula [I] or a pharmaceutically-acceptable salt thereof: 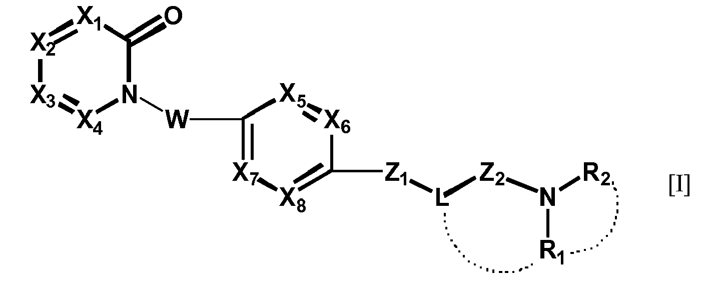 [wherein:
   R₁ and R₂ are the same or different, each representing a hydrogen atom, a lower alkyl group optionally having substituent(s), or a lower cycloalkyl group optionally having substituent(s), and R₁ and R₂, taken together with the nitrogen atom to which they bond, may form a 4- to 11-membered, crosslinked, non-crosslinked or spiro-cyclic aliphatic nitrogen-containing hetero ring optionally having substituent(s);
   X₁ and X₂ each represent a methine optionally having substituent(s) selected from a group α, a methine substituted with Ar-Y₁-Y₂-Y₃-, or a nitrogen atom;
   any one of X₁ and X₂ is a methine substituted with Ar-Y₁-Y₂-Y₃-, and the other is a methine optionally having substituent(s) selected from the group consisting of a α or a nitrogen atom;
   substituent selected from the group consisting of the group α: a halogen atom, a lower alkyl group optionally substituted with halogen atom(s), and a lower alkyloxy group optionally substituted with halogen atom(s);
   X₃ and X₄ are the same or different, each representing a methine optionally having substituent(s) or a nitrogen atom;
   X₅, X₆, X₇ and X₈ are the same or different, each representing a methine optionally having substituent(s) or a nitrogen atom, however, at least 3 atoms of X₅, X₆, X₇ and X₈ are not nitrogen atoms at the same time;
   Y₁ represents a single bond, -O-, -NR-, -S-, -SO- or -SO₂-;
   Y₂ represents a single bond, a lower alkylene group optionally having substituent(s), optionally a lower alkenylene group, or a lower cycloalkylene group optionally having substituent(s);
   Y₃ represents a single bond, -O-, -NR-, -S-, -SO- or -SO₂-;
   R represents a hydrogen atom, or a lower alkyl group optionally having substituent(s);
   W represents -(O)m₁-(CH₂)n-(O)m₂-, in which -(CH₂)n- in W may optionally have substituent(s);
   m₁ and m₂ are the same or different, each indicating 0 or 1; n indicates an integer of from 1 to 4; and they satisfy 2 ≤ m₁+m₂+n ≤ 4, but m₁, m₂ and n are not 1 at the same time;
   L represents a single bond or a methylene group optionally having substituent(s), and L, taken together with Z₂ and R₁ and with the nitrogen atom adjacent to R₁, may form an aliphatic nitrogen-containing hetero ring optionally having substituent(s);
   Z₁ represents a single bond, -O-, or a C₁₋₄ alkylene group optionally having substituent(s);
   Z₂ represents a single bond or a C₁₋₄ alkylene group optionally having substituent(s);
   however, Y₁, Y₂, Y₃,Z₁, L and Z₂ are not all single bonds at the same time;
   Ar represents an aromatic carbocyclic group optionally having substituent(s), an aromatic heterocyclic group optionally having substituent(s), or an aliphatic carbocyclic group optionally having substituent(s)].
   The invention further provides:
(2) a melanin concentrating hormone receptor antagonist comprising a compound of (1) or a pharmaceutically-acceptable salt thereof as the active ingredient;
(3) a pharmaceutical composition comprising a pharmaceutically-acceptable additive and a compound of (1) or a pharmaceutically-acceptable salt thereof,
(4) a preventive, treating or remedial agent comprising a compound of (1) or a pharmaceutically-acceptable salt thereof as the active ingredient, for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; digestive disorders; respiratory disorders; cancer or pigmentation.

The invention is described in more detail hereinunder.

In this description, the term "lower" means that the number of the carbon atoms constituting the group or the compound with the term is at most 6, preferably at most 4.

"Lower alkyl group" includes a linear alkyl group having from 1 to 6 carbon atoms or a branched alkyl group having from 3 to 6 carbon atoms, concretely, for example, a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-amyl group, a 1-methylbutyl group, a 2-methylbutyl group, a 1,2-dimethylpropyl group, a 1-ethylpropyl group, an n-hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1-ethylbutyl group, a 1,1,2-trimethylpropyl group, a 1,2,2-trimethylpropyl group, a 1-ethyl-2-methylpropyl group, a 1-ethyl-1-methylpropyl group et al.

"Lower cycloalkyl group" includes a cycloalkyl group having from 3 to 6 carbon atoms, concretely, for example, a cyclopropyl group, a cyclobutyl group, a cyclopentyl group, a cyclohexyl group.

"Lower alkylene group" includes a linear alkylene group having from 1 to 6 carbon atoms or a branched alkylene group having from 3 to 6 carbon atoms, concretely, for example, a methylene group, an ethylene group, a propylene group, a butylene group, a pentylene group, a hexylene group et al.

"Lower alkenylene group" includes a linear alkenylene group having from 2 to 6 carbon atoms or a branched alkenylene group having from 3 to 6 carbon atoms, having one carbon-carbon double bond in the chain, concretely, for example, a vinylene group, a 1-propenylene group, a 2-propenylene group, a 1-butenylene group, a 2-butenylene group, a 3-butenylene group, a 2-pentenylene group, a 3-pentenylene group, a 4-pentenylene group, a 1-hexenylene group, a 2-hexenylene group, a 3-hexenylene group, a 4-hexenylene group, a 5-hexenylene group et al.

"Lower cycloalkylene group" includes a cycloalkylene group having from 3 to 6 carbon atoms, concretely, for example, a 1,1-cyclopropylene group, a 1,2-cyclopropylene group, a 1,1-cyclobutanylene group, a 1,2-cyclobutanylene group, a 1,3-cyclobutanylene group, a 1,1-cyclopentenylene group, a 1,2-cyclohexenylene group, a 1,3-cyclohexenylene group, a 1,4-cyclohexenylene group et al.

Examples of the substituent in "methine optionally having substituent(s)" may be those selected from the group consisting of the group α.

### Group α:

A halogen atom, lower alkyl group optionally substituted with a halogen atom, and a lower alkyloxy group optionally substituted with a halogen atom.

Examples of the substituent in "lower alkyl group optionally having substituent(s)", "lower cycloalkyl group optionally having substituent(s)", "lower alkylene group optionally having substituent(s)", " C₁₋₄ alkylene optionally having substituent(s)", "lower alkenylene group optionally having substituent(s)" and "methylene optionally having substituent(s)" may be those selected from the group consisting of a group β; and the above-mentioned lower alkyl group and others may be substituted with one or more such substituents.

### Group β:

A halogen atom, a cyano group, a hydroxyl group, an amino group, a lower alkyl group optionally substituted with a fluorine atom or a hydroxyl group, a mono-lower alkylamino group, a di-lower alkylamino group, a lower alkyloxy group optionally substituted with a fluorine atom, a lower alkyloxy-lower alkyl group, a lower alkyloxycarbonyl group, a lower alkyloxycarbonylamino group, a lower alkyloxycarbonyl(lower alkyl)amino group, a lower alkylcarbonyl group, a lower alkylcarbonyloxy group, a lower alkylcarbonylamino group, a lower alkylcarbonyl(lower alkyl)amino group, a carbamoyl group, a mono-lower alkylcarbamoyl group, a di-lower alkylcarbamoyl group, a carbamoylamino group, a mono-lower alkylcarbamoylamino group, a di-lower alkylcarbamoylamino group, a mono-lower alkylcarbamoyl(lower alkyl)amino group, a di-lower alkylcarbamoyl(lower alkyl)amino group, a carbamoyloxy group, a mono-lower alkylcarbamoyloxy group, a di-lower alkylcarbamoyloxy group, a lower alkylsulfonyl group, a lower alkylsulfonylamino group, a lower alkylsulfonyl(lower alkyl)amino group, a sulfamoyl group, a mono-lower alkylsulfamoyl group, a di-lower alkylsulfamoyl group, a sulfamoylamino group, a mono-lower alkylsulfamoylamino group, a di-lower alkylsulfamoylamino group, a mono-lower alkylsulfamoyl(lower alkyl)amino group, and a di-lower alkylsulfamoyl(lower alkyl)amino group.

"Aliphatic nitrogen-containing heterocyclic group" includes a 3- to 7-membered monocyclic, or 5 to 12-membered polycyclic, saturated or partially-unsaturated heterocyclic group, containing at least one, preferably from 1 to 3 nitrogen atoms as a part of the ring-constitutive members, and optionally containing from 0 to 2 oxygen atoms or from 0 to 2 sulfur atoms; and concretely, for example, it includes an aziridinyl group, an azetidinyl group, a pyrrolidinyl group, a piperidinyl group, a homopiperazinyl group, a homopiperidinyl group, a morpholinyl group, a thiomorpholinyl group, an octahydocyclopenta[b]pyrrolyl group, a hexahydropyrrolidinyl group, an octahydroindolidinyl group, an octahydroquinolidinyl group, an octahydropyrido[2.1-c]oxazinyl group, a 2,5,6,7-tetrahydro-5H-pyrrolo[1.2-a]imidazolyl group et al.

"Aromatic carbocyclic group" includes a monocyclic or polycyclic aromatic carbocyclic group having from 6 to 14 carbon atoms, preferably from 6 to 10 carbon atoms, concretely, for example, a phenyl group, a naphthyl group, a phenanthryl group et al.

"Aromatic heterocyclic group" includes a 5- or 6-membered monocyclic or 8- to 14-membered polycyclic heteroaromatic cyclic group containing at least one, preferably from 1 to 5 hetero atoms selected from a nitrogen atom, an oxygen atom and a sulfur atom as a part of the ring-constitutive members; and concretely, for example, it includes a pyridinyl group, a pyrimidinyl group, a pyridazinyl group, a pyrazyl group, a pyrazolyl group, a pyrrolyl group, an imidazolyl group, a triazolyl group, an oxazolyl group, an isoxazolyl group, an oxadiazolyl group, a thiazolyl group, an isothiazolyl group, a thiadiazolyl group, a tetrazolyl group, a pyridazinyl group, a pyrazinyl group, a furyl group, a thienyl group, an indolyl group, a benzofuranyl group, a benzothienyl group, a benzimidazolyl group, a benzoxazolyl group, a benzisoxazolyl group, a benzothiazolyl group, a benzisothiazolyl group, an indazolyl group, a purinyl group, a quinolyl group, an isoquinolyl group, a phthalazinyl group, a naphthyridinyl group, a quinoxalinyl group, a quinazolinyl group, a cinnolinyl group, a pteridinyl group, a pyrido[3,2-b]pyridyl group et al.

"Aliphatic carbocyclic group" includes a monocyclic or polycyclic, saturated or partially-unsaturated carbocyclic group having from 3 to 10, preferably from 3 to 8 carbon atoms, concretely, for example, a cyclopropyl group, a cyclobutenyl group, a cyclopentyl group, a cyclohexyl group, a cycloheptyl group, a cyclooctyl group, a cyclononyl group, a cyclodecyl group, a bicyclohexyl group, an adamantyl group et al.

As to "4- to 11-membered, crosslinked, non-crosslinked or spiro-cyclic aliphatic nitrogen-containing hetero ring", examples of the crosslinked aliphatic nitrogen-containing hetero ring include 2,5-diazabicyclo[2.2.1]heptane, 2,5-diazabicyclo[2.2.2]octane, octahydropyrrolo[3.4-b]pyrrole, octahydropyrrolo[3.4-c]pyrrole, 3-azabicyclo[3.1.0]hexane, decahydropyrrolo[3.4-d]azepine; examples of the non-crosslinked aliphatic nitrogen-containing hetero ring include an azetidine ring, a pyrrolidine ring, a piperidine ring, a hexamethyleneimine ring, a heptamethyleneimine ring, a morpholine ring; and further, the spiro-cyclic aliphatic nitrogen-containing hetero ring includes 2-azaspiro[4.4]nonane, 1-oxa-7-azaspiro[4.4]nonane, 2-oxa-7-azaspiro[4.4]nonane, 1,7-diazaspiro[4.4]nonane, 3-oxa-1,7-diazaspiro[4.4]nonane, 2,7-diazaspiro[4.4]nonane, 2,7-diazaspiro[3.5]nonane, 2-azaspiro[3.3]heptane, 2-oxa-6-azaspiro[3.3]heptane, 2,8-diazaspiro[4.5]decane et al.

Examples of the substituent in "aromatic heterocyclic group optionally having substituent(s)" or "aromatic carbocyclic group optionally having substituent(s)" may be those selected from the group consisting of the group β; and the above-mentioned cyclic groups may be substituted with one or more such substituents.

The substituent in "aliphatic nitrogen-containing heterocyclic group optionally having substituent(s)" or "aliphatic carbocyclic group optionally having substituent(s)" includes, in addition to the substituents selected from the group consisting of the group β, an oxo group and a lower cycloalkyl group; and the above-mentioned cyclic groups may be substituted with one or more such substituents.

The substituent in "lower alkyl group optionally having substituent(s)" defined for R is, for example, preferably a halogen atom, a lower alkoxy group, a lower haloalkoxy group.

In the definition of the above-mentioned substituents, "halogen atom" includes a fluorine atom, a chlorine atom, a bromine atom and an iodine atom.

"Oxo group" means a group (=O) that forms a carbonyl group (C=O) along with the carbon atom in an organic compound.

"Lower alkyl group optionally substituted with a fluorine atom or a hydroxyl group" includes a lower alkyl group, or a lower alkyl group in which a part or all of the hydrogen atoms of the lower alkyl group are substituted with a fluorine atom or a hydroxyl group; The latter lower alkyl group substituted with a fluorine atom or a hydroxyl group includes, for example, a fluoromethyl group, a difluoromethyl group, a trifluoromethyl group, a 2-fluoroethyl group, a 1,2-difluoroethyl group, a 2-hydroxyethyl group, a 1,2-dihydroxyethyl group et al.

"Lower alkyloxy group optionally substituted with a fluorine atom" includes a group comprising a lower alkyl group or a lower alkyl group substituted with a fluorine atom, bonding to an oxygen atom. Concretely, the lower alkyloxy group includes a methoxy group, an ethoxy group, an n-propyloxy group, an isopropyloxy group, an n-butoxy group, an isobutoxy group, a tert-butoxy group, an n-pentyloxy group et al; and the lower alkyloxy group substituted with a fluorine atom includes, for example, a fluoromethoxy group, a difluoromethoxy group, a trifluoromethoxy group, a 1,2-difluoroethoxy group et al.

"Mono-lower alkylamino group" is an amino group (-NH₂) in which one hydrogen atom is substituted with a lower alkyl group, concretely, for example, including a methylamino group, an ethylamino group, an n-propylamino group, an isopropylamino group, an n-butylamino group, a sec-butylamino group, a tert-butylamino group et al.

"Di-lower alkylamino group" is an amino group (-NH₂) in which two hydrogen atoms are substituted with lower alkyl groups, concretely, for example, including a dimethylamino group, a diethylamino group, an ethylmethylamino group, a di(n-propyl)amino group, a methyl(n-propyl)amino group, a diisopropylamino group et al.

"Lower alkyloxy-lower alkyl group" is a lower alkyl group substituted with a lower alkyloxy group, and concretely includes, for example, a methoxymethyl group, an ethoxymethyl group, an n-propyloxymethyl group, an isopropyloxymethyl group, a 1-methoxyethyl group, a 2-methoxyethyl group et al.

"Lower alkyloxycarbonyl group" is a lower alkyloxy group bonding to a carbonyl group (-CO-) and includes an alkyloxycarbonyl group having from 1 to 6 carbon atoms, concretely, for example, a methoxycarbonyl group, an ethoxycarbonyl group, an n-propyloxycarbonyl group, an isopropyloxycarbonyl group, an n-butoxycarbonyl group, an isobutoxycarbonyl group, a tert-butoxycarbonyl group, an n-pentyloxycarbonyl group et al.

"Lower alkyloxycarbonylamino group" is a group of an amino group (-NH₂) to which a lower alkyloxycarbonyl group bonds, and includes an alkyloxycarbonylamino group having from 1 to 6 carbon atoms, concretely, for example, a methoxycarbonylamino group, an ethoxycarbonylamino group, an n-propyloxycarbonylamino group, an isopropyloxycarbonylamino group, an n-butoxycarbonylamino group, an isobutoxycarbonylamino group, a tert-butoxycarbonylamino group, an n-pentyloxycarbonylamino group et al.

"Lower alkyloxycarbonyl(lower alkyl)amino group" is a group of a mono-lower alkylamino group in which the hydrogen atom on the nitrogen atom is substituted with a lower alkyloxycarbonyl group and concretely includes, for example, a methoxycarbonyl(methyl)amino group, an ethoxycarbonyl(methyl)amino group, an n-propyloxycarbonyl(methyl)amino group et al.

"Lower alkylcarbonyl group" is a group of a carbonyl group (-CO-) bonding to a lower alkyl group, and includes an alkylcarbonyl group having from 1 to 6 carbon atoms, concretely, for example, an acetyl group, a propionyl group, a butyryl group, an isobutyryl group, a valeryl group, an isovaleryl group, a pivaloyl group et al.

"Lower alkylcarbonyloxy group" is a lower alkylcarbonyl group bonding to an oxygen atom, and concretely includes, for example, an acetoxy group, a propionyloxy group, a valeryloxy group, an isovaleryloxy group, a pivaloyloxy group et al.

"Lower alkylcarbonylamino group" is a group of an amino group (-NH₂) in which one hydrogen atom is substituted with a lower alkylcarbonyl group, and concretely includes, for example, an acetamido group, a propionylamino group, an isobutyrylamino group, a valerylamino group, an isovalerylamino group, a pivaloylamino group et al.

"Lower alkylcarbonyl(lower alkyl)amino group" is a mono-lower alkylamino group in which the hydrogen atom on the nitrogen atom is substituted with a lower alkylcarbonyl group, and includes, for example, a methylcarbonyl(methyl)amino group, an ethylcarbonyl(methyl)amino group, an n-propylcarbonyl(methyl)amino group et al.

"Mono-lower alkylcarbamoyl group" is a carbamoyl group (-CONH₂) in which one hydrogen atom is substituted with a lower alkyl group, and concretely includes, for example, a methylcarbamoyl group, an ethylcarbamoyl group, an n-propylcarbamoyl group, an isopropylcarbamoyl group, an n-butylcarbamoyl group a sec-butylcarbamoyl group, a tert-butylcarbamoyl group et al.

"Di-lower alkylcarbamoyl group" is a carbamoyl group (-CONH₂) in which two hydrogen atoms are substituted with lower alkyl groups, and concretely includes, for example, a dimethylcarbamoyl group, a diethylcarbamoyl group, an ethylmethylcarbamoyl group, a di(n-propyl)carbamoyl group, a methyl(n-propyl)carbamoyl group, a diisopropylcarbamoyl group et al.

"Mono-lower alkylcarbamoylamino group" is an amino group (-NH₂) in which one hydrogen atom is substituted with a mono-lower alkylcarbamoyl group, and concretely includes, for example, a methylcarbamoylamino group, an ethylcarbamoylamino group, an n-propylcarbamoylamino group, an isopropylcarbamoylamino group, an n-butylcarbamoylamino group, a sec-butylcarbamoylamino group, a tert-butylcarbamoylamino group et al.

"Di-lower alkylcarbamoylamino group" is an amino group (-NH₂) in which one hydrogen atom is substituted with a di-lower alkylcarbamoyl group, and concretely includes, for example, a dimethylcarbamoylamino group, a diethylcarbamoylamino group, a di(n-propyl)carbamoylamino group, a diisopropylcarbamoylamino group, a di(n-butyl)carbamoylamino group, a di(sec-butyl)carbamoylamino group, a di(tert-butyl)carbamoylamino group et al.

"Mono-lower alkylcarbamoyl(lower alkyl)amino group" is a mono-lower alkylamino group in which the hydrogen atom on the nitrogen atom is substituted with a mono-lower alkylcarbamoyl group, and concretely includes, for example, a monomethylcarbamoyl(methyl)amino group, a monoethylcarbamoyl(methyl)amino group, a [mono(n-propyl)carbamoyl](methyl)amino group et al.

"Di-lower alkylcarbamoyl(lower alkyl)amino group" is a mono-lower alkylamino group in which the hydrogen atom on the nitrogen atom is substituted with a di-lower alkylcarbamoyl group, and concretely includes, for example, a dimethylcarbamoyl(methyl)amino group, a diethylcarbamoyl(methyl)amino group, a [di(n-propyl)carbamoyl](methyl)amino group et al.

"Mono-lower alkylcarbamoyloxy group" is a mono-lower alkylcarbamoyl group bonding to an oxygen atom, and concretely includes, for example, a methylcarbamoyloxy group, an ethylcarbamoyloxy group, an n-propylcarbamoyloxy group, an isopropylcarbamoyloxy group, an n-butylcarbamoyloxy group, a sec-butylcarbamoyloxy group, a tert-butylcarbamoyloxy group et al.

"Di-lower alkylcarbamoyloxy group" is a di-lower alkylcarbamoyl group bonding to an oxygen atom, and concretely includes, for example, a dimethylcarbamoyloxy group, a diethylcarbamoyloxy group, an ethylmethylcarbamoyloxy group, a di(n-propyl)carbamoyloxy group, a methyl(n-propyl)carbamoyloxy group, a diisopropylcarbamoyloxy group et al.

"Lower alkylsulfonyl group" is a lower alkyl group bonding to a sulfonyl group (-SO₂-), and concretely includes, for example, a methylsulfonyl group, an ethylsulfonyl group, an n-propylsulfonyl group, an isopropylsulfonyl group, an n-butylsulfonyl group, a sec-butylsulfonyl group, a tert-butylsulfonyl group et al.

"Lower alkylsulfonylamino group" is an amino group (-NH₂) in which one hydrogen atom is substituted with a lower alkylsulfonyl group, and concretely includes, for example, a methylsulfonylamino group, an ethylsulfonylamino group, an n-propylsulfonylamino group, an isopropylsulfonylamino group, an n-butylsulfonylamino group, a sec-butylsulfonylamino group, a tert-butylsulfonylamino group et al.

"Lower alkylsulfonyl(lower alkyl)amino group" is a group of a mono-lower alkylamino group in which the hydrogen atom on the nitrogen atom is substituted with a lower alkylsulfonyl group, and concretely includes, for example, a methanesulfonyl group, an ethanesulfonyl group, an n-propanesulfonyl group, an isopropanesulfonyl group et al.

"Mono-lower alkylsulfamoyl group" is a group of a sulfamoyl group (-SO₂NH₂) in which one hydrogen atom is substituted with a lower alkyl group, and concretely includes, for example, a monomethylsulfamoyl group, a monoethylsulfamoyl group, a mono(n-propyl)sulfamoyl group, a monoisopropylsulfamoyl group, a mono(n-butyl)sulfamoyl group, a mono(sec-butyl)sulfamoyl group, a mono(tert-butyl)sulfamoyl group et al.

"Di-lower alkylsulfamoyl group" is a group of a sulfamoyl group (-SO₂NH₂) in which two hydrogen atoms are substituted with lower alkyl groups, and concretely includes, for example, a dimethylsulfamoyl group, a diethylsulfamoyl group, a di(n-propyl)sulfamoyl group, a diisopropylsulfamoyl group, a di(n-butyl)sulfamoyl group, a di(sec-butyl)sulfamoyl group, a di(tert-butyl)sulfamoyl group et al.

"Mono-lower alkylsulfamoylamino group" is a group of an amino group (-NH₂) in which one hydrogen atom is substituted with a mono-lower alkylsulfamoyl group, and concretely includes, for example, a (monomethylsulfamoyl)amino group, a (monoethylsulfamoyl)amino group, a [mono(n-propyl)sulfamoyl]amino group, a (monoisopropylsulfamoyl)amino group, a [mono(n-butyl)sulfamoyl]amino group, a [(mono-sec-butyl)sulfamoyl]amino group, a [(mono-tert-butyl)sulfamoyl] amino group et al.

"(Di-lower alkylsulfamoyl)amino group" is a group of an amino group (-NH₂) in which one hydrogen atom is substituted with a di-lower alkylsulfamoyl group, and concretely includes, for example, a (dimethylsulfamoyl)amino group, a (diethylsulfamoyl)amino group, an (ethylmethylsulfamoyl)amino group, a [di(n-propyl)sulfamoyl]amino group, a [methyl(n-propyl)sulfamoyl]amino group, a (diisopropylsulfamoyl)amino group et al.

"Mono-lower alkylsulfamoyl(lower alkyl)amino group" is a group of a mono-lower alkylamino group in which the hydrogen atom on the nitrogen atom is substituted with a mono-lower alkylsulfamoyl group, and concretely includes, for example, a monomethylsulfamoyl(methyl)amino group, a monoethylsulfamoyl(methyl)amino group, a [mono(n-propyl)sulfamoyl](methyl)amino group et al.

"Di-lower alkylsulfamoyl(lower alkyl)amino group" is a group of a mono-lower alkylamino group in which the hydrogen atom on the nitrogen atom is substituted with a di-lower alkylsulfamoyl group, and concretely includes, for example, a dimethylsulfamoyl(methyl)amino group, a diethylsulfamoyl(methyl)amino group, a [di(n-propyl)sulfamoyl](methyl)amino group et al.

"Pharmaceutically-acceptable salts" of a pyridone compound of formula [I] mean ordinary salts that are acceptable as medicines. Their examples are acid-addition salts to the amine group of the compound of formula [I] or acid-addition salts to the nitrogen-containing hetero ring thereof, or base-addition salts to the carboxyl group, if any, of the compound of formula [I].

The acid-addition salts include inorganic acid salts such as hydrochlorides, sulfates, nitrates, phosphates, perchlorates; organic acid salts such as maleates, fumarates, tartrates, citrates, ascorbates, trifluoroacetates; and sulfonates such as methanesulfonates, isethionates, benzenesulfonates, p-toluenesulfonates et al.

The base-addition salts include alkali metal salts such as sodium salts, potassium salts; alkaline earth metal salts such as calcium salts, magnesium salts; and organic amine salts such as ammonium salts; trimethylamine salts, triethylamine salts, dicyclohexylamine salts, ethanolamine salts, diethanolamine salts, triethanolamine salts, procaine salts, N,N'-dibenzylethylenediamine salts et al.

For the purpose of more concretely disclosing the pyridone compounds of the invention hereinunder, various symbols used in formula [I] are described in detail with reference to their examples.

In formula [I], X₁ and X₂ each represent a methine optionally having substituent(s) selected from the group consisting of a group α, a methine substituted with Ar-Y₁-Y₂-Y₃-, or a nitrogen atom, and any one of X₁ and X₂ is a methine substituted with Ar-Y₁-Y₂-Y₃-, and the other is a methine optionally having substituent(s) selected from the group consisting of the group α or a nitrogen atom.

Preferred examples of the substituent selected from the group α for X₁ and X₂ include a fluorine atom, a chlorine atom, a methyl group, a methoxy group et al.

X₁ and X₂ concretely include:
X₁ is a methine substituted with Ar-Y₁-Y₂-Y₃-, X₂ is a methine optionally having substituent(s) selected from the group consisting of the group α;
X₁ is a methine substituted with Ar-Y₁-Y₂-Y₃, X₂ is a nitrogen atom;
X₁ is a methine optionally having substituent(s) selected from the group consisting of the group α, X₂ is a methine substituted with Ar-Y₁-Y₂-Y₃;
X₁ is a nitrogen atom, X₂ is a methine substituted with Ar-Y₁-Y₂-Y₃.

Preferably,
X₁ is a methine optionally having substituent(s) selected from the group consisting of the group α,
X₂ is a methine substituted with Ar-Y₁-Y₂-Y₃;
X₁ is a nitrogen atom, X₂ is a methine substituted with Ar-Y₁-Y₂-Y₃.

More preferably,
X₁ is an unsubstituted methine, X₂ is a methine substituted with Ar-Y₁-Y₂-Y₃.

Y₁ represents a single bond, -O-, -NR-, -S-, -SO- or -SO₂-;

Y₂ represents a single bond, a lower alkylene optionally having substituent(s), a lower alkenylene optionally having substituent(s), or a lower cycloalkylene group optionally having substituent(s);

Y₃ represents a single bond, -O-, -NR-, -S-, -SO- or -SO₂-;

R represents a hydrogen atom, or a lower alkyl group optionally having substituent(s).

Y₁ is preferably a single bond.

Y₂ is preferably a single bond, a methylene group optionally having substituent(s), a vinylene group optionally having substituent(s), or an ethylene group optionally having substituent(s).

Y₃ is preferably a single bond or -O-.

Preferred combinations of Y₁-Y₂-Y₃ are:
-lower alkylene-O-,
-lower alkylene-,
-lower alkenylene-,
-lower cycloalkylene; more preferably,
-CH₂-O-,
-CH=CH-,
-CH₂-CH₂- are recommended.

Ar represents an aromatic carbocyclic group optionally having substituent(s), an aromatic heterocyclic group optionally having substituent(s), or an aliphatic carbocyclic group optionally having substituent(s).

In Ar, examples of the substituent in "substituent optionally having substituent(s)" include substituents selected from the group consisting of the group β, or a phenyl group, a phenoxy group; and above all, preferred are those selected from a group consisting of a fluorine atom, a chlorine atom, a methyl group, an ethyl group, a methoxy group, a trifluoromethyl group, a difluoromethoxy group and a trifluoromethoxy group.

Examples of Ar include a phenyl group, a naphthyl group, a pyridinyl group, a pyrimidinyl group, a pyridazinyl group, a pyrazyl group, a pyrazole group, a pyrrolyl group, an imidazolyl group, a triazolyl group, an oxazolyl group, an isoxazolyl group, an oxadiazolyl group, a thiazolyl group, an isothiazolyl group, a thiadiazolyl group, a tetrazolyl group, a cyclopentyl group, a cyclohexyl group, a cycloheptyl group, a cyclooctyl group et al; and these may optionally have substituent(s).

In particular, preferred is a phenyl group optionally having substituent(s), or a pyridinyl group optionally having substituent(s); and more preferably recommended are a phenyl group, a 4-chlorophenyl group, a 4-fluorophenyl group, a 4-trifluoromethoxyphenyl group, a 2-pyridinyl group, a 5-fluoro-2-pyridinyl group, a 5-chloro-2-pyridinyl group et al.

X₃ and X₄ are the same or different, each representing a methine optionally having substituent(s) or a nitrogen atom.

Preferred combinations of X₃ and X₄ are:
X₃ and X₄ are a methine optionally having substituent(s), or
any one of X₃ and X₄ is a nitrogen atom, and the other is a methine optionally having substituent(s);
especially recommended is:
X₃ and X₄ are both unsubstituted methines, or any one of X₃ and X₄ is a nitrogen atom, and the other is unsubstituted methine.

X₅, X₆, X₇ and X₈ are the same or different, each representing a methine optionally having substituent(s) or a nitrogen atom, however, at least 3 atoms of X₅, X₆, X₇ and X₈ are not nitrogen atoms at the same time.

Preferred examples of X₅, X₆, X₇ and X₈ are that all of them are methines optionally having substituent(s); and especially recommended is that all of them are methines substituted with a halogen or a lower alkoxy group, or unsubstituted methines.

W represents -(O)m₁-(CH₂)n-(O)m₂-, in which -(CH₂)n- may optionally have substituent(s).

m₁ and m₂ are the same or different, each indicating 0 or 1; n indicates an integer of from 1 to 4; and they satisfy 2 ≤ m₁+m₂+n ≤ 4, but m₁, m₂ and n are not 1 at the same time.

Concretely, examples of W include:
-O-(CH₂)₂-O-,
-O-(CH₂)₃-,
-O-(CH₂)₂-,
-O-(CH₂)-,
-(CH₂)₃-O-,
-(CH₂)₂-O-,
-(CH₂)₄-,
-(CH₂)₃-,
-(CH₂)₂-.
In these, the alkylene moiety may optionally have substituent(s). Preferably recommended is -O-(CH₂)- optionally having substituent(s) (that is, ml=0, n=1 and m2=0); or -(CH₂)₂- optionally having substituent(s) (that is, m1=0, n=2 and m2=0). As the optional substituent, recommended are the substituents selected from the group consisting of the group α.

L represents a single bond or a methylene optionally having substituent(s), and L, taken together with Z₂ and R₁ and with the nitrogen atom adjacent to R₁, may form an aliphatic nitrogen-containing hetero ring optionally having substituent(s).

Z₁ represents a single bond, -O-, or a C₁₋₄ alkylene group optionally having substituent(s);

Z₂ represents a single bond or a C₁₋₄ alkylene group optionally having substituent(s).

However, Y₁, Y₂, Y₃, L, Z₁ and Z₂ are not all single bonds at the same time.

Preferably L is a single bond, or a methylene optionally having substituent(s).

Preferably, Z₁ is a single bond, a methylene optionally having substituent(s), or -O-

Preferably, Zₛ is a single bond, or a methylene optionally having substituent(s).

Preferred combinations of -Z₁-L-Z₂- are:
-CH₂-,
-(CH₂)₂-,
-(CH₂)₃-,
-(CH₂)₄-,
-O-(CH₂)₂-,
-O-(CH₂)₃-,
-CH₂-O-(CH₂)₂-.
In these, the alkylene moiety may optionally have substituent(s). More preferred are:
-CH₂-,
-(CH₂)₂-,
-O-(CH₂)₂-.
In these, the alkylene moiety may optionally have substituent(s) and preferably, as optional substituent, recommended are the substituents selected from the group consisting of the group α.

R₁ and R₂ are the same or different, each representing a hydrogen atom, a lower alkyl group optionally having substituent(s), or a lower cycloalkyl group optionally having substituent(s), and R₁ and R₂, taken together with the nitrogen atom to which they bond, may form a 3- to 11-membered, crosslinked, non-crosslinked or spiro-cyclic aliphatic nitrogen-containing hetero ring optionally having substituent(s).

Preferably, R₁ and R₂ are the same or different, each representing a hydrogen atom, a C₁₋₄ alkyl group optionally having substituent(s), or a C₃₋₆ cycloalkyl group optionally having substituent(s).

The aliphatic nitrogen-containing hetero ring optionally having substituent(s), which is formed by R₁, Z₂ and L as taken together with the nitrogen atom adjacent to R₁, is preferably a pyrrolidine ring optionally having substituent(s), in which the optional substituent is preferably selected from the group consisting of the group α.

Further, "3- to 11-membered, crosslinked, non-crosslinked or spiro-cyclic aliphatic nitrogen-containing hetero ring optionally having substituent(s)", which is formed by R₁ and R₂ taken together with the nitrogen atom bonding to them, is preferably an azetidine ring optionally having substituent(s), a pyrrolidine ring optionally having substituent(s), a piperidine ring optionally having substituent(s); more preferably an azetidinyl group optionally having substituent(s), a pyrrolidinyl group optionally having substituent(s). As the optional substituent(s), preferred are the substituents selected from the group consisting of the group α.

Preferred examples of the compounds of the invention are:
4-[(5-chloropyridin-2-yl)methoxy]-1-(2- {4-[(diethylamino)methyl]phenyl}ethyl)pyridin-2(1H)-one,
1- {2-4-(azetidin-1-ylmethyl)-3-fluorophenyl]ethyl} -4- [(4-fluorobenzyl)oxy]pyridin-2(1H)-one,
4-[(5-chloropyridin-2-yl)methoxy]-1-(2- {4-[(cyclopropylamino)methyl]phenyl} ethyl)pyridin-2(1H)-one,
4-[(5-chloropyridin-2-yl)methoxy]-1-{2-[4-(1-pyrrolidin-1-ylethyl)phenyl] ethyl}pyridin-2(1H)-one,
1-{2-[4-(1-azetidin-1-ylethyl)phenyl]ethyl}-4-[(5-chloropyridin-2-yl)methoxy]pyridin-2(1H)-one,
1- {2-[4-(azetidin-1-ylmethyl)phenyl]ethyl}-4-[(5-chloropyridin-2-yl)methoxy]pyridin-2(1H)-one,
4-[(5-chloropyridin-2-yl)methoxy]-1-{2-[4-(pyrrolidin-1-ylmethyl)phenyl]ethyl}pyridin-2(1H)-one,
4-[(4-fluorobenzyl)oxy]-1-{2-[5-(pyrrolidin-1-ylmethyl)pyridin-2-yl]ethyl}pyridin-2(1H)-one,
1- {2-[5-(azetidin-1-ylmethyl)pyridin-2-yl]ethyl}-4-[(4-fluorobenzyl)oxy]pyridin-2(1H)-one,
1- {2-[5-(azetidin-1-ylmethyl)pyridin-2-yl]ethyl}-4-[(4-chlorobenzyl)oxy]pyridin-2(1H)-one,
4-[(4-fluorobenzyl)oxy]-1-(2-fluoro-2-{4-[(propylamino)methyl]phenyl}ethyl)pyridin-2(1H)-one,
4-[(5-chloropyridin-2-yl)methoxy]-1-(2-{4-[(propylamino)methyl]phenyl}ethyl)pyridin-2(1H)-one,
1-(2,2-difluoro-2- {4-[(propylamino)methyl]phenyl}ethyl)-4-[(4-fluorobenzyl)oxy]pyridin-2(1H)-one, et al.

More preferably recommended are:
4-[(5-chloropyridin-2-yl)methoxy]-1-(2- {4-[(diethylamino)methyl]phenyl}ethyl)pyridin-2(1H)-one,
1-{2-[4-(1-azetidin-1-ylethyl)phenyl]ethyl}-4-[(5-chloropyridin-2-yl)methoxy]pyridin-2(1H)-one,
1-{2-[4-(azetidin-1-ylmethyl)phenyl]ethyl}-4-[(5-chloropyridin-2-yl)methoxy]pyridin-2(1H)-one,
1- {2-5-(azetidin-1-ylmethyl)pyridin-2-yl]ethyl}-4- [(4-fluorobenzyl)oxy]pyridin-2(1H)-one, and
1- {2-5-(azetidin-1-ylmethyl)pyridin-2-yl]ethyl}-4-[(4-chlorobenzyl)oxy]pyridin-2(1H)-one, et al.

### Production Methods for Compounds of Formula [I]

The compounds of formula [I] may be produced, for example, according to the following production methods. However, the production methods for the compounds of the invention are not limited to these reaction examples.

### Production Method 1:

Production Method 1 is for obtaining a compound of a formula [IV-a] by condensing a compound of a formula [IIa] and a compound of a formula [IIIa] according to Mitsunobu reaction. 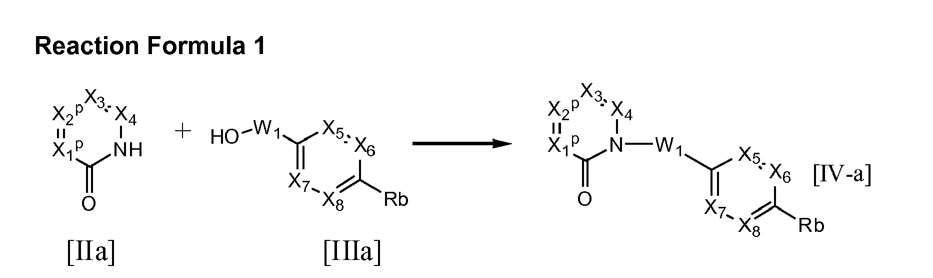 [In the formula, X₁p and X₂p each represent a methine optionally having substituent(s) selected from the group consisting of the group α, Dp, or a nitrogen atom;
any one of X₁ and X₂ is Dp, and the other is a methine optionally having substituent(s) selected from the group consisting of the group α, or a nitrogen atom,
Dp represents the following formula (a) or (b): 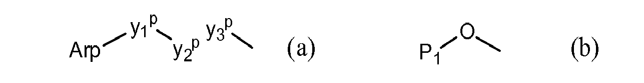 (wherein Arp represents an aromatic carbocyclic group optionally having substituent(s), an aromatic heterocyclic group optionally having substituent(s), or an aliphatic carbocyclic group optionally having substituent(s);
y₁p represents a single bond, -O-, -NR-, -S-, -SO- or -SO₂-;
y₂p represents a single bond, a lower alkylene group optionally having substituent(s), a lower alkenylene group optionally having substituent(s), or a lower cycloalkylene group optionally having substituent(s);
y₃p represents a single bond, -O-, -NR-, -S-, -SO- or -SO₂-;
R represents a hydrogen atom, or a lower alkyl group optionally having substituent(s);
P₁ represents a protective group);
Rb represents the following formula (A) or (B): 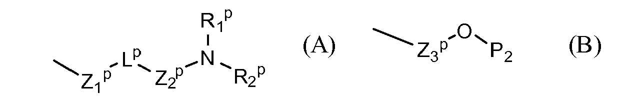 (wherein Z₁p represents a single bond, -O-, or a C₁₋₄ alkylene group optionally having substituent(s);
Lp represents a single bond, or a methylene group optionally having substituent(s); and as taken together with Z₂p and R₁p and with the nitrogen atom adjacent to R₁p, Lp may form an aliphatic nitrogen-containing hetero ring optionally having substituent(s);
Z₂p represents a single bond, or a C₁₋₄ alkylene group optionally having substituent(s);
R₁ₚ and R₂ₚ are the same or different, each representing a hydrogen atom, a lower alkyl group optionally having substituent(s), or a lower cycloalkyl group optionally having substituent(s); and
R₁p and R₂p may form, as taken together with the nitrogen atom to which they bond, a 4- to 11-membered, crosslinked, non-crosslinked or spiro-cyclic aliphatic, nitrogen-containing hetero ring; Z₃p represents a single bond, or a C₁₋₄ alkylene group optionally having substituent(s);
P₂ represents a protective group);
W₁ represents -(CH₂)n₁-(O)m₃- optionally having substituent(s); n₁ indicates an integer of from 1 to 4; m₃ indicates 0 or 1; and 1 ≤ n₁+m₃ ≤ 4; and
X₃, X₄, X₅, X₆, X₇ and X₈ have the same meanings as above.]

Specifically, in a reaction solvent, in the presence of an azodicarbonyl compound and an organophosphorus compound such as triaryl phosphine or trialkyl phosphine, a compound of formula [IIa] is condensed with a compound of formula [IIIa] to obtain a compound of formula [IV-a].

The azodicarbonyl compound includes dimethyl azodicarboxylate, diethyl azodicarboxylate, diisopropyl azodicarboxylate, di-t-butyl azodicarboxylate, 1,1'-(azodicarbonyl)dipiperidide et al; the triaryl phosphine includes triphenyl phosphine, tritolyl phosphine et al; the trialkyl phosphine includes triethyl phosphine, tributyl phosphine, trioctyl phosphine et al. Above all, recommended is a combination of diisopropyl azodicarboxylate and triphenyl phosphine, or a combination of 1,1'-(azodicarbonyl)dipiperidide and tributyl phosphine.

The amount of the compound of formula [IIIa] to be used may be from 1 mol to 3 mol relative to 1 mol of the compound of formula [IIa], preferably from 1 mol to 1.5 mols.

Regarding the amount of the azodicarbonyl compound and the organophosphorus compound to be used, the amount of the azodicarbonyl compound may be from 1 mol to 5 mols relative to 1 mol of the compound of formula [IIa], preferably from 1 mol to 3 mols, and the amount of the organophosphorus compound may be from 1 mol to 5 mols relative to one mol of the compound of formula [IIa], preferably from 1 mol to 3 mols.

The reaction solvent includes halogenocarbons such as methylene chloride, chloroform, dichloroethane, carbon tetrachloride; aliphatic hydrocarbons such as n-heptane, n-hexane; aromatic hydrocarbons such as benzene, toluene, xylene; ethers such as diethyl ether, tetrahydrofuran (hereinafter referred to as "THF"), dioxane, ethylene glycol dimethyl ether; esters such as methyl acetate, ethyl acetate; acetonitrile, N-methylpyrrolidone (hereinafter referred to as "NMP"), N,N'-dimethylformamide (hereinafter referred to as "DMF"), dimethyl sulfoxide (hereinafter referred to as "DMSO") et al; and their mixed solvents.

The reaction temperature may be from 0°C to 100°C, preferably from 0°C to 50°C; and in general, the reaction takes 2 hours to 24 hours.

### Production Method 2:

Production method 2 is for obtaining a compound of a formula [IV-a] by condensing a compound of a formula [IIa] with a compound of a formula [IIIb]. 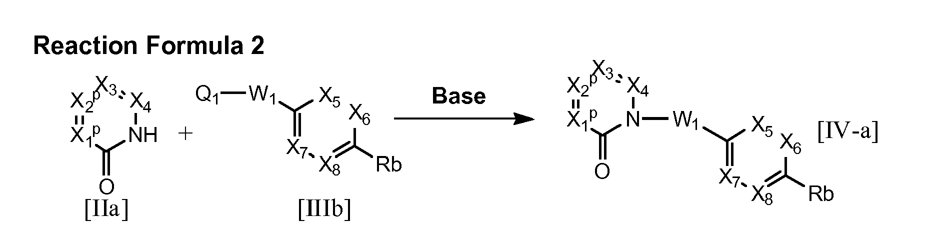 [In the formula, Q₁ represents a halogen such as chlorine, bromine; a leaving group such as a benzenesulfonyloxy group, a p-toluenesulfonyloxy group, a methanesulfonyloxy group; W₁, Rb, X₁p, X₂p, X₃, X₄, X₅, X₆, X₇ and X₈ have the same meanings as above.]

Specifically, a compound of formula [IIa] is condensed with a compound of formula [IIIb] in a reaction solvent in the presence of a base to obtain a compound of formula [IV-a].

The base includes inorganic bases such as cesium carbonate, sodium carbonate, potassium carbonate, sodium hydride, potassium hydride, cesium fluoride, sodium hydroxide, potassium hydroxide; organic bases such as sodium tert-butoxide, potassium tert-butoxide.

The amount of the compound of formula [IIa] to be used may be from 1 to 5 mols relative to 1 mol of the compound of formula [IIIb], preferably from 1 to 3 mols.

The amount of the base to be used may be from 1 to 3 mols relative to 1 mol of the compound of formula [IIa], preferably from 1 to 2 mols.

The reaction solvent includes halogenocarbons such as methylene chloride, chloroform, dichloroethane, carbon tetrachloride; aliphatic hydrocarbons such as n-heptane, n-hexane; aromatic hydrocarbons such as benzene, toluene, xylene; ethers such as diethyl ether, THF, dioxane, ethylene glycol dimethyl ether; acetonitrile, NMP, DMF, DMSO et al; and their mixed solvents.

The reaction temperature may be from 0°C to 300°C, preferably from 20°C to 150°C; and in general, the reaction takes 30 minutes to 24 hours.

### Production Method 3:

In a compound of formula [IV-a], when Rb is represented by the formula (A); or that is, in formula [I-P] when the compound of formula [I-P] has a protective group, then the compound is deprotected to give a compound of formula [I]. 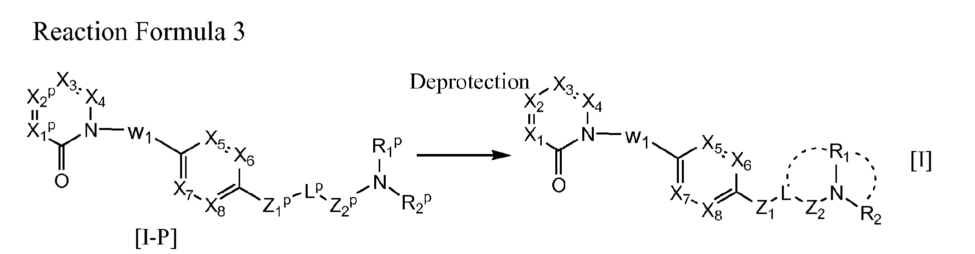 [In the formula, W₁, X₁ₚ, X₂p, X₁, X₂, X₃, X₄, X₅, X₆, X₇, X₈, Z₁p, Lp, Z₂ₚ, R₁p, R₂ₚ, Z₁, L, Z₂, R₁ and R₂ have the same meanings as above.]

In the above reaction, when the reactants have a functional group not participating in the reaction, such as an amino group, an imino group, a hydroxyl group, a carboxyl group, an oxo group or a carbonyl group, then the functional group may be suitably protected with a protective group, and the protective group may be removed after the reaction.

Having its function, the protective group for amino group and imino group is not specifically limited, and includes, for example, an aralkyl group such as a benzyl group, a p-methoxybenzyl group, a 3,4-dimethoxybenzyl group, an o-nitrobenzyl group, a p-nitrobenzyl group, a benzhydryl group, a trityl group; a lower alkanoyl group such as a formyl group, an acetyl group, a propionyl group, a butyryl group, a pivaloyl group; an arylalkanoyl group such as a benzoyl group, a phenylacetyl group, a phenoxyacetyl group; a lower alkoxycarbonyl group such as a methoxycarbonyl group, an ethoxycarbonyl group, a propyloxycarbonyl group; a tert-butoxycarbonyl group; an aralkyloxycarbonyl group such as a benzyloxycarbonyl group, a p-nitrobenzyloxycarbonyl group, a phenethyloxycarbonyl group; a lower alkylsilyl group such as a trimethylsilyl group, a tert-butyldimethylsilyl group; a tetrahydropyranyl group; a trimethylsilylethoxymethyl group; a lower alkylsulfonyl group such as a methylsulfonyl group, an ethylsulfonyl group; an arylsulfonyl group such as a benzenesulfonyl group, a toluenesulfonyl group et al; and especially preferred are an acetyl group, a benzoyl group, a tert-butoxycarbonyl group, a trimethylsilylethoxymethyl group, a methylsulfonyl group et al.

Having its function, the protective group for hydroxyl group is not specifically limited, and includes, for example, a lower alkyl group such as a methyl group, an ethyl group, a propyl group, an isopropyl group, a tert-butyl group; a lower alkylsilyl group such as a trimethylsilyl group, a tert-butyldimethylsilyl group; a lower alkoxymethyl group such as a methoxymethyl group, a 2-methoxyethoxymethyl group; a tetrahydropyranyl group; a trimethylsilylethoxymethyl group; an aralkyl group such as a benzyl group, a p-methoxybenzyl group, a 2,3-dimethoxybenzyl group, an o-nitrobenzyl group, a p-nitrobenzyl group, a trityl group; an acyl group such as a formyl group, an acetyl group et al. Especially preferred are a methyl group, a methoxymethyl group, a tetrahydropyranyl group, a trityl group, a trimethylsilylethoxymethyl group, a tert-butyldimethylsilyl group, an acetyl group et al.

Not specifically limited, the carboxyl-protective group may be any one having its function, and includes, for example, a lower alkyl group such as a methyl group, an ethyl group, a propyl group, an isopropyl group, a tert-butyl group; a lower haloalkyl group such as a 2,2,2-trichloroethyl group; a lower alkenyl group such as a 2-propenyl group; an aralkyl group such as a benzyl group, a p-methoxybenzyl group, a p-nitrobenzyl group, a benzhydryl group, a trityl group et al. Especially preferred are a methyl group, an ethyl group, a tert-butyl group, a 2-propenyl group, a benzyl group, a p-methoxybenzyl group, a benzhydryl group et al.

Not specifically limited, the protective group for oxo group and carbonyl group may be any one having its function, and includes, for example, acetals and ketals such as ethylene ketal, trimethyl ketal, dimethyl ketal et al.

The removal of the protective group, though differing depending on the type of the protective group and the stability of the product compound [I], may be attained, for example, through solvolysis with acid or base, for example, according to methods described in literature [see Protective Groups in Organic Synthesis, T. W. Greene, John Wiley & Sons, 1981], concretely, for example, according to a method of processing with from 0.01 mol to a large excessive amount of an acid, preferably trifluoroacetic acid, formic acid, hydrochloric acid, or with from an equimolar amount to a large excessive amount of a base, preferably potassium hydroxide, calcium hydroxide; or through chemical reduction with a metal hydride complex, or through catalytic reduction with a palladium-carbon catalyst or a Raney-nickel catalyst.

### Production Method 4:

In a compound of formula [IV-a], when Rb is the formula (B) and Z₃p is a single bond, or that is, when the compound is a formula [IV-b], then a compound of a formula [I-1P] may be obtained according to the following method. The obtained compound may be led to a compound of formula [I] according to the production method 3.

### (1) Production Method 4a:

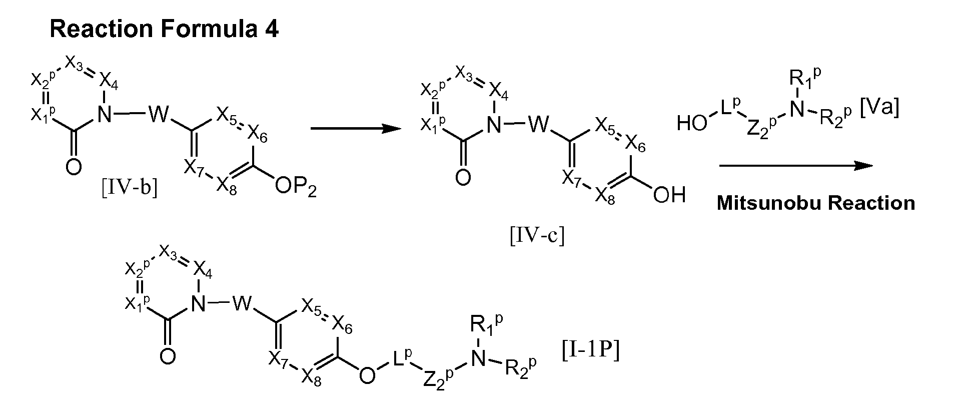

[In the formula, R₁p, R₂p, W, P₂, Lp, Z₂p, X₁p, X₂p, X₃, X₄, X₅, X₆, X₇ and X₈ have the same meanings as above.]

Specifically, the protective group P₂ in a compound of formula [IV-b] is removed to give a compound of formula [IV-c], and subsequently, the compound of formula [IV-c] is reacted with a compound of formula [Va] according to the production method 1 to give a compound of formula [I-1P], and further, when the compound has a protective group, the compound is deprotected according to the production method 3 to obtain the intended compound.

### (2) Production Method 4b:

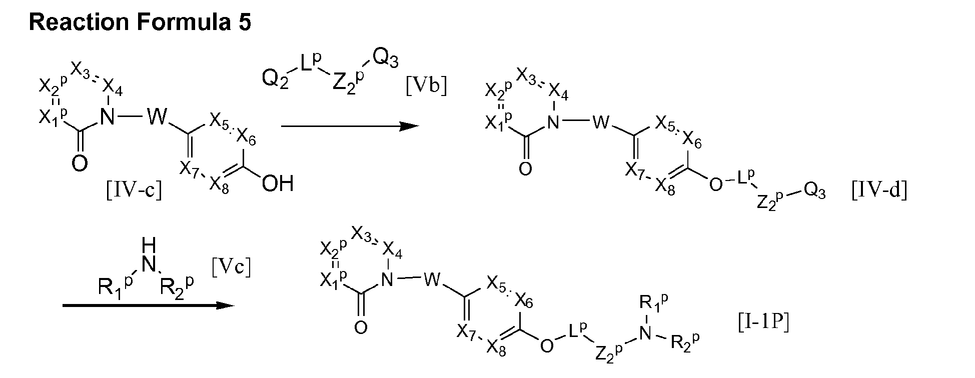

[In the formula, Q₂ and Q₃ are the same or different, each representing a halogen such as chlorine or bromine, or a leaving group such as a benzenesulfonyloxy group, a p-toluenesulfonyloxy group, a methanesulfonyloxy group; Lp, Z₂p, W, R₁p, R₂p, X₁p, X₂p, X₃, X₄, X₅, X₆, X₇ and X₈ have the same meanings as above.]

In this method, 1) a compound of formula [IV-c] is condensed with a compound of formula [Vb] in a reaction solvent in the presence of a base to give a compound of formula [IV-d], and then 2) the compound of formula [IV-d] is reacted with an amine derivative of formula [Vc] in the presence of a base to give a compound of formula [I-1P].

### Step 1):

The base includes amines such as triethylamine, diisopropylethylamine, pyridine, 4-dimethylaminopyridine, tetrabutylammonium fluoride; inorganic bases such as cesium carbonate, sodium carbonate, potassium carbonate, sodium hydride, potassium hydride, sodium hydroxide, potassium hydroxide; and organic bases such as sodium tert-butoxide, potassium tert-butoxide.

The amount of the base to be used may be from 1 mol to an excessive molar amount relative to 1 mol of the compound of formula [Vb], preferably from 2 mols to 10 mols.

The amount of the compound of formula [Vb] to be used may be from 1 mol to an excessive molar amount relative to 1 mol of the compound of formula [IV-c], preferably from 5 mols to 20 mols.

The reaction solvent includes halogenocarbons such as methylene chloride, chloroform, dichloroethane, carbon tetrachloride; aliphatic hydrocarbons such as n-heptane, n-hexane; aromatic hydrocarbons such as benzene, toluene, xylene; ethers such as diethyl ether, THF, dioxane, ethylene glycol dimethyl ether; acetonitrile, NMP, DMF, DMSO et al; and their mixed solvents.

The reaction temperature may be from 0°C to 200°C, preferably from 50°C to 150°C, and in general, the reaction takes 2 hours to 24 hours.

The compound of formula [Vb] may be a commercially-available reagent, including, for example, 1,2-dibromoethane, 1,2-dichloroethane, 1-bromo-2-chloroethane, 1-chloro-2-iodoethane, 1,2-diiodoethane, 1-chloro-3-iodopropane, 1-bromo-3-iodopropane, 1,3-dibromopropane, 1,3-dichloropropane, 1-bromo-3-chloropropane et al.

### Step 2):

The base includes inorganic bases such as cesium carbonate, sodium carbonate, potassium carbonate.

The amount of the base may be from 1 mol to an excessive molar amount relative to 1 mol of the compound of formula [IV-d], preferably from 2 mols to 10 mols.

The amount of the compound of formula [Vc] to be used may be from 1 mol to an excessive molar amount relative to 1 mol of the compound of formula [IV-d], preferably from 5 mols to 20 mols.

The reaction solvent includes halogenocarbons such as methylene chloride, chloroform, dichloroethane, carbon tetrachloride; aliphatic hydrocarbons such as n-heptane, n-hexane; aromatic hydrocarbons such as benzene, toluene, xylene; ethers such as diethyl ether, THF, dioxane, ethylene glycol dimethyl ether; acetonitrile, NMP, DMF, DMSO et al; and their mixed solvents.

The reaction temperature may be from 0°C to 200°C, preferably from 20°C to 150°C, and in general, the reaction takes 30 minutes to 12 hours.

The compound of formula [Vc] includes dimethylamine, diethylamine, di-n-propylamine, N-ethylmethylamine, N-methyl-n-propylamine, N-methyl-isopropylamine, N-ethyl-n-propylamine, N-ethyl-isopropylamine, aziridine, pyrrolidine, piperidine, N-methylpiperazine, morpholine, methylamine, ethylamine, n-propylamine, isopropylamine, cyclopropylamine, n-butylamine, sec-butylamine, tert-butylamine, cyclopropanemethylamine et al.

### Production Method 5:

In a compound of formula [IV-a], when Dp is (b), or that is, a compound of formula [IV-d] may be converted into a compound of formula [IV-f] according to the following method. 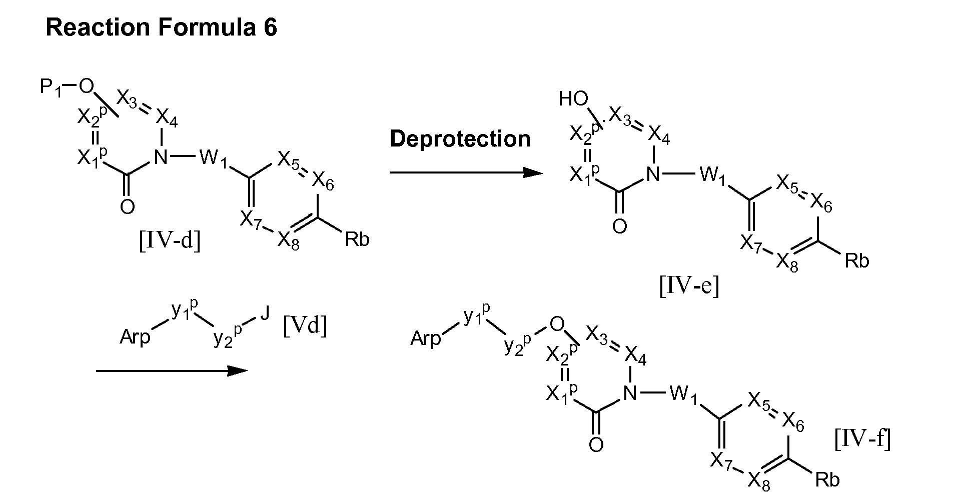 [In the formula, J represents a hydroxyl group, or a halogen such as chlorine or bromine, or a leaving group such as a benzenesulfonyloxy group, a p-toluenesulfonyloxy group, a methanesulfonyloxy group; P₁, Rb, Arp, y₁p, y₂p, W₁, X₁p, X₂p, X₃, X₄, X₅, X₆, X₇ and X₈ have the same meanings as above.]

Specifically, the protective group P₁ in the compound of formula [IV-d] is removed to give a compound of formula [IV-e], and then the compound of formula [IV-e] is reacted with a compound of formula [Vd] to give a compound of formula [IV-f].

The reaction of a compound of formula [IV-e] with a compound of formula [Vd] where J is a hydroxyl group may be attained according to the production method 1 to give a compound of formula [IV-f].

In the reaction of a compound of formula [IV-e] with a compound of formula [Vd] where J is a leaving group, in general, from 1 mol to an excessive molar amount, preferably from 1 mol to 2 mols of the compound [Vd] may be used relative to 1 mol of the compound of formula [IV-e].

The base includes inorganic bases such as cesium carbonate, sodium carbonate, potassium carbonate, sodium hydride, potassium hydride, sodium hydroxide, potassium hydroxide; organic bases such as sodium tert-butoxide, potassium tert-butoxide, preferably cesium carbonate, sodium carbonate, potassium carbonate, sodium hydroxide.

The amount of the base to be used may be from 1 mol to an excessive molar amount relative to 1 mol of the compound of formula [IVe], preferably from 1 mol to 4 mols.

The reaction solvent includes halogenocarbons such as methylene chloride, chloroform, dichloroethane, carbon tetrachloride; aliphatic hydrocarbons such as n-heptane, n-hexane; aromatic hydrocarbons such as benzene, toluene, xylene; ethers such as diethyl ether, THF, dioxane, ethylene glycol dimethyl ether; acetonitrile, NMP, DMF, DMSO et al; and their mixed solvents.

The reaction temperature may be generally from -20°C to 200°C, preferably from 0°C to 100°C, and in general, the reaction takes 2 hours to 24 hours.

In the compound of formula [IV-f], if desired, the protective group may be removed to give a compound of formula [I].

### Production Method 6:

The production method 6 is for obtaining a compound of formula [VI-a] by condensing a compound of formula [IIb] and a compound of formula [IIIb]. 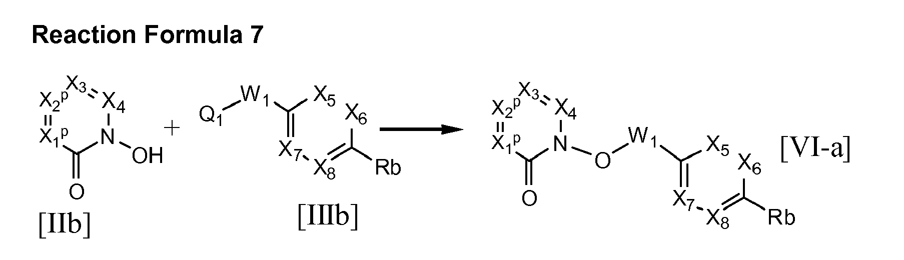 [In the formula, Rb, W₁, Q₁, X₁p, X₂p, X₃, X₄, X₅, X₆, X₇ and X₈ have the same meanings as above.]

Specifically, a compound of formula [IIb] is condensed with a compound of formula [IIIb] in a reaction solvent in the presence of a base to give a compound of formula [VIa].

The base includes amines such as triethylamine, diisopropylethylamine, pyridine, 4-dimethylaminopyridine, tetrabutylammonium fluoride; inorganic bases such as cesium carbonate, sodium carbonate, potassium carbonate, sodium hydride, potassium hydride, sodium hydroxide, potassium hydroxide; organic bases such as sodium tert-butoxide, potassium tert-butoxide.

The amount of the compound of formula [IIb] to be used may be from 1 mol to 2 mols relative to 1 mol of the compound of formula [IIIb], preferably from 1 mol to 1.5 mols.

The amount of the base to be used may be from 1 mol to 3 mols relative 1 mol of the compound of formula [IIb], preferably from 1 mol to 2 mols.

The reaction solvent includes halogenocarbons such as methylene chloride, chloroform, dichloroethane, carbon tetrachloride; aliphatic hydrocarbons such as n-heptane, n-hexane; aromatic hydrocarbons such as benzene, toluene, xylene; ethers such as diethyl ether, THF, dioxane, ethylene glycol dimethyl ether; acetonitrile, NMP, DMF, DMSO et al; and their mixed solvents.

The reaction temperature may be from -20°C to 200°C, preferably from 0°C to 100°C, and in general, the reaction takes 5 minutes to 12 hours.

### Production Method 7:

A compound of formula [IIb] and a compound of formula [IIIa] are reacted according to the production method 1 to give a compound of formula [VI-a]. 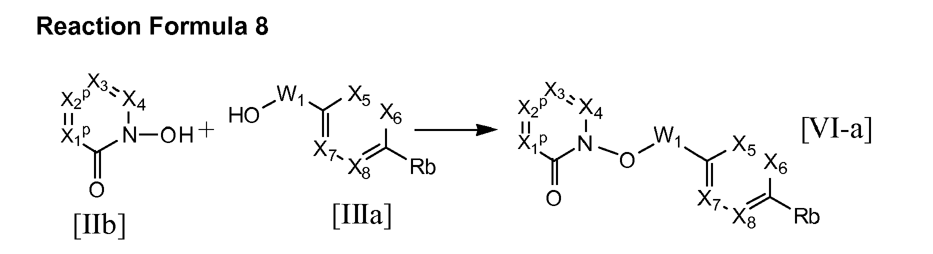 [In the formula, Rb, W₁, X₁p, X₂p, X₃, X₄, X₅, X₆, X₇ and X₈ have the same meanings as above.]

The compound of formula [IV-a] in which Rb is (B) may be reacted according to the production method 4; and the compound where Dp is (b) may be reacted according to the production method 5; and the production method 4 and the production method 5 may be suitably combined.

### Production Method 8:

The production method 8 provides a production method for a compound of formula [I] where Z₁ is a carbon atom, or that is, a compound of formula [I-2P]. 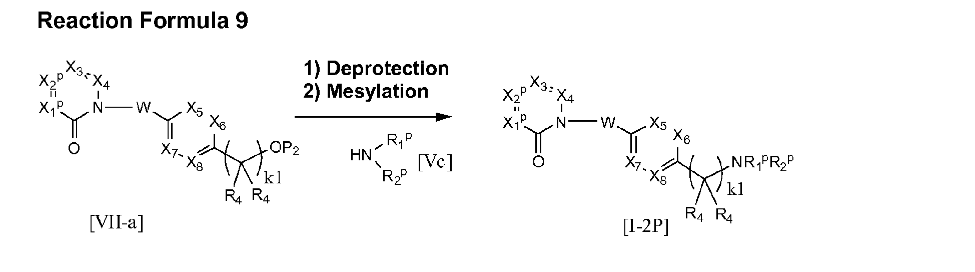 [In the formula, k₁ indicates 0 or an integer of from 1 to 3; W, R₁p, R₂p, R₄, P₂, X₁p, X₂p, X₃, X₄, X₅, X₆, X₇ and X₈ have the same meanings as above.]

Specifically, an alcohol obtained through removal of the hydroxyl-protective group P₂ in a compound of formula [VII-a] is mesylated (or tosylated or halogenated for leaving group introduction) in a conventional known method; then the obtained compound is reacted with an amine derivative of a formula [Vc] in the presence of a base to give a compound of formula [I-2P]. The removal of the hydroxyl protective group P₂, the mesylation (or leaving group introduction) may be attained in a conventional known method.

### Production Method 9:

A compound of formula [VII-a] ([VII-a1] or [VII-a2]) may be obtained by condensing a compound of formula [IIa] or [IIb] with a compound of formula [VIIIa] or [VIIIa] as in the production method 9. For the reaction condition, referred to are those for the above-mentioned production methods. 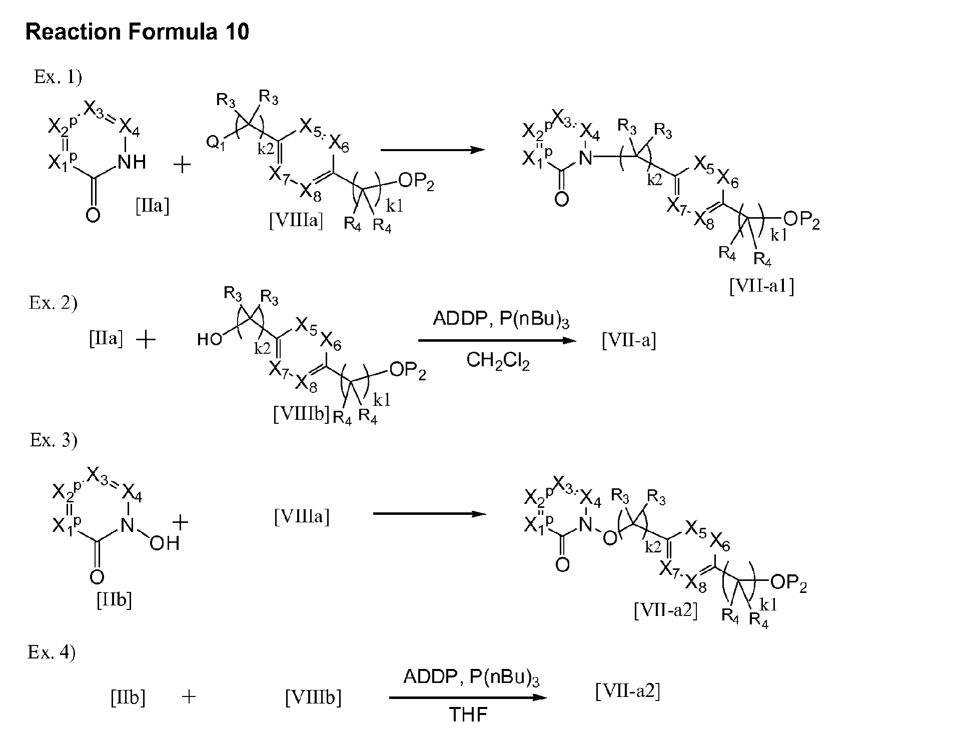 [In the formula, ADDP is an abbreviation for 1,1'-(azodicarbonyl)dipiperidide; R₃ is the same or different, representing a hydrogen atom or a group selected from the group β; R₄ has the same meaning as R₃; k2 has the same meaning as k1; Q₁, X₁p, X₂p, X₃, X₄, X₅, X₆, X₇, X₈, P₂ and K1 has the same meanings as above.]

### Production method for compound of formula [IIa], compound of formula [IIb] or their derivatives:

A compound of formula [IIa], a compound of formula [IIb] or their derivatives may be produced according to the following production methods:

### Production Method 10a:

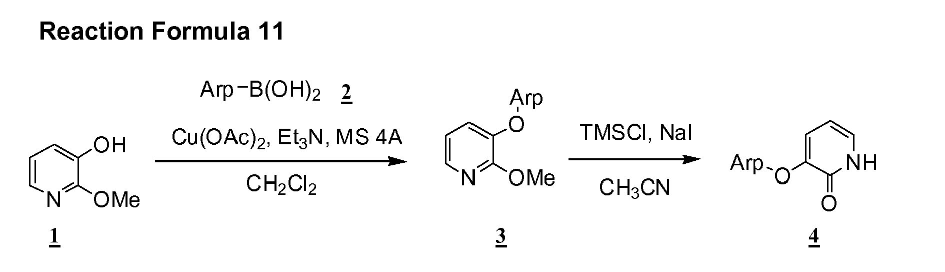

[In the formula, Arp has the same meaning as above.]

A compound 1 described in Tetrahedron (2004), 60(31), 6461-6474 is reacted with a compound 2, in methylene chloride in the presence of molecular sieves (MS-4A) along with copper acetate and triethylamine at room temperature to give a compound 3. The compound 3 is reacted in acetonitrile with trichlorosilane and sodium iodide at 0°C to room temperature to give a compound 4.

The compound 2 may be a commercial reagent, or may be prepared according to the method described in Examples.

### Production Method 10b:

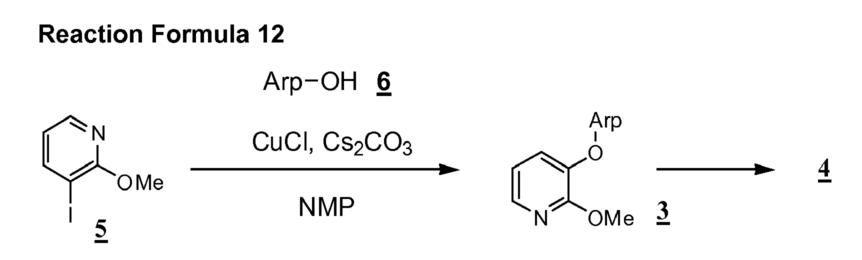

[In the formula, Arp has the same meaning as above.]

A compound 5 described in J.O.C. (1988), 57(7), 1367-1371 is condensed with a compound 6 in N-methylpyrrolidone in the presence of cesium carbonate with a catalyst of copper chloride at 120°C to give a compound 3. Further, the compound 3 is processed according to the production method 9 to give a compound 4.

The compound 6 may be a commercial reagent, or may be prepared according to the method described in Examples.

### Production Method 10c:

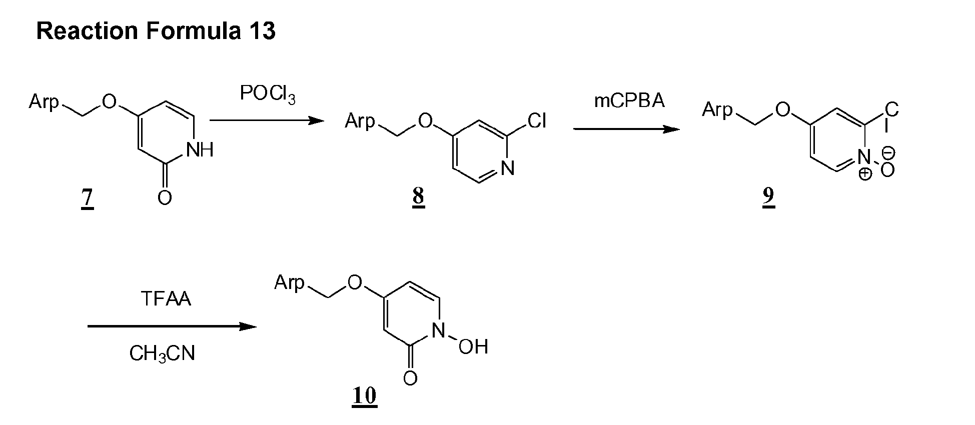

[In the formula, Arp has the same meaning as above.]

A compound 7 is reacted with phosphorus oxychloride under reflux to give a compound 8. Next, the compound 8 is reacted with m-chloroperbenzoic acid (mCPBA) in chloroform at room temperature to give a compound 9. Subsequently, in acetonitrile, this is reacted with trifluoroacetic acid anhydride (TFAA) at room temperature to give a compound 10. Production Method 10d:

The compound 7 used in the production method 10c may be prepared according to the following method: 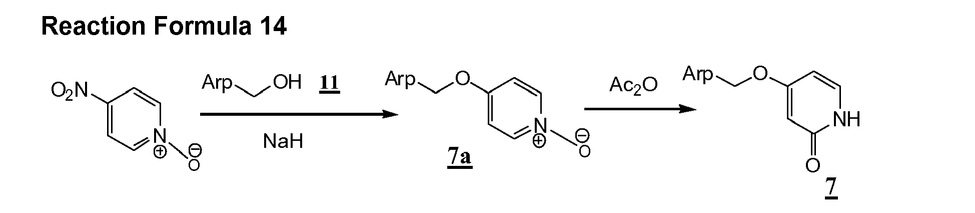 [In the formula, Arp has the same meaning as above.]

4-Nitropyridine-1-oxide is reacted with sodium hydride in DMF at 0°C for about 30 minutes, then a compound 11 is added and further reaction to give a compound 7a. Subsequently, the compound 7a is refluxed in acetic anhydride for about 1 hour to give a compound 7.

### Production Method for compound of formula [IIIa], compound of formula [IIIb] or their derivative:

A compound of formula [IIIa], a compound of formula [IIIb], their derivative and compounds of formula [VIII] can be prepared according to the following production methods. Production Method 11: 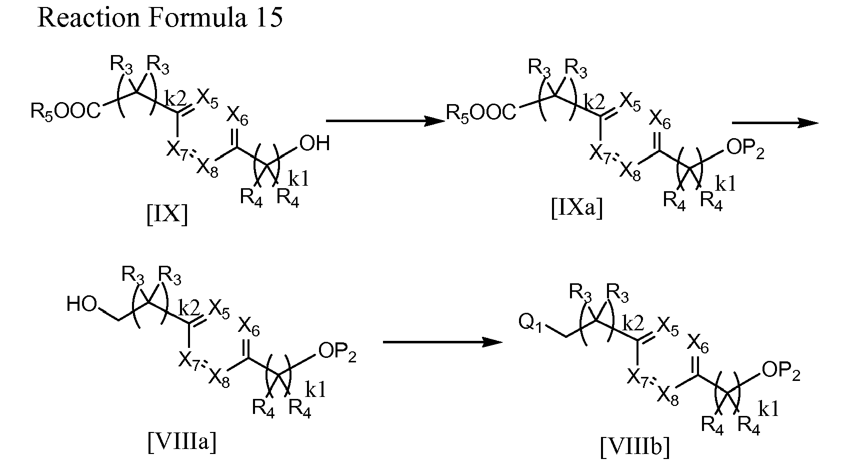 [In the formula, R₅ represents a lower alkyl group; X₅, X₆, X₇, X₈, R₃, R₄, P₂, Q₁, k1 and k2 have the same meanings as above.]

The hydroxyl group of a compound of a formula [IX] is protected to give a compound of a formula [IXa], then the ester group of the compound of formula [IXa] is reduced, for example, with lithiumaluminium hydride (LAH) to give a compound of a formula [VIIIa]. Next, the compound of formula [VIIIa] is processed for introduction of a leaving group thereinto through tosylation, mesylation or halogenation to give a compound of a formula [VIIIb].

### Production Method 12:

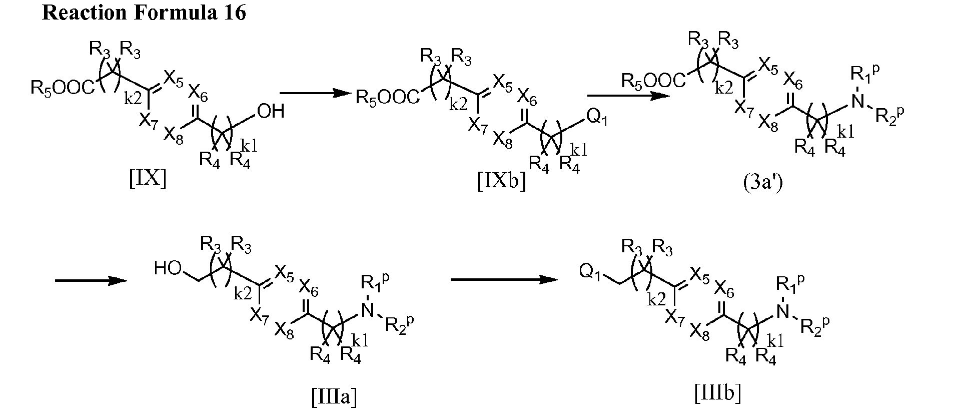

[In the formula, R₁p, R₂p, R₃, R₄, R₅, X₅, X₆, X₇, X₈, k1, k2 and Q₁ have the same meanings as above.]

The hydroxyl group of a compound of a formula [IX] is processed according to the production method 8 to give a compound of a formula (3a') via a compound of a formula [IXb]. Next, the ester moiety in the compound of formula (3a') is reduced, for example, with LAH to give a compound of a formula [IIIa], and optionally it may be converted into a compound of a formula [IIIb].

The compounds of formula [I] obtained according to the above-mentioned methods may be readily isolated and purified in any conventional known separation method. The method includes, for example, solvent extraction, recrystallization, column chromatography, liquid chromatography, or preparative thin-layer chromatography.

Depending on the type of the substituent therein, the compounds of the invention may be in any form of stereoisomers and tautomers such as optical isomers, diastereomers, geometrical isomers; and the compounds of the invention include all those stereoisomers and tautomers and their mixtures.

### Pharmacological Test of Compounds of Formula [I]

The usefulness of the compounds of the invention as medicines is verified, for example, by the following pharmacological test example.

### Pharmacological Test Example (MCH binding inhibition test)

A human MCH-1R encoding cDNA sequence [FEBS Letters, Vol. 398, 253 (1996); Biochimica et Biophisica Acta, Vol. 1401, 216 (1998)] was cloned to a plasmid vector pEF/myc/cyto (Invitrogen Corporation). The obtained expression vector was transfected to host cells CHO-K1 (American Type Culture Collection) using Lipofectamine Plus Reagent (Life Technology Inc.) to provide MCH-1R expression cells.

Membrane samples prepared from the MCH-1R expression cells were incubated with each test compound and 50 pM of [¹²⁵I]MCH (NEN Co.), in an assay buffer (50 mM Tris buffer comprising 10 mM magnesium chloride, 2 mM ethylenediamine tetraacetate, 0.01 % bacitracin and 0.2 % bovine serum albumin; pH 7.4) at 25°C for an hour, followed by filtration through a glass filter GF/C (Wattman Co.). After washing the glass filter with 50 mM Tris buffer (pH 7.4) comprising 10 mM magnesium chloride, 2 mM ethylenediamine tetraacetate and 0.04 % Tween-20, the radioactive activity on the glass filter was measured. The non-specific binding was measured in the presence of 1 µM human MCH and 50 % inhibition concentration (IC₅₀ value) of each test compound to the specific [¹²⁵I]MCH binding was determined. The results are shown in Table 1.

| Compound | IC50 (nM) |
|---|---|
| Example 1 | 5.0 |
| Example 2 | 5.9 |
| Example 14 | 7.3 |
| Example 26 | 5.8 |
| Example 41 | 3.0 |
| Example 72 | 2.6 |

The compounds of the invention has an MCH-1R antagonistic effect, and are useful as a preventive or a remedy for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation; especially as a preventive or a remedy for obesity.

### Pharmaceutical Composition Comprising Compound of Formula [I]

The compound of the invention can be orally or parenterally administered, and can be formulated into preparations suitable to the administration thereof, which may be used as a preventive or a remedy for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation; especially as a preventive or a remedy for obesity.

In its clinical use, the compound of the invention may be formulated into various preparations along with a pharmaceutically-acceptable carrier added thereto generally in accordance with the administration route thereof, and the thus-formulated pharmaceutical composition may be administered. As the carriers, usable are various conventional additives known in the field of pharmaceutical preparations. For example, they include gelatin, lactose, white sugar, titanium oxide, starch, crystalline cellulose, hydroxypropylmethyl cellulose, carboxymethyl cellulose, corn starch, microcrystalline wax, white petrolatum, magnesium aluminate metasilicate, anhydrous calcium phosphate, citric acid, trisodium citrate, hydroxypropyl cellulose, sorbitol, sorbitan fatty acid esters, polysorbate, sucrose fatty acid esters, polyoxyethylene, hardened castor oil, polyvinylpyrrolidone, magnesium stearate, light silicic anhydride, talc, vegetable oils, benzyl alcohol, gum arabic, propylene glycol, polyalkylene glycol, cyclodextrin and hydroxypropylcyclodextrin et al.

Preparations to be formed of a mixture of the carrier and a compound of the invention include, for example, solid preparations such as tablets, capsules, granules, powders and suppositories; and liquid preparations such as syrups, elixirs and injections. These may be formulated according to conventional methods known in the field of pharmaceutical preparations. The liquid preparations may also be in such a form that may be dissolved or suspended in water or in any other suitable medium in their use. Especially for injections, if desired, the preparations may be dissolved or suspended in physiological saline or glucose liquid, and a buffer or a preservative may be optionally added thereto.

The pharmaceutical compositions may contain a compound of the invention in an amount of from 1.0 to 100 % by weight, preferably from 1.0 to 60 % by weight of the composition, and may contain a pharmaceutically-acceptable carrier in an amount of from 0 to 99.9 % by weight, preferably from 40 to 99.0 % by weight. The compositions may further contain any other therapeutically-effective compound, for example, a remedial agent for diabetes, a remedial agent for hypertension, a remedial agent for arteriosclerosis.

In case where the compounds of the invention are used for prevention, treatment or remedy of the above-mentioned diseases or disorders, then the dose and the dosing frequency may be varied, depending on the sex, the age, the body weight and the disease condition of the patient and on the type and the range of the intended remedial effect. In general, the dose may be from 0.001 to 50 mg/kg of body weight/day, and it may be administered at a time or in a few times. The dose is preferably from about 0.01 to about 25 mg/kg/day, more preferably from about 0.05 to about 10 mg/kg/day.

### Combination Therapy using Compound of Formula [1]

The compounds of the invention can be used in combination with drugs effective for hypertension, obesity-associated hypertension, hypertension-associated diseases, hypertrophy, left ventricular hypertrophy, metabolic disorders, obesity, obesity-associated diseases and the like (hereafter referred to as "co-drugs"). Such drugs can be administered simultaneously, separately or in succession, for prevention or treatment of the above-mentioned diseases. When a compound of the invention is used simultaneously with one, two or more of co-drugs, they may be formulated into a medical preparation suited for single administration form. Whereas, in combination therapy, a composition containing a compound of the invention and a co-drug may be administered to the object of medication in different packages, either simultaneously, separately or successively. They may be administered at time intervals.

The dose of the co-drug may be determined in accordance with the clinically adopted dose thereof, which can be suitably selected according to the individual object of medication, the administration route, the specific disease, the combination of drugs, and the like. The form of the co-drug for administration is not specifically limited, and it may be combined with a compound of the invention when they are administered. The administration mode includes, for example, the following: (1) A compound of the invention is combined with a co-drug to give a single preparation for single administration; (2) a compound of the invention and a co-drug are separately formulated into different two preparations, and the two preparations are simultaneously administered in one administration route; (3) a compound of the invention and a co-drug are separately formulated into different two preparations, and they are administered at different times in one and the same administration route; (4) a compound of the invention and a co-drug are separately formulated into different two preparations, and they are administered at the same time in two different administration routes; (5) a compound of the invention and a co-drug are separately formulated into different two preparations, and they are administered at different times in different administration routes (for example, a compound of the invention and a co-drug are administered in that order, or in an order contrary to this). The blend ratio of the compound of the invention and the co-drug may be suitably determined depending on the administration object, the administration route, and the disease for the administration.

The co-drugs usable in the invention include, for example, "drugs for diabetes", "drugs for hyperlipidemia", "drugs for hypertension", "anti-obesity drugs". Two or more such co-drugs may be combined in an adequate ratio and used.

"Drugs for diabetes" include, for example,
1) PPAR-γ agonists such as glitazones (e.g., ciglitazone, darglitazone, englitazone, isaglitazone, MCC-555, et al), pioglitazone, rosiglitazone, troglitazone, BRL49653, CLX-0921, 5-BTZD, GW-0207, LG-100641, LY-300512, et al;
2) biguanides such as metformin, buformin, phenformin, et al;
3) protein tyrosine phosphatase 1B inhibitors;
4) sulfonylureas such as acetohexamide, chloropropamide, diabinese, glibenclamide, glipizide, glyburide, glimepiride, gliclazide, glipentide, gliquidone, glisolamide, trazamide, tolubutamide, et al;
5) meglitinides such as repaglinide, nateglinide, et al;
6) α-glucoside hydrolase inhibitors such as acarbose, adiposine, camiglibose, emiglitate, miglitol, voglibose, pradimicin-Q, salbostatin, CKD-711, MDL-25, 673, MDL-73, 945, MOR14, et al;
7) α-amylase inhibitors such as tendamistat, trestatin, A1 3688, et al;
8) insulin secretion promoters such as linogliride, A-4166, et al;
9) fatty acid oxidation inhibitors such as clomoxir, etomoxir, et al;
10) A2 antagonists such as midaglizole, isaglidole, deriglidole, idazoxan, earoxan, fluparoxan, et al;
11) insulin or insulin mimetics such as biota, LP-100, novalapid, insulin detemir, insulin lispro, insulin glargine, insulin zinc, Lys-Pro-insulin, GLP-1 (73-7), GLP1 amide (7-36), et al;
12) non-thiazolidinediones such as JT-501, farglitazar, et al;
13) PPARα/γ dual-agonists such as MK-0767, CLX-0940, GW-1536, GW-1929, GW-2433, KRP-297, L-796449, LR-90 and SB219994, et al.

"Drugs for hyperlipidemia" include, for example,
1) bile acid absorption promoters such as cholesterylamine, colesevelem, colestipol, crosslinked dextran dialkylaminoalkyl derivatives, Colestid^{™}, LoCholest^{™}, Questran^{™}, et al;
2) HMG-CoA reductase inhibitors such as atorvastatin, itavastatin, fluvastatin, lovastatin, pravastatin, rivastatin, rosuvastatin, simvastatin, ZD-4522, et al;
3) HMG-CoA synthase inhibitors;
4) cholesterol absorption inhibitors such as snatol ester, β-sitosterol, sterol glucoside, ezetimibe, et al;
5) acyl-coenzyme A·cholesterol acyl transferase inhibitors such as avasimibe, eflucimibe, KY-505, SMP-709, et al;
6) CETP inhibitors such as JTT705, torcetrapib, CP532632, BAY-63-2149, SC-591, SC-795, et al;
7) squalane synthetase inhibitors;
8) antioxidants such as probucol;
9) PPAR-α agonists such as beclofibrate, benzafibrate, syprofibrate, clofibrate, etofibrate, fenofibrate, gemcabene, gemfibrozil, GW-7647, BM-170744, LY-518674, fibric acid derivatives (e.g., Atromid^{™}, Lopid^{™}, Tricor^{™}), et al;
10) FXR receptor antagonists such as GW-4064, SR-103912, et al;
11) LXR receptor agonists such as GW3965, T9013137, XTCO-179628, et al;
12) lipoprotein synthesis inhibitors such as niacin;
13) renin-angiotensin system inhibitors;
14) microsome-triglyceride transport inhibitors;
15) bile acid resorption inhibitors such as BARA1453, SC435, PHA384640, S-435, AZD7706, et al;
16) PPAR-δ agonists such as GW501516, GW590735;
17) triglyceride synthesis inhibitors;
18) MTTP inhibitors such as LAB687, CP346086;
19) low-density lipoprotein receptor inducers;
20) squalane epoxidase inhibitors;
21) thrombocyte agglutination inhibitors;
22) 5-lipoxygenase activated protein inhibitors such as MK-591.

"Drugs for hypertension" include, for example,
1) thiazide diuretics such as chlorothialidon, chlorothiazide, dichlorofenamide, hydrofluorothiazide, indapamide, hydrochlorothiazide, et al; loop diuretics such as bumetanide, ethacrynic acid, flosemide, tolusemide, et al; sodium diuretics such as amyloride, triamuteren, et al; aldosterone antagonist diuretics such as spironolactone, epilenone, et al;
2) β-adrenaline blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, propanolol, sotalol, tartatolol, tilisolol, timolol, et al;
3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, verapamil, et al;
4) angiotensin converting enzyme inhibitors such as benazepril, captopril, cilazapril, delapril, enalapril, fosinopril, imidapril, rosinopril, moexipril, quinapril, quinaprilat, ramipril, perindopril, perindoropril, quanipril, spirapril, tenocapril, trandolapril, zofenopril, et al;
5) neutral endopeptidase inhibitors such as omapatrilat, cadoxatril, ecadotril, fosidotril, sampatrilat, AVE7688, ER4030, et al;
6) endotheline antagonists such as tezosentan, A308165, YM62899, et al;
7) vasodilators such as hydraladine, clonidine, minoxidil, nicotinyl alcohol, et al;
8) angiotensin II antagonists such as candesartan, eporsartan, iribesartan, losartan, pratosartan, tasosartan, telmisartan, valsartan, EXP-3137, FI6828K, RNH6270, et al;
9) α/β adrenaline blockers such as nipradilol, arotinolol, amoslalol, et al;
10) α1 blockers such as terazosin, urapidil, purazosin, bunazosin, trimazosin, doxazosin, naphthopidil, indolamin, WHIP 164, XEN010, et al;
11) α2 agonists such as lofexidine, tiamenidine, moxonidine, rilmenidine, guanobenz, et al;
12) aldosterone inhibitors.

"Anti-obesity drugs" include, for example,
1) 5HT (serotonin) transporter inhibitors such as paroxetine, fluoxetine, fenfluramine, fluvoxamine, sertraline, imipuramine, et al;
2) norepinephrine transporter inhibitors such as GW320659, desipramin, talsupramin, nomifensin, et al;
3) cannabinoid-1 receptor 1 (CB-1) antagonists/inverse-agonists such as limonabant (Sanofi Synthelabo), SR-147778 (Sanofi Synthelabo), BAY-65-2520 (Bayer), SLV-319 (Sorbei), as well as compounds disclosed in USP 5,532,237, USP 4,973,587, USP 5,013,837, USP 5,081,122, USP 5,112,820, USP 5,292,736, USP 5,624,941, USP 6,028,084, WO96/33159, WO98/33765, WO98/43636, WO98/43635, WO01/09120, WO01/96330, WO98/31227, WO98/41519, WO98/37061, WO00/10967, WO00/10968, WO97/29079, WO99/02499, WO01/58869, WO02/076949, WO01/64632, WO01/64633, WO01/64634, WO03/006007, WO03/007887, and EP-658546, et al;
4) ghrelin antagonists such as compounds disclosed in WO01/87355, WO02/08250, et al;
5) histamine(H3) antagonists/inverse-agonists such as thioperamide, 3-(1H-imidazol-4-yl)propyl N-(pentenyl)carbonate, clobenpropit, iodofenpropit, imoproxyfen, GT2395, A331440, compounds disclosed in WO02/15905, O-[3-(1H-imidazol-4-yl)propanol] carbamate, piperazine-containing H3-receptor antagonists (Lazewska, D. et al., Pharmazie, 56: 927-32 (2001)), benzophenone derivatives (Sasse, A. et al., Arch. Pharm (Weinheim) 334: 45-52 (2001)), substituted N-phenylcarbamates (Reidemeister, S. et al., Pharmazie, 55: 83-6 (2000)), proxyfen derivatives (Sasse, A. et al., J. Med. Chem., 43: 3335-43 (2000)), et al;
6) MCH-1R antagonists such as T-226296 (Takeda), SNP-7941 (Synaptic), other compounds disclosed in WO01/82925, WO01/87834, WO02/051809, WO02/06245, WO02/076929, WO02/076947, WO02/04433, WO02/51809, WO02/083134, WO02/094799, WO03/004027, and JP-A 2001-226269, et al;
7) MCH-2R agonists/antagonists;
8) NPY1 antagonists such as isopropyl 3-chloro-5-(1-(6-[2-(5-ethyl-4-methyl-thiazol-2-yl)-ethyl]-4-morpholinyl-4-yl-piridin-2-ylamino)-ethyl)phenyl]carbamate, BIBP3226, BIBO3304, LY-357897, CP-671906, GI-264879, and other compounds disclosed in USP 6,001,836, WO96/14307, WO01/23387, WO99/51600, WO01/85690, WO01/85098, WO01/85173, and WO01/89528, et al;
9) NPY5 antagonists such as 152804, GW-569180A, GW-594884A, GW-587081X, GW-548118X, FR235,208, FR226928, FR240662, FR252384, 1229U91, GI-264879A, CGP71683A, LY-377897, LY366377, PD-160170, SR-120562A, SR-120819A, JCF-104, H409/22, and other compounds disclosed in USP 6,140,354, USP 6,191,160, USP 6,258,837, USP 6,313,298, USP 6,337,332, USP 6,329,395, USP 340,683, USP 6,326,375, USP 6,329,395, USP 6,337,332, USP 6,335,345, EP-01010691, EP-01044970, WO97/19682, WO97/20820, WO97/20821, WO97/20822, WO97/20823, WO98/27063, WO00/107409, WO00/185714, WO00/185730, WO00/64880, WO00/68197, WO00/69849, WO01/09120, WO01/14376, WO01/85714, WO1/85730, WO01/07409, WO01/02379, WO01/02379, WO01/23388, WO01/23389, WO01/44201, WO01/62737, WO01/62738, WO01/09120, WO02/20488, WO02/22592, WO02/48152, WO02/49648, WO02/094789, and compounds disclosed in Norman et al., J. Med. Chem., 43:4288-4312(2000), et al;
10) leptins such as human recombinant leptin (PEG-OB, Hoffman La Roche), recombinant methionylleptin (Amgen), et al;
11) leptin derivatives such as compounds disclosed in USP 5,552,524, USP 5,552,523, USP 5,552,522, USP 5,521,283, WO96/23513, WO96/23514, WO96/23515, WO96/23516, WO96/23517, WO96/23518, WO96/23519, and WO96/23520, et al;
12) opioid antagonists such as nalmefen (Revex^{™}), 3-methoxynaltorexon, naloxone, naltorexone, compounds disclosed in WO00/21509, et al;
13) orexin antagonists such as SB-334867A, and other compounds disclosed in WO01/96302, WO01/68609, WO02/51232, WO02/51838, and WO03/023561, et al;
14) bonbesin receptor subtype-3 agonists;
15) cholecystokinin A (CCK-A) agonists such as AR-R15849, GI-181771, JMV-180, A-71378, A-71623, SR-146131, and other compounds disclosed in USP 5,739,106, et al;
16) CNTF (ciliary neurotrophic factors) such as GI-181771 (Glaxo-Smith Kline), SR146131 (Sanofi Synthelabo), butabindide, PD170,292, PD149164 (Pfizer), et al;
17) CNTF derivatives such as axokine (Regeneron), and other compounds disclosed in WO94/09134, WO98/22128, WO99/43813, et al;
18) growth hormone secretion receptor agonists such as NN703, hexarelin, MK-0677, SM-130686, CP-424,391, L-692,429, L-163,255, and compounds disclosed in USP 6,358,951, US Patent Application Nos. 2002/049196, 2002/022637, WO01/56592, WO02/32888, et al;
19) serotonin receptor-2C agonists such as BVT933, DPCA37215, IK264, PNU22394, WAY161503, R-1065, YM348, and other compounds disclosed in USP 3,914,250, WO02/36596, WO02/48124, WO02/10169, WO01/66548, WO02/44152, WO02/51844, WO02/40456, WO02/40457, et al;
20) melanocortin-3 receptor agonists;
21) melanocortin-4 receptor agonists such as CHIR86036 (Chiron), ME-10142, ME-10145 (Melacure), and other compounds disclosed in WO99/64002, WO00/74679, WO01/991752, WO01/74844, WO01/70708, WO01/70337, WO01/91752, WO02/059095, WO02/059107, WO02/059108, WO02/059117, WO02/12166, WO02/11715, WO02/12178, WO02/15909, WO02/068387, WO02/068388, WO02/067869, WO03/007949, and WO03/009847, et al;
22) monoamine resorption inhibitors such as sibutramine (Meridia^{™}/Reductil^{™}) and its salts, and other derivatives disclosed in USP 4,476,680, USP 4,806,570, USP 5,436,272, US Patent Application No. 2002/0006964, WO01/27068, and WO01/62341, et al;
23) serotonin re-uptake inhibitors such as dexfenfluramine, fluoxetine, and other compounds disclosed in USP 6,365,633, WO01/27060, and WO01/162341, et al;
24) glucagon-like peptide-1 agonists;
25) topiramate (Topimax^{™});
26) phytopharm compound 57 (e.g., CP644,673);
27) acetyl CoA carboxylase-2 (ACC2) inhibitors;
28) β-adrenalin receptor-3 agonists such as AD9677/TAK677 (Dai-Nippon Pharmaceutical/Takeda Chemical), CL-316,243, SB418790, BRL-37344, L-796568, BMS-196085, BRL-35135A, CGP12177A, BTA-243, W427353, trecadrine, Zeneca D7114, SR59119A, and other compounds disclosed in USP 5,705,515, USP 5,451,677, WO01/74782, and WO02/32898, et al;
29) diacylglycerol acyltransferase-1 inhibitors;
30) diacylglycerol acyltransferase-2 inhibitors,
31) fatty acid synthetase inhibitors such as carulenin, C75;
32) phosphodiesterase inhibitors such as theophylline, pentoxiphylline, zaprinast, sildenafil, amrinone, milrinone, cilostamide, rolipram, and cilomilast, et al;
32) thyroid hormone-β agonists such as KB-2611 (KaroBio BMS), and other compounds disclosed in WO02/15845, JP-A 2000-256190, et al;
33) UCP (uncoupling protein)-1, 2, or 3 activators such as phytanic acid, 4-[(E)-2-(5,6,7,8-tetrahydro-5,5,8,8-tetramethyl-2-naphthalenyl-1-propenyl]benzoic acid (TTNPB), retinoic acid, and other compounds disclosed in WO99/00123, et al;
34) acylestrogens such as oleoylestrone, and other compounds disclosed in del Mar-Grasa, M. et al., Obesity Research, 9:202-9 (2001), et al;
35) glucocorticoid antagonists;
36) 11-β-hydroxysteroid dehydrogenase-1 inhibitors such as BVT3498, BVT2733, and other compounds disclosed in WO01/90091, WO01/90090, WO01/90092, et al;
37) stearoyl-CoA desaturase-1 inhibitors;
38) dipeptidyl peptidase-IV inhibitors such as isoleucine thiazolidine, valine pyrrolidide, NVP-DPP728, AF237, P93/01, TSL225, TMC-2A/2B/2C, FE999011, P9310/K364, VIP0177, SDZ274-444, and other compounds disclosed in WO03/004498, WO03/004496, EP1258476, WO02/083128, WO02/062764, WO03/000250, WO03/002530, WO03/002531, WO03/002553, WO03/002539, WO03/000180, and WO03/000181, et al;
39) lipase inhibitors such as tetrahydroliptatin (Orlistat/Xenical^{™}), Triton WR1339, RHC80267, lipstatin, teasaponin, diethylumbelliferyl phosphate, FL-386, WAY-121898, Bay-N-3176, valilactone, esteracin, ebelactone A, ebelactone B, RHC80267, and other compounds disclosed in WO01/77094, USP 4,598,089, USP 4,452,813, USP 5,512,565, USP 5,391,571, USP 5,602,151, USP 4,405,644, USP 4,189,438, and USP 4,242,453, et al;
39) fatty acid transporter inhibitors;
40) dicarboxylate transporter inhibitors;
41) glucose transporter inhibitors;
42) phosphate transporter inhibitors.

Those combination drugs are obtained by concurrent use of a compound of the invention with one, two or more of the above co-drugs. Furthermore, the combination drugs are useful for prevention or therapy of metabolic disorders, when combined with one, two or more drugs selected from the group consisting of diabetes-treating agents and hyperlipidemia-treating agents. Combinations containing, in particular, hypertension-treating agent and anti-obesity agent are useful for prevention or treatment for metabolic disorders with synergistic effect, when diabetes-treating agent and/or hyperlipidemia-treating agent are added thereto.

### BEST MODE FOR CARRYING OUT THE INVENTION

The invention is described more concretely with reference to the following Examples, to which, however, the invention should not be limited. As a microwave organic synthesis device, used was Initiator^{™} 60 (Biotage Japan). As silica gel for columns, used was Wakogel^{™} C-200 or C-300 (Wako Pure Chemical Industries); as a filled silica gel column, used was a FLASH+^{™} cartridge, KP-Sil FLASH12+M, FLASH25+M or FLASH40+M (Biotage Japan); as a reversed-phase column for HPLC, used was YMC-Pack^{™} pro C-18 (YMC); as a chiral column for HPLC, used were CHIRALPAK^{™} AD, CHIRALPAK^{™} AS, CHIRALPAK^{™} IA, CHIRALCEL^{™} OD, CHIRALCEL^{™} OJ (Daicel Chemical). For mass spectrometry, used was Quattro^{II} (Micromass).

### EXAMPLES

### Reference Example 1:

### 4-[(4-Chlorobenzyl)oxy]pyridin-2(1H)-one:

### (1) Production of 4-[(4-chlorobenzyl)oxy]pyridine 1-oxide:

With cooling with ice, 4-chlorobenzyl alcohol (5.35 g, 37.5 mmol) was added to a DMF (50 mL) suspension of sodium hydride (60 % oily, 1.60 g, 39.3 mmol), and stirred at room temperature for 30 minutes. 4-Nitropyridine 1-oxide (5.00 g, 35.7 mmol) was added to the reaction liquid little by little with stirring, and stirred overnight at room temperature. Next, aqueous sodium hydrogencarbonate solution was added to the reaction liquid, and extracted with chloroform. The obtained organic layer was dried with anhydrous sodium sulfate, and filtered. The obtained filtrate was concentrated under reduced pressure, and ethyl acetate and diisopropyl ether were added to the residue, then the formed solid was collected by filtration to obtain the entitled compound (7.1 g, 84 %).
Mass Spectrum (ESI): 236.1 (M+H).

### (2) Production of 4-[(4-chlorobenzyl)oxy]pyridin-2(1H)-one:

4-[(4-Chlorobenzyl)oxy]pyridine 1-oxide (4.0 g, 17.0 mmol) was refluxed in acetic anhydride (15 mL) for 15 hours. The reaction liquid was cooled to room temperature, then concentrated under reduced pressure, and a mixed solvent of aqueous saturated sodium hydrogen carbonate solution and methanol was added to the obtained residue and stirred at room temperature for 15 minutes. The reaction liquid was filtered through Celite, then concentrated, and aqueous sodium hydrogencarbonate solution was added to the obtained residue, and extracted with chloroform. The organic layer was dried with anhydrous sodium sulfate, then filtered, and the obtained filtrate was concentrated under reduced pressure. The obtained residue was purified by silica gel column chromatography (methanol:chloroform = 0:10 to 1:19) to obtain the entitled compound (1.96 g, 49 %).
¹HNMR (400 MHz, DMSO, δ ppm): 5.06 (2H, s), 5.77 (1H, d, J=2.4 Hz), 5.85-5.95 (1H, m), 7.25 (1H, d, J=5.6 Hz).7.40-7.50 (4H, m).

### Reference Example 2:

### 4-[(4-Fluorobenzyl)oxy]pyridin-2(1H)-one

In the same manner as in Reference Example 1 but changing 4-chlorobenzyl alcohol used in Reference Example 1-(1) to a corresponding benzyl alcohol compound, the entitled compound was obtained.
¹HNMR (400 MHz, DMSO-d₆, δ ppm): 5.04 (2H, s), 5.79 (1H, d, J=2.4 Hz), 5.90 (1H, dd, J=7.3, 2.4 Hz), 7.20-7.26 (3H, m), 7.45-7.50 (2H, m).

### Reference Example 3:

### 4-[(5-Chloropyridin-2-yl)methoxy]pyridin-2(1H)-one:

### (1) Production of (5-chloropyridin-2-yl)methanol:

With cooling with ice, a toluene solution (66 mL, 66.0 mmol) of 1 N diisopropylaluminium hydride was gradually added to a THF solution (40 mL) of ethyl 5-chloropyridine-2-carboxylate (4.05 g, 22.0 mmol) [Heterocycles, 51(11), 2589 (1999)], then stirred at 0°C for 2 hours. Next, sodium borohydride (832 mg, 22.0 mmol) and methanol (10 mL) were added to the reaction liquid with cooling with ice, and stirred at room temperature for 3 hours. Ether was added to the reaction liquid, then water (5 mL) and sodium sulfate 10-hydrate were added, and stirred overnight at room temperature. The insoluble matter was removed from the reaction liquid by filtration through Celite, then the obtained filtrate was concentrated under reduced pressure to obtain the entitled compound (3.14 g, 100 %).
¹HNMR (400 MHz, CDCl₃, δ ppm): 4.75 (2H, s), 7.20-7.30 (1H, m), 7.67 (1H, dd, J=8.0, 2.4 Hz), 8.53 (1H, d, J=2.4 Hz).

### (2) Production of 4-[(5-chloropyridin-2-yl)methoxy]pyridin-2(1H)-one:

In the same manner as in Reference Example 1 but changing 4-chlorobenzyl alcohol used in Reference Example 1-(1) to (5-chloropyridin-2-yl)methanol, the compound of Reference Example 3 was obtained.
¹HNMR (400 MHz, DMSO-d₆, δ ppm): 5.14 (2H, s), 5.76 (1H, d, J=2.6 Hz), 5.95 (1H, dd, J=7.2, 2.6), 7.26 (1H, d, J=7.2 Hz), 7.55 (1H, d, J=8.8 Hz), 8.00 (1H, dd, J=8.8 Hz, 2.4 Hz), 8.63-8.66 (1H, m).

### Reference Example 4:

### 4-[(5-Fluoropyridin-2-yl)methoxy]pyridin-2(1H)-one:

In the same manner as in Reference Example 3 but changing ethyl 5-chloropyridine-2-carboxylate used in Reference Example 3-(1) to ethyl (5-fluoropyridine-2-carboxylate) [Tetrahedron, 49(7), 1445 (1993)], the entitled compound was obtained.
Mass Spectrum (ESI): 221.2 (M+H).

### Reference Example 5:

### 4-(Benzyloxy)pyrimidin-2(1H)-one:

N-butyl lithium (2.6 M n-hexane solution, 12.75 mL) was added to a THF solution (80 mL) of benzyl alcohol (5.21 mL, 50.0 mmol) at 0°C, then stirred at the same temperature for 30 minutes. The reaction liquid was gradually added to a tetrahydrofuran suspension (80 mL) of 2,4-dichloropyrimidine (5.00 g, 34.0 mmol), and stirred at room temperature for 2.5 hours. Next, saturated saline was added to the reaction liquid, and extracted with ethyl acetate. The organic layer was washed with water and saturated saline, then dried with anhydrous magnesium sulfate, and filtered. The obtained filtrate was concentrated under reduced pressure, then a solution obtained by adding, with cooling with ice, n-butyllithium (2.6 M n-hexane solution, 19.05 mL) to a THF (80 mL) solution of allyl alcohol (4.80 mL, 70 mmol) followed by stirring for 30 minutes was dropwise added to a solution of the residue dissolved in THF (80 mL), with cooling with ice over 40 minutes, and then stirred at room temperature for 13 hours. Next, saturated saline was added to the reaction liquid, and extracted with ethyl acetate. The obtained organic layer was washed with water and saturated saline, then dried with anhydrous magnesium sulfate, and filtered. The obtained filtrate was concentrated under reduced pressure, then the residue was dissolved in THF (140 mL), and piperidine (5.0 mL, 50.0 mmol) and tetrakis(triphenylphosphine)palladium (1.9 g, 1.7 mmol) were added and stirred at room temperature for 6 hours. Ethyl acetate was added to the reaction liquid, then filtered to remove the insoluble matter. The obtained filtrate was washed with water and saturated saline, then dried with anhydrous magnesium sulfate, and filtered. The obtained filtrate was concentrated under reduced pressure, and the formed solid was collected by filtration to obtain the entitled compound (735 mg, 11 %).
Mass Spectrum (ESI): 203.1 (M+H).

### Reference Example 6:

### 4-[(4-fluorobenzyl)oxy]-1-hydroxypiperidin-2(1H)-one:

### (1) Production of 2-chloro-4-[(4-fluorobenzyl)oxy]pyridine:

The compound of Reference Example 2 (1.00 g, 4.56 mmol) was refluxed in phosphorus oxychloride (5 mL) for 2 hours. The reaction liquid was concentrated, then aqueous saturated sodium hydrogencarbonate solution was added to the obtained residue, and extracted with ethyl acetate. The obtained organic layer was washed with saturated saline, dried with anhydrous sodium sulfate, and filtered. The obtained filtrate was concentrated, then the residue was purified by silica gel column chromatography (hexane:ethyl acetate = 10:0 to 8:2) to obtain the entitled compound (567 gm, 60 %).
Mass Spectrum (ESI): 238.0 (M+H).

### (2) 4-[(4-Fluorobenzyl)oxy]-1-hydroxypyridin-2(1H)-one.

With cooling with ice, m-chloroperbenzoic acid (1.44 g, 8.37 mmol) was added to a chloroform solution (20 mL) of 2-chloro-4-[(4-fluorobenzyl)oxy]pyridine (1.53 g, 6.44 mmol), and stirred overnight at from 0°C to room temperature. Aqueous saturated sodium hydrogencarbonate solution was added to the reaction liquid, then extracted with chloroform. The obtained organic layer was dried with anhydrous sodium sulfate, then filtered, and the filtrate was concentrated under reduced pressure. The obtained residue was purified by silica gel column chromatography (chloroform:methanol = 10:0 to 19:1) to obtain a white solid. Acetonitrile (47 mL) was added to the obtained solid, then trifluoroacetic acid anhydride (1.34 mL, 9.46 mmol) was added, and stirred overnight at room temperature. The reaction liquid was concentrated under reduced pressure, then aqueous saturated ammonium chloride solution was added, and extracted with chloroform. The organic layer was dried with anhydrous sodium sulfate, then filtered, and the obtained filtrate was concentrated under reduced pressure to obtain the entitled compound (982 mg, 64 %).
Mass Spectrum (ESI): 236.1 (M+H).

### Reference Example 7:

### 4-(2-Naphthyl)pyridin-2(1H)-one:

### (1) Production of 2-fluoro-4-(2-naphthyl)pyridine:

Tetrakis(triphenylphosphine)palladium (25 mg, 0.023 mmol) and aqueous 2 N sodium carbonate solution (0.5 mL) were added to a DME (3.3 mL) solution of 2-fluoro-4-iodopyridine (100 mg, 0.45 mmol) and 2-naphthylboronic acid (117 mg, 0.68 mmol), and irradiated with microwaves (120°C, 10 minutes). Next, water was added to the reaction liquid, and extracted with ethyl acetate. The organic layer was dried with anhydrous sodium sulfate, filtered, then the filtrate was concentrated under reduced pressure, and the obtained residue was purified by silica gel column chromatography (hexane:ethyl acetate = 85:15) to obtain the entitled compound (77 mg, 76 %).
¹HNMR (400 MHz, CDCl₃, δ ppm): 7.23-7.30 (1H, m), 7.50-7.60 (3H, m), 7.73 (1H, dd, J=8.8, 2.0 Hz), 7.87-8.00 (3H, m), 8.10-8.15 (1H, m), 8.31 (1H, d, J=5.6 Hz).

### (2) Production of 4-(2-naphthyl)pyridin-2(1H)-one:

4 N hydrochloric acid (20 mL) was added to 2-fluoro-4-(2-naphthyl)pyridine (810 mg, 3.63 mmol), and heated under reflux for 1.5 days. Water was added to the reaction liquid, then the formed solid was collected by filtration to obtain the entitled compound (802 mg, 100 %).
Mass Spectrum (ESI): 222.1 (M+H).

### Reference Example 8:

### 4-[(E)-2-(4-fluorophenyl)vinyl]pyridin-2-(1H)-one:

### (1) Production of 2-fluoro-4-[(E)-2-(4-fluorophenyl)vinyl]pyridine:

Palladium acetate (92 mg, 0.41 mmol), triphenyl phosphine (215 mg, 0.82 mmol) and triethylamine (2.26 mL, 16.2 mmol) were added to 2-fluoro-4-iodopyridine (3.0 g, 13.5 mmol) and 1-fluoro-4-vinylbenzene (1.93 mL, 16.2 mmol), and stirred at 100°C for 24 hours. Ethyl acetate was added to the reaction liquid, then the formed insoluble matter was removed by filtration, the obtained filtrate was washed with aqueous saturated ammonium chloride solution and saturated saline, dried with anhydrous magnesium sulfate and filtered. The filtrate was concentrated under reduced pressure, and the obtained residue was purified by silica gel column chromatography (hexane:ethyl acetate = 10:0 to 8:2) to obtain the entitled compound (1.46 g, 50 %).
Mass Spectrum (ESI): 218.1 (M+H).

### (2) Production of 4-[(E)-2-(4-fluorophenyl)vinyl]pyridin-2-(1H)-one:

In the same manner as in Reference Example 7-(2) but changing 2-fluoro-4-(2-naphthyl)pyridine used in Reference Example 7-(2) to 2-fluoro-4-[(E)-2-(4-fluorophenyl)vinyl]pyridine, the entitled compound was obtained.
Mass Spectrum (ESI): 216.1(M+H).

### Reference Examples 9, 10:

In the same manner as in Reference Example 8 but changing 1-fluoro-4-vinylbenzene used in Reference Example 8 to corresponding vinyl compounds, the compounds of Reference Examples 9 and 10 were obtained.

### Reference Example 9:

### 4-[(E)-2-(4-chlorophenyl)vinyl]pyridin-2(1H)-one

Mass Spectrum (ESI): 232.1 (M+H).

### Reference Example 10:

### 4-[(E)-2-phenylvinyl]pyridin-2(1H)-one

Mass Spectrum (ESI): 198.1 (M+H).

### Reference Example 11:

### 3-(4-Phenoxyphenyl)pyridin-2(1H)-one:

### (1) Production of 2-fluoro-3-(4-phenoxyphenyl)pyridine:

Tetrakis(triphenylphosphine)palladium (492 mg, 0.45 mmol) and aqueous 2 N sodium carbonate solution (10 mL) were added to an ethylene glycol dimethyl ether (hereinafter referred to as "DME") (66 mL) solution of 2-fluoro-3-iodopyridine (2.00 g, 8.97 mmol) and 4-(phenoxyphenyl)boronic acid (2.50 g, 11.66 g), and heated under reflux for 12 hours. Water was added to the reaction liquid, and extracted with ethyl acetate. The obtained organic layer was washed with saturated saline, then dried with anhydrous sodium sulfate, and filtered. The obtained filtrate was concentrated under reduced pressure, and the residue was purified by silica gel column chromatography (hexane:ethyl acetate = 10:0 to 17:3) to obtain the entitled compound (2.06 g, 87 %).
Mass Spectrum (ESI): 266.1 (M+H).

### (2) Production of 3-(4-phenoxyphenyl)pyridin-2(1H)-one:

In the same manner as in Reference Example 7-(2) but changing 2-fluoro-4-(2-naphthyl)pyridine used in Reference Example 7-(2) to 2-fluoro-3-(4-phenoxyphenyl)pyridine, the entitled compound was obtained.
¹HNMR (400 MHz, DMSO-d₆, δ ppm): 6.28 (1H, t, J=6.8 Hz), 6.95-7.05 (4H, m), 7.10-7.20 (1H, m), 7.35-7.45 (3H, m), 7.63 (1H, dd, J=6.8, 2.0 Hz), 7.70-7.80 (2H, m), 11.79 (1H, s). Reference Example 12:

### 3-[4-(Trifluoromethoxy)phenoxy]pyridin-2(1H)-one:

### (1) Production of 2-methoxy-3-(4-(trifluoromethoxy)phenoxy]pyridine:

2,6-Dimethylheptane-3,5-dione (0.019 mL, 0.085 mmol), copper(I) chloride (43 mg, 0.43 mmol) and cesium carbonate (554 mg, 1.70 mmol) were added to an NMP (2 mL) solution of 3-iodo-2-methoxypyridine (200 mg, 0.85 mmol), and stirred for 2.5 days in the presence of nitrogen at 120°C. Aqueous saturated sodium hydrogencarbonate solution was added to the reaction liquid, then the insoluble matter was removed by filtration through Celite, followed by extraction with ethyl acetate. The obtained organic layer was washed with aqueous saturated sodium hydrogencarbonate solution, then dried with anhydrous sodium sulfate, and filtered. The obtained filtrate was concentrated under reduced pressure, then the residue was purified by silica gel column chromatography (hexane:ethyl acetate = 10:0 to 17:3) to obtain the entitled compound (49 mg, 20 %).
Mass Spectrum (ESI): 286.0 (M+H).

### (2) Production of 3-[4-(trifluoromethoxy)phenoxy]pyridin-2(1H)one:

With cooling with ice, sodium iodide (2.12 g, 14.2 mmol) was added to an acetonitrile solution (10 mL) of trimethylsilyl chloride (1.76 mL, 13.9 mmol), and stirred at room temperature for 15 minutes, and then an acetonitrile solution(5 ml) of 2-methoxy-3-[4-(trifluoromethoxy)phenoxy]pyridine (794 mg, 2.78 mmol) was added to the above solution, and stirred overnight at room temperature. Aqueous saturated sodium hydrogencarbonate solution was added to the reaction liquid, and extracted with chloroform. The obtained organic layer was dried with anhydrous sodium sulfate, then filtered. The obtained filtrate was concentrated under reduced pressure, then the formed solid was collected by filtration to obtain the entitled compound (443 mg, 59 %).
Mass Spectrum (ESI): 272.0 (M+H).

### Reference Example 13:

### 3-[4-(Trifluoromethyl)phenoxy]pyridin-2(1H)-one:

### (1) Production of 2-methoxy-3-[4-(trifluoromethyl)phenoxy]pyridine:

[4-(Trifluoromethyl)phenyl]boronic acid (668 mg, 3.52 mmol), copper(II) acetate (480 mg, 2.64 mmol), triethylamine (0.74 mL, 5.28 mmol) and Molecular Sieve 4A (1.76 g) were added to a methylene chloride solution (20 mL) of 2-methoxypyridin-3-ol (220 mg, 1.76 mmol), and stirred at room temperature for 2 days. The reaction liquid was filtered through Celite, then aqueous saturated sodium hydrogencarbonate solution was added to the filtrate, and extracted with ethyl acetate. The obtained organic layer was washed with saturated saline, then dried with anhydrous sodium sulfate, and filtered. The obtained filtrate was concentrated under reduced pressure, then the residue was purified by silica gel column chromatography (hexane:ethyl acetate = 10:0 to 9:1) to obtain the entitled compound (298 mg, 63 %).
Mass Spectrum (ESI): 270.0 (M+H).

### (2) Production of 3-[4-(trifluoromethyl)phenoxy]pyridin-2(1H)-one:

In the same manner as in Reference Example 12-(2) but changing the compound used in Reference Example 12-(2) to 2-methoxy-3-[4-(trifluoromethyl)phenoxy]pyridine, the compound of Reference Example 13 was obtained.
Mass Spectrum (ESI): 256.1 (M+H).

### Reference Examples 14, 15:

In the same manner as in Reference Example 13 but changing [4-(trifluoromethyl)phenyl]boronic acid used in Reference Example 13 to corresponding boronic acid compounds, the compounds of Reference Examples 14 and 15 were obtained.

### Reference Example 14:

### 3-(4-Chlorophenoxy)pyridin-2(1H)-one:

Mass Spectrum (ESI): 222.0 (M+H).

### Reference Example 15:

### 3-(biphenyl-4-yloxy)pyridin-2(1 1H)-one:

Mass Spectrum (ESI): 264.1 (M+H).

### Reference Example 16:

### 2-[4-(pyrrolidin-1-ylmethyl)phenyl]ethanol:

### (1) Production of methyl [4-(pyrrolidin-1-ylmethyl)phenyl]acetate:

A mixture of methyl [4-(bromomethyl)phenyl]acetate (0.50 g, 2.10 mmol) [J. Med. Chem., 44(5), 703(2001)], pyrrolidine (0.35 mL, 4.20 mmol), potassium carbonate (1.45 g, 10.50 mmol) and NMP (10 mL) was stirred at room temperature for 2 days. Next, aqueous sodium hydrogencarbonate solution was added to the reaction liquid, and extracted with ethyl acetate. The obtained organic layer was washed with aqueous sodium hydrogencarbonate solution and saturated saline, then dried with anhydrous sodium sulfate, and filtered. The obtained filtrate was concentrated under reduced pressure, then the residue was purified by silica gel column chromatography (methanol:chloroform = 0:10 to 1:19) to obtain the entitled compound (0.30 g, 62 %).
Mass Spectrum (ESI): 234.1 (M+H).

### (2) Production of 2-[4-(pyrrolidin-1-ylmethyl)phenyl]ethanol:

At -78°C, a THF solution (10 mL) of methyl [4-(pyrrolidin-1-ylmethyl)phenyl]acetate (1.15 g, 4.93 mmol) was added to a THF solution (10 mL) of LAH (225 mg, 5.92 mmol), then the reaction liquid was stirred for 5 hours with gradually heating up to room temperature. Excessive sodium sulfate 10-hydrate was gradually added to the reaction liquid, then stirred at room temperature overnight. The reaction liquid was filtered through Celite, then the obtained filtrate was concentrated under reduced pressure to obtain the entitled compound (0.97 g, 95 %).
Mass Spectrum (ESI): 206.1 (M+H).

### Reference Example 17:

### 2-{4-[(diethylamino)methyl]phenyl}ethanol:

In the same manner as in Reference Example 16 but changing pyrrolidine used in Reference Example 16 to diethylamine, the compound of Reference Example 17 was obtained. Mass Spectrum (ESI): 208.1 (M+H).

### Reference Example 18:

### [4-(Pyrrolidin-1-ylmethyl)phenyl]methanol:

In the same manner as in Reference Example 16-(2) but changing methyl [4-(pyrrolidin-1-ylmethyl)phenyl]acetate used in Reference Example 16-(2) to methyl 4-(pyrrolidin-1-ylmethyl)benzoate [J. Med. Chem., 38(2), 234(1995)], the compound of Reference Example 18 was obtained.
Mass Spectrum (ESI): 192.2 (M+H).

### Reference Example 19:

### 2-[2-Fluoro-4-(pyrrolidin-1-ylmethyl)phenyl]ethanol:

### (1) Production of (4-bromo-2-fluorophenyl)acetonitrile:

Tetraethylammonium cyanide (3.8 g, 24.3 mmol) was added to a DMF solution (50 mL) of 4-bromo-1-(bromomethyl)-2-fluorobenzene (5.0 g, 18.7 mmol), and stirred at room temperature for 19.5 hours. Next, aqueous saturated ammonium chloride solution was added to the reaction liquid, and extracted with ethyl acetate. The obtained organic layer was washed with saturated saline, then dried with anhydrous sodium sulfate, and filtered. The obtained filtrate was concentrated under reduced pressure, then the residue was purified by silica gel column chromatography (hexane:ethyl acetate = 10:0 to 9:1) to obtain the entitled compound (2.11 g, 53 %).
Mass Spectrum (APCI): 214.0 (M+H).

### (2) Production of ethyl (4-bromo-2-fluorophenyl)acetate:

Hydrochloric acid (10 mL) was added to an ethanol solution (20 mL) of (4-bromo-2-fluorophenyl)acetonitrile (2.11 g, 9.86 mmol), and heated under reflux for 15 hours. Next, with cooling with ice, aqueous saturated sodium hydrogencarbonate solution was gradually added to the reaction liquid for neutralization. After extracted with ethyl acetate, the obtained organic layer was washed with saturated saline, then dried with anhydrous sodium sulfate, and filtered. The obtained filtrate was concentrated under reduced pressure, then the residue was purified by silica gel column chromatography (hexane:ethyl acetate = 10:0 to 9:1) to obtain the entitled compound (1.81 g, 70 %).
¹HNMR (400 MHz, DMSO-d₆, δ ppm): 1.26 (3H, t, J=7.2 Hz), 3.62 (2H, s), 4.17 (2H, q, J=7.2 Hz), 7.12-7.18 (1H, m), 7.23-7.28 (2H, m).

### (3) Production of 2-(4-bromo-2-fluorophenyl)ethanol:

In the same manner as in Reference Example 16-(2) but changing methyl [4-(pyrrolidin-1-ylmethyl)phenyl]acetate used in Reference Example 16-(2) to ethyl (4-bromo-2-fluorophenyl)acetate, the compound of Reference Example 19-(3) was obtained.
¹HNMR (400 MHz, CDCl₃, δ ppm): 2.85-2.90 (2H, m), 3.80-3.90 (2H, m), 7.10-7.16 (1H, m), 7.20-7.27 (2H, m).

### (4) Production of [2-(4-bromo-2-fluorophenyl)ethoxy](tert-butyl)dimethylsilane:

With cooling with ice, tert-butyl(chloro)dimethylsilane (678 mg, 4.5 mmol) and imidazole (306 mg, 4.5 mmol) were added to a THF solution (10 mL) of 2-(4-bromo-2-fluorophenyl)ethanol (894 mg, 4.1 mmol), and stirred at room temperature for 4 hours. Aqueous saturated ammonium chloride solution was added to the reaction liquid, and extracted with ethyl acetate. The obtained organic layer was washed with saturated saline, dried with anhydrous sodium sulfate, and filtered. The obtained filtrate was concentrated under reduced pressure, then the residue was purified by silica gel column chromatography (hexane:ethyl acetate = 10:0 to 9:1) to obtain the entitled compound (945 mg, 69 %).
¹HNMR (400 MHz, CDCl₃, δ ppm): -0.032 (6H, s), 0.85 (9H, s), 2.77-2.85 (2H, m), 3.78 (2H, t, J=6.8 Hz), 7.10 (1H, t, J=8.8 Hz), 7.17-7.22 (2H, m).

### (5) Production of propyl 4-(2- {[tert-butyl(dimethyl)silyl]oxy}ethyl)-3-fluorobenzoate:

Palladium acetate (63 mg, 0.28 mmol), bis(diphenylphosphino)ferrocene (310 mg, 0.56 mmol) and triethylamine (0.79 mL, 5.7 mmol) were added to a n-propanol solution (10 mL) of the compound obtained in the above (4) (945 mg, 2.84 mmol), and stirred in the presence of carbon monoxide at 100°C for 15 hours. The reaction liquid was concentrated under reduced pressure, then ethyl acetate was added, and filtered. The obtained filtrate was washed with aqueous saturated sodium hydrogencarbonate solution and saturated saline, then dried with anhydrous sodium sulfate, and filtered. The obtained filtrate was concentrated under reduced pressure, then the residue was purified by silica gel column chromatography (hexane:ethyl acetate = 10:0 to 9:1) to obtain the entitled compound (817 mg, 85 %).
¹HNMR (400 MHz, CDCl₃, δ ppm): -0.04 (6H, s), 0.85 (9H, s), 1.03 (3H, t, J=7.2 Hz), 1.79 (2H, qt, J=7.2, 6.4 Hz), 2.91 (2H, brd, J=6.4 Hz), 3.83 (2H, t, J=6.8 Hz), 4.27 (2H, t, J=6.8 Hz), 7.30 (1H, t, J=7.8 Hz), 7.67 (1H, dd, J=10.4, 1.6 Hz), 7.75 (1H, dd, J=7.8, 1.6 Hz).

### (6) Production of [4-(2- {[tert-butyl(dimethyl)silyl]oxy}ethyl)-3-fluorophenyl]methanol:

In the same manner as in Reference Example 16-(2) but changing the compound used in Reference Example 16-(2) to propyl 4-(2-{[tert-butyl(dimethyl)silyl]oxy}ethyl)-3-fluorobenzoate, the compound of Reference Example 19-(6) was obtained.
¹HNMR (400 MHz, CDCl₃, δ ppm): -0.02 (6H, s), 0.86 (9H, s), 2.85 (2H, t, J=7.0 Hz), 3.80 (2H, t, J=7.0 Hz), 4.66 (2H, s), 7.04 (2H, d, J=9.2 Hz), 7.21 (1H, t, J=8.0 Hz).

### (7) Production of 1-[4-(2-{[tert-butyl(dimethyl)silyl]oxy}ethyl)-3-fluorobenzyl]pyrrolidine:

With cooling with ice, triethylamine (0.40 mL, 2.88 mmol) and methanesulfonyl chloride (0.20 mL, 2.64 mmol) were added to an ethyl acetate solution (10 mL) of the compound obtained in the above (6) (685 mg, 2.40 mmol), and stirred at room temperature for 1 hour. The reaction liquid was filtered, then the filtrate was concentrated under reduced pressure. Next, in the same manner as in Reference Example 16-(1) but changing methyl [4-(bromomethyl)phenyl]acetate used in Reference Example 16-(1) to the obtained residue, the compound of Reference Example 19-(7) was obtained.
¹HNMR (400 MHz, CDCl₃, δ ppm): -0.03 (6H, s), 0.85 (9H, s), 1.75-1.80 (4H, m), 2.45-2.52 (4H, m), 2.84 (2H, t, J=6.6 Hz), 3.57 (2H, s), 3.79 (2H, t, J=6.6 Hz), 7.00 (2H, d, J=9.2 Hz), 7.14 (1H, t, J=7.2 Hz).

### (8) Production of 2-[2-fluoro-4-(pyrrolidin-1-ylmethyl)phenyl]ethanol:

With cooling with ice, tetrabutylammonium fluoride (1.0 M THF solution, 2.2 mL) was added to a THF solution (10 mL) of the compound obtained in the above (7), and stirred at room temperature for 1 hour. Aqueous saturated sodium hydrogencarbonate solution was added to the reaction liquid, then extracted with ethyl acetate. The obtained organic layer was washed with saturated saline, then dried with anhydrous sodium sulfate, and filtered. The obtained filtrate was concentrated under reduced pressure, then the residue was purified by silica gel column chromatography (chloroform:methanol = 10:0 to 9:1) to obtain the entitled compound (274 mg, 66 %).
¹HNMR (400 MHz, CDCl₃, δ ppm): 1.76-1.82 (4H, m), 2.47-2.54 (4H, m), 2.90 (2H, t, J=6.8 Hz), 3.58 (2H, s), 3.85 (2H, t, J=6.8 Hz), 7.04 (2H, d, J=9.2 Hz), 7.17 (1H, t, J=7.2 Hz). Reference Example 20:

### 2-[4-(1,3-Dioxan-2-ylmethyl)phenyl]ethyl methanesulfonate:

With cooling with ice, triethylamine (0.78 mL, 7.84 mmol) and methanesulfonyl chloride (0.56 mL, 7.18 mmol) were added to an ethyl acetate solution (20 mL) of 2-[4-(1,3-dioxan-2-ylmethyl)phenyl]ethanol (1.36 g, 6.53 mmol), and stirred overnight at room temperature. The reaction liquid was filtered, then the obtained filtrate was concentrated under reduced pressure and without purification, it was dried to obtain the entitled compound, which was used in the next reaction.

### Reference Example 21:

### 2-[4-({[Tert-butyl(dimethyl)silyl]oxy}methyl)ethyl] 4-methylbenzenesulfonate:

### (1) Production of methyl [4-({[tert-butyl(dimethyl)silyl]oxy}methyl)phenyl]acetate:

In the same manner as in Referential Example 19-(4) but changing methyl 2-(4-bromo-2-fluorophenyl)ethanol used in Reference Example 19-(4) to methyl [4-(hydroxymethyl)phenyl]acetate, the compound of Reference Example 21-(1) was obtained. ¹HNMR (400 MHz, CDCl₃, δ ppm): 0.03 (6H, s), 0.84 (9H, s), 3.59 (2H, s), 3.77 (3H, s), 4.63 (2H, s), 7.14 (2H, d, J=8.0 Hz), 7.18 (2H, d, J=8.0 Hz).

### (2) Production of 2-[4-({[Tert-butyl(dimethyl)silyl]oxy}methyl)phenyl]ethanol:

The same process as in Referential Example 16-(2) but changing methyl [4-(pyrrolidin-1-ylmethyl)phenyl]acetate used in Reference Example 16-(2) to methyl [4-({[tert-butyl(dimethyl)silyl]oxy}methyl)phenyl]acetate was carried out, and without purification, the product was used in the next reaction.

### (3) Production of 2-[4-({[tert-butyl(dimethyl)silyl]oxy}methyl)ethyl] 4-methylbenzenesulfonate:

4-Toluenesulfonyl chloride (8.9 g, 46.9 mmol), triethylamine (6.5 mL, 46.9 mmol) and 4-dimethylaminopyridine (476 mg, 3.9 mmol) were added to a chloroform solution of the compound obtained in the above (2) (10.42 g, 39.10 mmol), and stirred overnight at room temperature. Aqueous potassium carbonate solution was added to the reaction liquid, and extracted with chloroform. The obtained organic layer was washed with aqueous ammonium chloride solution and saturated saline, dried with anhydrous sodium sulfate, and filtered. The obtained filtrate was concentrated under reduced pressure, and the residue was purified by silica gel column chromatography (hexane:ethyl acetate = 10:0 to 17:3) to obtain the entitled compound (14.64 g, 89 %).
¹HNMR (400 MHz, CDCl₃, δ ppm): 0.10 (6H, s), 0.94 (9H, s), 2.43 (3H, s), 2.94 (2H, t, J=7.2 Hz), 4.19 (2H, t, J=7.2 Hz), 4.70 (2H, s), 7.07 (2H, d, J=8.0 Hz), 7.20 (2H, d, J=8.0 Hz), 7.28 (2H, d, J=8.6 Hz), 7.68 (2H, d, J=8.6 Hz).

### Reference Example 22:

### 2-[6-{[(Tert-butyl(dimethyl)silyl)oxy]methyl}pyridin-3-yl]ethyl 4-methylbenzenesulfonate:

In the same manner as in Reference Example 21 but changing methyl [4-(hydroxymethyl)phenyl]acetate used in Reference Example 21-(1) to methyl [6-hydroxymethyl)pyridin-3-yl]acetate (JP-A 48-099178), the compound of Reference Example 22 was obtained.
Mass Spectrum (ESI): 422.2 (M+H).

### Reference Example 23:

### Tert-butyl 2-[4-(2- {[(4-methylphenyl)sulfonyl]oxy}ethyl)phenyl]pyrrolidine-1-carboxylate:

### (1) Production of tert-butyl 2-[4-(2-hydroxyethyl)phenyl]-1H-pyrrole-1-carboxylate:

[1-(Tert-butoxycarbonyl)-1H-pyrrol-2-yl]boronic acid (789 mg, 3.74 mmol), dichlorobis(triphenylphosphine)palladium (175 mg, 0.25 mmol) and aqueous 2 M sodium carbonate solution (4 mL) were added to a THF solution (5 mL) of 2-(4-bromophenyl)ethanol (500 mg, 2.49 mmol), and stirred at 80°C for 4 hours in the presence of nitrogen. Next, aqueous saturated sodium hydrogencarbonate solution was added to the reaction liquid, and extracted with ethyl acetate. The obtained organic layer was washed with saturated saline, then dried with anhydrous sodium sulfate, and filtered. The obtained filtrate was concentrated under reduced pressure, and the residue was purified by silica gel column chromatography (hexane:ethyl acetate = 10:0 to 7:3) to obtain the entitled compound (519 mg, 73 %).
Mass Spectrum (ESI): 288.2 (M+H).

### (2) Production of Tert-butyl 2-[4-(2-hydroxyethyl)phenyl]pyrrolidine-1-carboxylate:

An aqueous solution (20 mL) of platinum (30 wt. % activated charcoal, 2.0 g) was added to an ethanol solution of the compound obtained in the above (1) (6.67 g, 23.0 mmol), and stirred at 70°C for 1.5 days under hydrogen pressure (60 kgf/cm²). Next, the reaction liquid was filtered, and the obtained filtrate was concentrated under reduced pressure. The obtained residue was purified by silica gel column chromatography (hexane:ethyl acetate = 10:0 to 1:1) to obtain the entitled compound (6.02 g, 90 %).
Mass Spectrum (ESI): 292.2 (M+H).

### (3) Production of tert-butyl 2-[4-(2-{[(4-methylphenyl)sulfonyl]oxy}ethyl)phenyl]pyrrolidine-1-carboxylate:

In the same manner as in Reference Example 21-(3) but exchanging 2-[4-({[tert-butyl(dimethyl)silyl]oxy}methyl)phenyl]ethanol used in Reference Example 21-(3) to tert-butyl 2-[4-(2-hydroxyethyl)phenyl]pyrrolidine-1-carboxylate, the compound of Reference Example 23 was obtained.
Mass Spectrum (ESI): 446.2 (M+H).

### Reference Example 24:

### 2-(4{[(Tert-butoxycarbonyl)(methyl)amino]methyl}phenyl)ethyl 4-methylbenzenesulfonate:

### (1) Production of tert-butyl methyl(4-vinylbenzyl)methylcarbamate:

Tributyl(vinyl)stannane (2.34 mL, 7.99 mmol) and tetrakis(triphenylphosphine)palladium (385 mg, 0.33 mmol) were added to a DMF solution (20 mL) of tert-butyl (4-bromobenzyl)methylcarbamate (2.0 g, 6.66 mmol) (WO00/014109), and stirred overnight at 80°C in the presence of nitrogen. Saturated saline was added to the reaction liquid, then extracted with ether. Aqueous 5 wt.% sodium fluoride solution was added to the obtained organic layer, then stirred at room temperature for 4 hours. Next, water was added to the reaction liquid, and extracted with ethyl acetate. The obtained organic layer was washed with water and saturated saline, then dried with anhydrous sodium sulfate, and filtered. The filtrate was concentrated under reduced pressure, and the obtained residue was purified by silica gel column chromatography (hexane:ethyl acetate = 10:0 to 17:3) to obtain the entitled compound (1.39 g, 84 %).
¹HNMR (400 MHz, CDCl₃, δ ppm): 1.54 (9H, s), 2.70-2.90 (3H, m), 4.41 (2H, s), 5.23 (1H, d, J=10.6 Hz), 5.74 (1H, d, J=17.6 Hz), 6.70 (1H, dd, J=17.6, 10.6 Hz), 7.10-7.24 (2H, m), 7.37 (2H, d, J=8.0 Hz).

### (2) Production of tert-butyl [4-(2-hydroxyethyl)benzyl]methylcarbamate:

9-BBN (0.5 M THF solution, 33.6 mL) was added to a THF solution (26 mL) of the compound obtained in the above (1) (1.39 g, 5.60 mmol), and stirred at room temperature for 3 days. Next, the reaction liquid was cooled to -30°C, 30 % hydrogen peroxide water (1.9 mL) was gradually added, and thereafter aqueous 3 N sodium hydroxide solution (3.0 mL) was added, and stirred at -30°C to room temperature for 7 hours. Water was added to the reaction liquid, and extracted with ether. The obtained organic layer was washed with water and saturated saline, then dried with anhydrous sodium sulfate, and filtered. The obtained filtrate was concentrated under reduced pressure, then the residue was purified by silica gel column chromatography (hexane:ethyl acetate = 10:0 to 1:1) to obtain the entitled compound (943 mg, 63 %).
¹HNMR (400 MHz, CDCl₃, δ ppm): 1.48 (9H, s), 2.70-3.00 (5H, m), 3.80-3.90 (2H, m), 4.38 (2H, s), 7.10-7.20 (4H, m).

### (3) Production of 2-(4- {[(tert-butoxycarbonyl)(methyl)amino]methyl}phenyl)ethyl 4-methylbenzenesulfonate:

In the same manner as in Reference Example 21-(3) but changing 2-[4-({[tert-butyl(dimethyl)silyl]oxy}methyl)phenyl]ethanol used in Reference Example 21-(3) to tert-butyl [4-(2-hydroxyethyl)benzyl]methylcarbamate, the compound of Reference Example 24 was obtained.
¹HNMR (400 MHz, CDCl₃,δ ppm): 1.48 (9H, s), 2.44 (3H, s), 2.70-2.85 (3H, m), 2.94 (2H, t, J=7.0 Hz), 4.20 (2H, t, J=7.0 Hz), 4.38 (2H, s), 7.07 (2H, d, J=8.0 Hz), 7.12 (2H, d, J=8.0 Hz), 7.29 (2H, d, J=8.0 Hz), 7.70 (2H, d, J=8.0 Hz).

### Reference Example 25:

### 2-(4- {[(tert-butoxycarbonyl)(propyl)amino]methyl} phenyl)ethyl 4-methylbenzenesulfonate:

In the same manner as in Reference Example 24 but changing tert-butyl (4-bromobenzyl)methylcarbamate used in Reference Example 24 to tert-butyl [4-(2-hydroxyethyl)benzyl]propylcarbamate (WO93/18035), the compound of Reference Example 25 was obtained.
¹HNMR (400 MHz, CDCl₃, δ ppm): 0.84 (3H, t, J=7.6 Hz), 1.30-1.80 (11H, m), 2.44 (3H, s), 2.94 (2H, t, J=7.0 Hz), 3.00-3.20 (2H, m), 4.19 (2H, t, J=7.0 Hz), 4.30-4.50 (2H, m), 7.06 (2H, d, J=7.8 Hz), 7.12 (2H, d, J=7.8 Hz), 7.29 (2H, d, J=8.0 Hz), 7.65-7.75 (2H, m).

### Reference Example 26:

### 2-(3-Fluoro-4- {[(4-methoxybenzyl)oxy]methyl}phenyl)ethyl 4-methylbenzenesulfonate:

### (1) Production of 4-bromo-2-fluoro-1-{[(4-methoxybenzyl)oxy]methyl}benzene:

With cooling with ice, sodium hydride (60 % oily, 164 mg, 4.1 mmol) and (4-methoxyphenyl)methanol (0.51 mL, 4.10 mmol) were added to a DMF solution (10 mL) of 4-bromo-1-(bromomethyl)-2-fluorobenzene (1.0 g, 3.73 mmol), and stirred overnight at 0°C to room temperature. Next, aqueous saturated sodium hydrogencarbonate solution was added to the reaction liquid, and extracted with ethyl acetate. The obtained organic layer was washed with saturated saline, dried with anhydrous sodium sulfate, and filtered. The obtained filtrate was concentrated under reduced pressure, and the residue was purified by silica gel column chromatography (hexane:ethyl acetate = 10:0 to 9:1) to obtain the entitled compound (1.11 g, 91 %).
¹HNMR (400 MHz, CDCl₃, δ ppm): 3.82 (3H, s), 4.51 (2H, s), 4.53 (2H, s), 6.87-6.92 (2H, m), 7.20-7.35 (5H, m).

### (2) Production of 2-(3-fluoro-4- {[(4-methoxybenzyl)oxy]methyl}phenyl)ethyl 4-methylbenzenesulfonate:

In the same manner as in Reference Example 24 but changing tert-butyl (4-bromobenzyl)methylcarbamate used in Reference Example 24-(1) to 4-bromo-2-fluoro-1-{[(4-methoxybenzyl)oxy]methyl}benzene, the compound of Reference Example 26 was obtained. ¹HNMR (400 MHz, CDCl₃, δ ppm): 2.42 (3H, s), 2.93 (2H, t, J=6.8 Hz), 3.81 (3H, s), 4.20 (2H, t, J=6.8 Hz), 4.51 (2H, s), 4.54 (2H, s), 6.74-6.80 (1H, m), 6.86-6.94 (3H, m), 7.25-7.35 (5H, m), 7.67 (2H, d, J=8.4 Hz).

### Reference Examples 27 to 29:

In the same manner as in Reference Example 24 but changing tert-butyl (4-bromobenzyl)methylcarbamate used in Reference Example 24 to corresponding known compounds, the compounds of Reference Examples 27 to 29 were obtained.

### Reference Example 27:

### 2-{4-[1-(methoxymethoxy)ethyl]phenyl}ethyl4-methylbenzenesulfonate:

¹HNMR (400 MHz, CDCl₃, δ ppm): 1.45 (3H, d, J=6.2 Hz), 2.43 (3H, s), 2.95 (2H, t, J=7.2 Hz), 3.37 (3H, s), 4.30 (2H, t, J=7.2 Hz), 4.51 (1H, d, J=6.6 Hz), 4.56 (1H, d, J=6.6 Hz), 4.72 (1H, q, J=6.2 Hz), 7.09 (2H, d, J=7.8 Hz), 7.23 (2H, d, J=7.8 Hz), 7.29 (2H, d, J=8.4 Hz), 7.71 (2H, d, J=8.4 Hz).

### Reference Example 28:

### Methyl 2-methoxy-4-(2-{[(4-methylphenyl)sulfonyl]oxy}ethyl)benzoate:

Mass Spectrum (ESI): 365.1 (M+H).

### Reference Example 29:

### Methyl 3 -methoxy-4-(2-{[(4-methylphenyl)sulfonyl]oxy}ethyl)benzoate:

Mass Spectrum (ESI): 365.0 (M+H).

### Reference Example 30:

### 2-{5-[(methoxymethoxy)methyl]pyridin-2-yl}ethyl 4-methylbenzenesulfonate

In the same manner as in Reference Example 21-(3) but changing 2-[4-({[tert-butyl(dimethyl)silyl]oxy}methyl)phenyl]ethanol used in Reference Example 21-(3) to 2-{5-[(methoxymethoxy)methyl]pyridin-2-yl)ethanol [J. Med. Chem., 39, 5050, (1996)], the compound of Reference Example 30 was obtained.
Mass Spectrum (ESI): 352.1(M+H).

### Reference Example 31:

### Ethyl 4-(1,1-difluoro-2- {[(4-methylphenyl)sulfbnyl]oxy} ethyl)benzoate:

### (1) Production of ethyl 4-(2-ethoxy-1,1-difluoro-2-oxoethyl)benzoate:

Ethyl bromo(difluoro)acetate (1.30 mL, 9.85 mL) and copper powder (4.04 g, 27.6 mmol) were added to a DMSO solution (20 mL) of ethyl 4-iodobenzoate (1.43 mL, 9.85 mmol), and stirred at 55°C for 2 days. Next, aqueous saturated ammonium chloride solution was added to the reaction liquid, and extracted with ethyl acetate. The organic layer was washed with aqueous ammonium chloride solution and saturated saline, dried with anhydrous sodium sulfate, and filtered. The obtained filtrate was concentrated under reduced pressure, and the residue was purified by silica gel column chromatography (hexane:ethyl acetate = 10:0 to 9:1) to obtain the entitled compound (1.48 g, 55 %).
¹HNMR (400 MHz, CDCl₃, δ ppm): 1.30 (3H, t, J=7.2 Hz), 1.39 (3H, t, J=7.2 Hz), 4.30 (2H, q, J=7.2 Hz), 4.40 (2H, q, J=7.2 Hz), 7.69 (2H, d, J=8.4 Hz), 8.13 (2H, d, J=8.4 Hz).

### (2) Production of ethyl 4-(1,1-difluoro-2-hydroxyethyl)benzoate:

With cooling with ice, sodium borohydride was added to a methanol solution (20 mL) of the compound obtained in the above (1) (1.48 g, 5.44 mmol), and stirred at 0°C to room temperature for 2 hours. Next, saturated saline was added to the reaction liquid, and extracted with ethyl acetate. The obtained organic layer was dried with anhydrous sodium sulfate, and filtered. The obtained filtrate was concentrated under reduced pressure, and the residue was purified by silica gel column chromatography (hexane:ethyl acetate = 10:0 to 7:3) to obtain the entitled compound (1.11 g, 89 %).
¹HNMR (400 MHz, CDCl₃, δ ppm): 1.41 (3H, t, J=7.2 Hz), 1.97 (1H, t, J=7.0 Hz), 3.99 (2H, dt, J=7.0, 13.2 Hz), 4.40 (2H, q, J=7.2 Hz), 7.60 (2H, d, J=8.8 Hz), 8.12 (2H, d, J=8.8 Hz).

### (3) Production of ethyl 4-(1,1-difluoro-2-{[(4-methylphenyl)sulfonyl]oxy}ethyl)benzoate:

In the same manner as in Reference Example 21-(3) but changing 2-[4-({[tert-butyl(dimethyl)silyl]oxy}methyl)phenyl]ethanol used in Reference Example 21-(3) to ethyl 4-(1,1-fluoro-2-hydroxyethyl)benzoate, the compound of Reference Example 31 was obtained.
¹HNMR (400 MHz, CDCl₃, δ ppm): 1.42 (3H, t, J=7.0 Hz), 2.45 (3H, s), 4.37 (2H, t, J=11.2 Hz), 4.41 (2H, q, J=7.0 Hz), 7.28 (2H, d, J=7.8 Hz), 7.46 (2H, d, J=7.8 Hz), 7.62-7.70 (2H, m), 8.05 (2H, d, J=8.4 Hz).

### Reference Example 32:

### 2-Fluoro-2- {4-[(methoxymethoxy)methyl]phenyl}ethanol:

### (1) Production of methyl (4-bromophenyl)(fluoro)acetate:

With cooling with ice, [bis(2-methoxyethyl)amino]sulfur trifluoride (6.0 mL, 32.3 mmol) was gradually added to a methylene chloride solution (50 mL) of methyl (4-bromophenyl)(hydroxy)acetate (5.27 g, 21.5 mmol) [Canadian Journal of Chemistry (1990), 68(2), 314-16], and stirred at 0°C to room temperature for 9 hours. With cooling with ice, aqueous saturated sodium hydrogencarbonate solution was added to the reaction liquid, and extracted with chloroform. The obtained organic layer was dried with anhydrous sodium sulfate, and filtered. The obtained filtrate was concentrated under reduced pressure, then the residue was purified by silica gel column chromatography (hexane:ethyl acetate = 10:0 to 8:2) to obtain the entitled compound (4.03 g, 72 %).
¹HNMR (400 MHz, CDCl₃, δ ppm): 3.79 (3H, s), 5.76 (1H, d, J=47.2 Hz), 7.34 (2H, d, J=8.4 Hz), 7.55 (2H, d, J=8.4 Hz).

### (2) Production of 2-(4-bromophenyl)-2-fluoroethanol:

In the same manner as in Reference Example 16-(2) but changing methyl [4-(pyrrolidin-1-ylmethyl)phenyl]acetate used in Reference Example 16-(2) to methyl (4-bromophenyl)(fluoro)acetate, the compound of Reference Example 32-(2) was obtained.
¹HNMR (400 MHz, CDCl₃, δ ppm): 1.90-2.00 (1H, m), 3.74-3.98 (2H, m), 5.51 (1H, ddd, J=48.4, 7.6, 3.6 Hz), 7.23 (2H, d, J=8.2 Hz), 7.53 (2H, d, J=8.2 Hz).

### (3) Production of [4-(2- {[tert-butyl(dimethyl)silyl]oxy}-1-fluoroethyl)phenyl]methanol:

In the same manner as in Reference Example 19-(4) to 19-(6) but changing 2-(4-bromo-2-fluorophenyl)ethanol used in Reference Example 19-(4) to 2-(4-bromophenyl)-2-fluoroethanol, the compound of Reference Example 32-(3) was obtained.
¹HNMR (400 MHz, CDCl₃, δ ppm): 0.04 (3H, s), 0.06 (3H, s), 0.90 (9H, s), 3.75-3.95 (2H, m), 4.71 (2H, d, J=5.2 Hz), 5.48 (1H, ddd, J=48.0, 7.2, 3.2 Hz), 7.30-7.40 (4H, m).

### (4) Production of tert-butyl (2-fluoro-2-{4-[(methoxymethoxy)methyl]phenyl}ethoxy)dimethylsilane:

With cooling with ice, chloromethyl methyl ether (0.26 mL, 3.40 mmol) and N,N-diisopropylethylamine 0.73 mL, 4.20 mmol) were added to a THF solution (10 mL) of the compound obtained in the above (3) (799 mg, 2.80 mmol), and stirred overnight at 0°C to room temperature. Next, aqueous saturated ammonium chloride solution was added to the reaction liquid, and extracted with ethyl acetate. The obtained organic layer was dried with anhydrous sodium sulfate, and filtered. The obtained filtrate was concentrated under reduced pressure, and the residue was purified by silica gel column chromatography (hexane:ethyl acetate = 10:0 to 9:1) to obtain the entitled compound (483 mg, 53 %).
¹HNMR (400 MHz, CDCl₃, δ ppm): 0.02 (3H, s), 0.03 (3H, s), 0.86 (9H, s), 3.39 (3H, s), 3.72-3.92 (2H, m), 4.58 (2H, s), 4.68 (2H, s), 5.45 (1H, ddd, J=48.4, 7.6, 3.6 Hz), 7.28-7.38 (4H, m).

### (5) Production of 2-fluoro-2-{4-[(methoxymethoxy)methyl]phenyl}ethanol:

In the same manner as in Reference Example 19-(8) but changing 1-[4-(2-{[tert-butyl(dimethyl)silyl]oxy}ethyl)-3-fluorobenzyl]pyrrolidine used in Reference Example 19-(8) to tert-butyl (2-fluoro-2-{4-[(methoxymethoxy)methyl]phenyl}ethoxy)dimethylsilane, the compound of Reference Example 32 was obtained.
¹HNMR (400 MHz, CDCl₃, δ ppm): 3.42 (3H, s), 3.75-4.00 (2H, m), 4.60 (2H, s), 4.71 (2H, s), 5.57 (1H, ddd, J=48.8, 7.6, 2.8 Hz), 7.34 (2H, d, J=8.0 Hz), 7.39 (2H, d, J=8.0 Hz).

### Reference Examples 33, 34:

In the same manner as in Reference Example 20 but changing 2-[4-(1,3-dioxan-2-ylmethyl)phenyl]ethanol used in Reference Example 20 to corresponding known compounds, the compounds of Reference Examples 33 and 34 were obtained.

### Reference Example 33:

### Methyl (6-{[methylsulfonyl)oxy]methyl}pyridin-3-yl)acetate:

Mass Spectrum (ESI): 260.1 (M+H).

### Reference Example 34:

### 4-({[Tert-butyl(dimethyl)silyl]oxy}methyl)benzyl methanesulfonate:

¹HNMR (400 MHz, CDCl₃,δ ppm): 0.10 (6H, s), 0.94 (9H, s), 2.89 (3H, s), 4.76 (2H, s), 5.24 (2H, s), 7.42-7.33 (4H, m).

### Example 1:

### 4-[(4-Fluorobenzyl)oxy]-1-{2-[4-(pyrrolidin-1-ylmethyl)phenyl]ethyl}pyridin-2(1H)-one:

The compound of Reference Example 2 (90 mg, 0.41 mmol), the compound of Reference Example 16 (100 mg, 0.49 mmol), triphenyl phosphine (163 mg, 0.62 mmol) and diisopropyl azodicarboxylate (0.12 mL, 0.62 mmol) were stirred in methylene chloride (10 mL) for 1 day and a half. Aqueous sodium hydrogencarbonate solution was added to the reaction liquid, then extracted with ethyl acetate. The obtained organic layer was washed with saturated saline, then dried with anhydrous sodium sulfate, and filtered. The obtained filtrate was concentrated under reduced pressure, and the residue was purified by silica gel column chromatography (methanol:chloroform = 1:19) to obtain the entitled compound (8.7 mg, 4 %). ¹HNMR (400 MHz, CDCl₃, δ ppm): 1.75-1.82 (4H, m), 2.44-2.56 (4H, m), 3.01 (2H, t, J=7.0 Hz), 3.59 (2H, s), 4.07 (2H, t, J=7.0 Hz), 4.94 (2H, s), 5.75 (1H, dd, J=7.4, 2.7 Hz), 5.98 (1H, d, J=2.7 Hz), 6.75 (1H, d, J=7.4 Hz), 7.05-7.12 (4H, m), 7.22-7.28 (2H, m), 7.34-7.40 (2H, m).

### Examples 2 to 13:

In the same manner as in Example 1 but changing 4-[(4-fluorobenzyl)oxy]pyridin-2(1H)-one and 2-[4-(pyrrolidin-1-ylmethyl)phenyl]ethanol used in Example 1 to corresponding compounds of Reference Examples or known compounds, the compounds of Examples 2 to 13 were obtained.

### Example 2:

### 1-(2- {4-[(Diethylamino)methyl]phenyl}ethyl)-4-[(4-fluorobenzyl)oxy]pyridin-2(1H)one:

¹HNMR (400 MHz, CDCl₃, δ ppm): 1.03 (6H, t, J=7.0 Hz), 2.50 (4H, q, J=7.0 Hz), 3.01 (2H, t, J=7.0 Hz), 3.53 (2H, s), 4.07 (2H, t, J=7.0 Hz), 4.94 (2H, s), 5.75 (1H, dd, J=7.4, 2.7 Hz), 5.98 (1H, d, J=2.7 Hz), 6.74 (1H, d, J=7.4 Hz), 7.06-7.10 (4H, m), 7.22-7.27 (2H, m), 7.37 (2H, dd, J=8.8, 5.3 Hz).

### Example 3:

### 4-[(4-Fluorobenzyl)oxy]-1-{[4-(pyrrolidin-1-ylmethyl)benzyl]oxy}pyridin-2(1H)one:

¹HNMR (400 MHz, CDCl₃, δ ppm): 1.75-1.83 (4H, m), 2.45-2.55 (4H, m), 3.61 (2H, s), 4.90 (2H, s), 5.22 (2H, s), 5.64 (1H, dd, J=7.8, 3.1 Hz), 6.04 (1H, d, J=3.1 Hz), 6.94 (1H, d, J=8.2 Hz), 7.07 (2H, t, J=8.8 Hz), 7.35-7.37 (6H, m).

### Example 4:

### 4-[(4-Fluorobenzyl)oxy]-1-{2-[2-fluoro-4-(pyrrolidin-1-ylmethyl)phenyl]ethyl}pyridin-2(1H)-one:

¹HNMR (400 MHz, CDCl₃, δ ppm): 1.74-1.84 (4H, m), 2.44-2.56 (4H, m), 3.06 (2H, t, J=7.0 Hz), 3.58 (2H, s), 4.08 (2H, t, J=7.0 Hz), 4.93 (2H, s), 5.76 (1H, dd, J=7.4, 2.7 Hz), 5.97 (1H, d, J=2.7 Hz), 6.80 (1H, d, J=7.4 Hz), 7.02-7.10 (5H, m), 7.34-7.40 (2H, m).

### Example 5:

### 1-(2-[4-(Dimethylamino)phenyl]ethyl}-4-[(4-fluorobenzyl)oxy]pyridin-2(1H)-one:

¹HNMR (400 MHz, CDCl₃, δ ppm): 2.89-2.95 (8H, m), 4.03 (2H, t, J=7.0 Hz), 4.94 (2H, s), 5.78 (1H, dd, J=7.4, 2.7 Hz), 5.99 (1H, d, J=2.7 Hz), 6.64-6.70 (2H, m), 6.78 (1H, d, J=7.4 Hz), 6.98-7.03 (2H, m), 7.05-7.13 (2H, m), 7.33-7.40 (2H, m).

### Example 6:

### 4-[(5-Fluoropyridin-2-yl)methoxy]-1-{2-[2-fluoro-4-(pyrrolidin-1-ylmethyl)phenyl]ethyl}pyridin-2(1H)-one:

¹HNMR (400 MHz, CDCl₃, δ ppm): 1.74-1.84 (4H, m), 2.44-2.54 (4H, m), 3.05 (2H, t, J=6.9 Hz), 3.57 (2H, s), 4.08 (2H, t, J=6.9 Hz), 5.09 (2H, s), 5.82 (1H, dd, J=7.6, 2.7 Hz), 5.97 (1H, d, J=2.7 Hz), 6.82 (1H, d, J=7.6 Hz), 6.97-7.07 (3H, m), 7.44 (2H, dd, J=6.2, 2.0 Hz), 8.45-8.48 (1H, s).

### Example 7:

### 1-(2- {4-[(Diethylamino)methyl]phenyl]ethyl}-4-[(5-fluoropyridin-2-yl)methoxy]pyridin-2(1H)-one:

¹HNMR (400 MHz, CDCl₃, δ ppm): 1.04 (6H, t, J=7.0 Hz), 2.52 (4H, q, J=7.0 Hz), 3.00 (2H, t, J=7.0 Hz), 3.54 (2H, s), 4.07 (2H, t, J=7.0 Hz), 5.09 (2H, s), 5.80 (1H, dd, J=7.8, 2.7 Hz), 5.98 (1H, d, J=2.7 Hz), 6.75 (1H, d, J=7.8 Hz), 7.08 (2H, d, J=8.2 Hz), 7.22-7.28 (2H, m), 7.45 (2H, dd, J=6.1, 1.8 Hz), 8.45-8.48 (1H, m).

### Example 8:

### 4-(Benzyloxy)-1-(2-{4-[(diethylamino)methyl]phenyl}ethyl)pyrimidin-2(1H)-one:

¹HNMR (400 MHz, CDCl₃, δ ppm): 1.03 (6H, t, J=7.1 Hz), 2.49 (4H, t, J=7.1 Hz), 3.06 (2H, t, J=6.8 Hz), 3.53 (2H, s), 4.06 (2H, t, J=6.8 Hz), 5.41 (2H, s), 5.67 (1H, d, J=7.0 Hz), 6.92 (1H, d, J=7.0 Hz), 7.08 (2H, d, J=7.8 Hz), 7.23-7.28 (2H, m), 7.33-7.45 (5H, m).

### Example 9:

### 1-(2- {4-[(Diethylamino)methyl]phenyl}ethyl)-3-(4-phenoxyphenyl)pyridin-2(1H)-one:

¹HNMR (400 MHz, CDCl₃, δ ppm): 1.04 (6H, t, J=7.3 Hz), 2.51 (4H, q, J=7.3 Hz), 3.09 (2H, t, J=7.3 Hz), 3.54 (2H, s), 4.20 (2H, t, J=7.3 Hz), 6.11 (1H, t, J=6.7 Hz), 6.98 (1H, dd, J=6.7, 2.2 Hz), 7.03-7.15 (7H, m), 7.22-7.28 (2H, m), 7.32-7.36 (2H, m), 7.45 (1H, dd, J=7.3, 2.0 Hz), 7.70 (2H, td, J=5.9, 3.4 Hz).

### Example 10:

### 3-(4-Chlorophenoxy)-1-(2- {4-[(diethylamino)methyl]phenyl}ethyl)pyridin-2(1H)-one:

¹HNMR (400 MHz, CDCl₃, δ ppm): 1.03 (6H, t, J=7.2 Hz), 2.50 (4H, q, J=7.2 Hz), 3.06 (2H, t, J=7.3 Hz), 3.53 (2H, s), 4.19 (2H, t, J=7.3 Hz), 5.95 (1H, t, J=7.0 Hz), 6.80 (1H, dd, J=7.0, 1.5 Hz), 6.92-6.96 (3H, m), 7.10 (2H, d, J=8.3 Hz), 7.22-7.30 (4H, m).

### Example 11:

### 1-(2-{4-[(Diethylamino)methyl]phenyl}ethyl)-3-[4-(trifluoromethyl)phenoxy)pyridin-2(1H)-one:

¹HNMR (400 MHz, CDCl₃, δ ppm): 1.03 (6H, t, J=7.3 Hz), 2.50 (4H, q, J=7.3 Hz), 3.06 (2H, t, J=7.2 Hz), 3.53 (2H, s), 4.19 (2H, t, J=7.2 Hz), 6.01 (1H, t, J=7.1 Hz), 6.87 (1H, dd, J=7.1, 1.7 Hz), 7.03 (2H, d, J=8.3 Hz), 7.09-7.12 (3H, m), 7.21-7.24 (2H, m), 7.57 (2H, d, J=8.3 Hz).

### Example 12:

### 3-(Biphenyl-4-yloxy)-1-(2-{4-[(diethylamino)methyl]phenyl}ethyl)pyridin-2(1H)-one:

¹HNMR (400 MHz, CDCl₃, δ ppm): 1.03 (6H, t, J=7.3 Hz), 2.50 (4H, q, J=7.3 Hz), 3.08 (2H, t, J=7.1 Hz), 3.53 (2H, s), 4.21 (2H, t, J=7.1 Hz), 5.96 (1H, t, J=7.1 Hz), 6.79 (1H, dd, J=7.1, 1.5 Hz), 6.96 (1H, dd, J=7.1, 1.5 Hz), 7.05-7.15 (4H, m), 7.23-7.28 (2H, m), 7.30-7.35 (1H, m), 7.40-7.45 (2H, m), 7.54-7.60 (4H, m).

### Example 13:

### 1-(2-{4-[(diethylamino)methyl]phenyl}ethyl)-3-[4-(trifluoromethoxy)phenoxy)pyridin-2(1H)-one:

¹HNMR (400 MHz, CDCl₃, δ ppm): 1.03 (6H, t, J=7.2 Hz), 2.50 (4H, q, J=7.2 Hz), 3.06 (2H, t, J=7.2 Hz), 3.53 (2H, s), 4.19 (2H, t, J=7.2 Hz), 5.96 (1H, t, J=7.0 Hz), 6.82 (1H, dd, J=7.0, 1.5 Hz), 6.98-7.00 (3H, m), 7.10 (2H, d, J=7.8 Hz), 7.16-7.19 (2H, m), 7.24-7.26 (2H, m).

### Example 14:

### 4-[(5-Chloropyridin-2-yl)methoxy]-1-(2-{4-[(diethylamino)methyl]phenyl}ethyl)pyridin-2(1H)-one:

### (1) Production of 1- {2-[4-({[tert-butyl(dimethyl)silyl]oxy}methyl)phenyl]ethyl}-4-[(5-chloropyridin- 2- yl)methoxy]pyridin-2(1H)-one:

The compound of Reference Example 21 (6.4 g, 15.2 mmol) and cesium carbonate (8.3 g, 25.4 mmol) were added to a DMF solution (150 mL) of the compound of Reference Example 3 (3.0 g, 12.7 mmol), and stirred at 80°C for 5 hours in the presence of nitrogen. The reaction liquid was concentrated under reduced pressure, saturated ammonium chloride was added, and extracted with chloroform. The obtained organic layer was dried with anhydrous sodium sulfate, and filtered. The obtained filtrated was concentrated under reduced pressure, then the residue was purified by silica gel column chromatography (chloroform:methanol = 10:0 to 19:1) to obtain the entitled compound (2.5 g, 41 %).
Mass Spectrum (ESI): 485.1 (M+H).

### (2) Production of 4-[(5-chloropyridin-2-yl)methoxy]-1-{2-[4-(hydroxymethyl)phenyl]ethyl}pyridin-2(1H)-one:

In the same manner as in Reference Example 19-(8) but changing the compound used in Reference Example 19-(8) to the compound obtained in the above (1), the compound of Example 14-(2) was obtained.
Mass Spectrum (ESI): 371.1 (M+H).

### (3) Production of 1- {2-[4-(chloromethyl)phenyl]ethyl}-4-[(5-chloropyridin-2-yl)methoxy]pyridin-2(1H)-one:

Thionyl chloride (0.58 mL, 6.64 mmol) was added to a chloroform solution (100 mL) of the compound obtained in the above (2) (2.05 g, 5.53 mmol), and stirred with heating under reflux for 1 hour. Next, aqueous saturated sodium hydrogencarbonate solution was added to the reaction liquid, and extracted with chloroform. The obtained organic layer was dried with anhydrous sodium sulfate, and filtered. The obtained filtrate was concentrated under reduced pressure to obtain the entitled compound (2.02 g, 94 %).
Mass Spectrum (ESI): 389.0 (M+H).

### (4) Production of 4-[(5-chloropyridin-2-yl)methoxy]-1-(2-{4-[(diethylamino)methyl]phenyl} ethyl)pyridin-2(1H)-one:

Diethylamine (0.64 mL, 6.18 mmol) and potassium carbonate (1.42 g, 10.3 mmol) were added to an NMP solution (8 mL) of the compound obtained in the above (3), and stirred at 80°C for 12 hours. Water was added to the reaction liquid, and extracted with ethyl acetate. The obtained organic layer was washed with aqueous sodium hydrogencarbonate solution and saturated saline, then dried with anhydrous sodium sulfate, and filtered. The obtained filtrate was concentrated under reduced pressure, and the residue was purified by high-performance liquid chromatography (YMC-Pack™ pro C-18, H₂O (0.1 % TFA):CH₃CN (0.1 % TFA) = 90:10 to 50:50) to obtain the entitled compound (557 mg, 64 %).
¹HNMR (400 MHz, CDCl₃, δ ppm): 1.05 (6H, t, J=7.0 Hz), 2.52 (4H, q, J=7.0 Hz), 3.00 (2H, t, J=7.1 Hz), 3.55 (2H, s), 4.07 (2H, t, J=7.1 Hz), 5.09 (2H, s), 5.80 (1H, dd, J=7.4, 2.7 Hz), 5.96 (1H, d, J=2.7 Hz), 6.76 (1H, d, J=7.4 Hz), 7.08 (2H, d, J=8.2 Hz), 7.22-7.28 (2H, m), 7.39 (1H, d, J=8.6 Hz), 7.71 (1H, dd, J=8.6, 2.3 Hz), 8.57 (1H, d, J=2.3 Hz).

### Examples 15 to 41:

In the same manner as in Example 14 but changing the compound of Reference Example 3 and the compound of Reference Example 21 used in Example 14-(1) to corresponding compounds of Reference Examples and changing diethylamine used in Example 14-(4) to corresponding known amine compounds, the compounds of Examples 15 to 41 were obtained.

### Example 15:

### 4-[(4-Fluorobenzyl)oxy]-1-[2-(4-{[(3S)-3-fluoropyridin-1-yl]methyl}phenyl)ethyl]pyridin-2(1H)-one:

¹HNMR (400 MHz, CDCl₃, δ ppm): 1.98-2.25 (2H, m), 2.41-2.51 (1H, m), 2.72-2.90 (3H, m), 3.01 (2H, t, J=7.1 Hz), 3.57-3.70 (2H, m), 4.07 (2H, t, J=7.1 Hz), 4.94 (2H, s), 5.07-5.26 (1H, m), 5.76 (1H, dd, J=7.8, 2.9 Hz), 5.98 (1H, d, J=2.9 Hz), 6.76 (1H, d, J=7.8 Hz), 7.06-7.12 (4H, m), 7.22-7.30 (2H, m), 7.35-7.39 (2H, m).

### Example 16:

### 1-(2-{4-[(Diethylamino)methyl]phenyl}ethyl)-4-(2-naphthyl)pyridin-2(1H)-one:

¹HNMR (400 MHz, CDCl₃, δ ppm): 1.04 (6H, t, J=7.2 Hz), 2.52 (4H, q, J=7.2 Hz), 3.10 (2H, t, J=7.2 Hz), 3.55 (2H, s), 4.20 (2H, t, J=7.2 Hz), 6.41 (1H, dd, J=7.1, 2.1 Hz), 6.94 (1H, d, J=2.1 Hz), 7.00 (1H, d, J=6.8 Hz), 7.15 (2H, d, J=7.8 Hz), 7.24-7.30 (2H, m), 7.50-7.56 (2H, m), 7.67 (1H, dd, J=8.3, 2.0 Hz), 7.85-7.94 (3H, m), 8.02-8.06 (1H, m).

### Example 17:

### 4-(2-Naphthyl)-1-{2-[4-(pyrrolidin-1-ylmethyl)phenyl]ethyl}pyridin-2(1H)-one:

¹HNMR (400 MHz, CDCl₃, δ ppm): 1.77-1.81 (4H, m), 2.45-2.55 (4H, m), 3.10 (2H, t, J=7.1 Hz), 3.60 (2H, s), 4.20 (2H, t, J=7.1 Hz), 6.41 (1H, dd, J=6.8, 2.0 Hz), 6.94 (1H, d, J=2.0 Hz), 7.01 (1H, d, J=6.8 Hz), 7.16 (2H, d, J=8.3 Hz), 7.25-7.30 (2H, m), 7.50-7.58 (2H, m), 7.67 (1H, dd, J=8.3, 2.0 Hz), 7.84-7.96 (3H, m), 8.02-8.06 (1H, m).

### Example 18:

### 1-[2-(4- {[Methyl(propyl)amino]methyl}phenyl)ethyl]-4-[(E)-2-phenylvinyl]pyridin-2(1H)-one:

¹HNMR (400 MHz, CDCl₃, δ ppm): 0.90 (3H, t, J=7.3 Hz), 1.48-1.60 (2H, m), 2.17 (3H, s), 2.28-2.35 (2H, m), 3.05 (2H, t, J=7.1 Hz), 3.45 (2H, s), 4.13 (2H, t, J=7.1 Hz), 6.23 (1H, dd, J=7.3, 1.8 Hz), 6.58 (1H, d, J=1.8 Hz), 6.85 (1H, d, J=7.2 Hz), 6.86 (1H, d, J=16.4 Hz), 7.08-7.15 (3H, m), 7.23 (2H, d, J=8.0 Hz), 7.30-7.40 (3H, m), 7.48-7.55 (2H, m).

### Example 19:

### 1-[2-(4-{[(35)-3-fluoropyrrolidin-1-yl]methyl}phenyl)ethyl]-4-[(E)-2-phenylvinyl]pyridin-2(1H)-one:

¹HNMR (400 MHz, CDCl₃, δ ppm): 2.00-2.10 (2H, m), 2.00-2.10 (2H, m), 2.40-2.50 (1H, m), 2.64-2.90 (3H, m), 3.06 (2H, t, J=7.2 Hz), 3.60 (1H, d, J=13.0 Hz), 3.66 (1H, d, J=13.0 Hz), 4.13 (2H, t, J=7.2 Hz), 5.06-5.26 (1H, m), 6.24 (1H, dd, J=7.3, 1.8 Hz), 6.57 (1H, d, J=1.8 Hz), 6.86 (1H, d, J=7.2 Hz), 6.86 (1H, d, J=16.0 Hz), 7.08-7.16 (3H, m), 7.22-7.28 (2H, m), 7.28-7.42 (3H, m), 7.48-7.54 (2H, m).

### Example 20:

### 4-[(E)-2-(4-fluorophenyl)vinyl]-1-[2-(4- {[methyl(propyl)amino]methyl}phenyl)ethyl]pyridin-2(1H)-one:

¹HNMR (400 MHz, CDCl₃,δ ppm): 0.90 (3H, t, J=7.2 Hz), 1.53 (2H, qt, J=7.5, 7.2 Hz), 2.17 (3H, s), 2.28-2.35 (2H, m), 3.05 (2H, t, J=7.2 Hz), 3.45 (2H, s), 4.13 (2H, t, J=7.2 Hz), 6.20 (1H, dd, J=7.2, 1.9 Hz), 6.56 (1H, d, J=1.9 Hz), 6.77 (1H, d, J=16.1 Hz), 6.85 (1H, d, J=7.2 Hz), 7.04-7.12 (5H, m), 7.20-7.30 (2H, m), 7.46-7.52 (2H, m).

### Example 21:

### 4-[(E)-2-(4-fluorophenyl)vinyl]-1-[2-(4-{[(3S)-3-fluoropyrrolidin-1-yl]methyl}phenyl)ethyl]pyridin-2(1H)-one:

¹HNMR (400 MHz, CDCl₃, δ ppm): 1.90-2.50 (2H, m), 2.40-2.50 (1H, m), 2.65-2.84 (3H, m), 3.05 (2H, t, J=7.2 Hz), 3.59 (1H, d, J=12.6 Hz), 3.67 (1H, d, J=12.6 Hz), 4.13 (2H, t, J=7.2 Hz), 5.05-5.26 (1H, m), 6.21 (1H, dd, J=7.3, 2.0 Hz), 6.56 (1H, d, J=2.0 Hz), 6.77 (1H, d, J=16.6 Hz), 6.86 (1H, d, J=7.3 Hz), 7.04-7.16 (5H, m), 7.22-7.30 (2H, m), 7.45-7.53 (2H, m).

### Example 22:

### 4-[(E)-2-(4-chlorophenyl)vinyl]-1-[2-(4-{[methyl(propyl)amino]methyl}phenyl)ethyl]pyridin-2(1H)-one:

¹HNMR (400 MHz, CDCl₃, δ ppm): 0.90 (3H, t, J=7.3 Hz), 1.53 (2H, qt, J=7.3, 7.6 Hz), 2.17 (3H, s), 2.28-2.35 (2H, m), 3.05 (2H, t, J=7.1 Hz), 3.45 (2H, s), 4.13 (2H, t, J=7.1 Hz), 6.20 (1H, dd, J=7.3, 2.0 Hz), 6.57 (1H, d, J=2.0 Hz), 6.82 (1H, d, J=16.5 Hz), 6.85 (1H, d, J=7.2 Hz), 7.05 (1H, d, J=16.5 Hz), 7.11 (2H, d, J=7.9 Hz), 7.23 (2H, d, J=7.9 Hz), 7.32-7.38 (2H, m), 7.40-7.46 (2H, m).

### Example 23:

### 4-[(E)-2-(4-chlorophenyl)vinyl]-1-[2-(4- {[(3S)-3-fluoropyrrolidin-1-yl]methyl}phenyl)ethyl]pyridin-2(1H)-one:

¹HNMR (400 MHz, CDCl₃, δ ppm): 1.95-2.25 (2H, m), 2.40-2.50 (1H, m), 2.67-2.87 (3H, m), 3.05 (2H, t, J=7.1 Hz), 3.59 (1H, d, J=13.0 Hz), 3.67 (1H, d, J=13.0 Hz), 4.13 (2H, t, J=7.1 Hz), 5.05-5.26 (1H, m), 6.21 (1H, dd, J=7.3, 1.8 Hz), 6.57 (1H, d, J=1.8 Hz), 6.82 (1H, d, J=16.1 Hz), 6.86 (1H, d, J=7.2 Hz), 7.05 (1H, d, J=16.1 Hz), 7.12 (2H, d, J=8.3 Hz), 7.22-7.28 (2H, m), 7.34-7.36 (2H, m), 7.41-7.46 (2H, m).

### Example 24:

### 1-{2-[4-Azetidin-1-ylmethyl)phenyl]ethyl}-4-[(5-chloropyridin-2-yl)methoxy]pyridin-2(1H)-one:

¹HNMR (400 MHz, CDCl₃, δ ppm): 2.09 (2H, d, J=6.8 Hz), 3.00 (2H, t, J=7.1 Hz), 3.21 (4H, t, J=6.8 Hz), 3.54 (2H, s), 4.06 (2H, t, J=7.1 Hz), 5.09 (2H, s), 5.80 (1H, dd, J=7.5, 2.8 Hz), 5.96 (1H, d, J=2.8 Hz), 6.74 (1H, d, J=7.5 Hz), 7.08 (2H, d, J=8.3 Hz), 7.19 (2H, d, J=8.3 Hz), 7.40 (1H, d, J=8.3 Hz), 7.71 (1H, dd, J=8.3, 2.4 Hz), 8.57 (1H, d, J=2.4 Hz).

### Example 25:

### 1-{2-[4-Azepan-1-ylmethyl)phenyl]ethyl}-4-[(5-chloropyridin-2-yl)methoxy]pyridin-2(1H)-one:

¹HNMR (400 MHz, CDCl₃, δ ppm): 1.62 (8H, m), 2.61 (4H, brs), 3.00 (2H, t, J=7.1 Hz), 3.61 (2H, s), 4.07 (2H, t, J=7.1 Hz), 5.09 (2H, s), 5.80 (1H, dd, J=7.5, 2.6 Hz), 5.96 (1H, d, J=2.6 Hz), 6.76 (1H, d, J=7.5 Hz), 7.08 (2H, d, J=8.3 Hz), 7.20-7.30 (2H, m), 7.39 (1H, d, J=8.3 Hz), 7.71 (1H, dd, J=8.3, 2.4 Hz), 8.57 (1H, d, J=2.4 Hz).

### Example 26:

### 4-[(5-Chloropyridin-2-yl)methoxy]-1-(2-[4-(pyrrolidin-1-ylmethyl)phenyl]ethyl}pyridin-2(1H)-one:

¹HNMR (400 MHz, CDCl₃, δ ppm): 1.79 (4H, s), 2.50 (4H, brs), 3.01 (2H, t, J=7.1 Hz), 3.59 (2H, s), 4.07 (2H, t, J=7.1 Hz), 5.09 (2H, s), 5.80 (1H, dd, J=7.7, 2.8 Hz), 5.96 (1H, d, J=2.8 Hz), 6.76 (1H, d, J=7.7 Hz), 7.09 (2H, d, J=8.3 Hz), 7.22-7.30 (2H, m), 7.40 (1H, d, J=8.3 Hz), 7.71 (1H, dd, J=8.3, 2.4 Hz), 8.57 (1H, d, J=2.4 Hz).

### Example 27:

### 4-[(5-Chloropyridin-2-yl)methoxy]-1-(2-[4-{[(2R)-2-methylpyrrolidin-1-yl]methyl}phenyl)ethyl]pyridin-2(1H)-one:

¹HNMR (400 MHz, CDCl₃, δ ppm): 1.16 (3H, d, J=6.3 Hz), 1.40-1.75 (2H, m), 1.90-2.15 (2H, m), 2.30-2.45 (2H, m), 2.80-2.90 (1H, m), 3.00 (2H, t, J=7.1 Hz), 3.10 (1H, d, J=12.7 Hz), 3.98 (1H, d, J=12.7 Hz), 4.05-4.09 (2H, m), 5.09 (2H, s), 5.78-5.82 (1H, m), 5.96 (1H, d, J=2.9 Hz), 6.74 (1H, d, J=7.6 Hz), 7.08 (2H, d, J=7.8 Hz), 7.20-7.30 (2H, m), 7.40 (1H, d, J=8.3 Hz), 7.71 (1H, dd, J=8.3, 2.4 Hz), 8.57 (1H, d, J=2.4 Hz).

### Example 28:

### 4-[(5-Chloropyridin-2-yl)methoxy]-1-{2-[4-(piperidm-1-ylmethyl)phenyl]ethyl}pyridin-2(1H)-one:

¹HNMR (400 MHz, CDCl₃, δ ppm): 1.40-1.50 (2H, m), 1.50-1.65 (4H, m), 2.30-2.40 (4H, m), 3.00 (2H, t, J=7.1 Hz), 3.44 (2H, s), 4.07 (2H, t, J=7.1 Hz), 5.09 (2H, s), 5.80 (1H, dd, J=7.3, 2.9 Hz), 5.96 (1H, d, J=2.9 Hz), 6.76 (1H, d, J=7.3 Hz), 7.08 (2H, d, J=8.3 Hz), 7.20-7.30 (2H, m), 7.39 (1H, d, J=8.3 Hz), 7.71 (1H, dd, J=8.3, 2.4 Hz), 8.57 (1H, d, J=2.4 Hz).

### Example 29:

### 1-(2- {5-[(Diethylamino)methyl]pyridin-2-yl}ethyl)-4-[(4-fluorobenzyl)oxy]pyridin-2(1H)-one:

¹HNMR (400 MHz, CDCl₃, δ ppm): 1.04 (6H, t, J=7.1 Hz), 2.45-2.55 (4H, m), 3.20 (2H, t, J=6.8 Hz), 3.54 (2H, s), 4.29 (2H, t, J=6.8 Hz), 4.92 (2H, s), 5.75 (1H, dd, J=7.8, 2.4 Hz), 5.96 (1H, d, J=2.4 Hz), 6.93 (1H, d, J=7.8 Hz), 7.04-7.10 (3H, m), 7.34-7.37 (2H, m), 7.56 (1H, d, J=7.3 Hz), 8.47 (1H, s).

### Example 30:

### 4-[(4-Fluorobenzyl)oxy]-1-[2-(5-{[methyl(propyl)amino]methyl}pyridin-2-yl)ethyl]pyridin-2(1H)-one:

¹HNMR (400 MHz, CDCl₃, δ ppm): 0.89 (3H, t, J=7.3 Hz), 1.49-1.57 (2H, m), 2.17 (3H, s), 2.32 (2H, t, J=7.3 Hz), 3.21 (2H, t, J=6.8 Hz), 3.46 (2H, s), 4.29 (2H, t, J=6.8 Hz), 4.92 (2H, s), 5.75 (1H, dd, J=7.7, 2.8 Hz), 5.96 (1H, d, J=2.8 Hz), 6.93 (1H, d, J=7.7 Hz), 7.04-7.11 (3H, m), 7.33-7.38 (2H, m), 7.54 (1H, dd, J=7.8, 2.4 Hz), 8.46 (1H, d, J=2.4 Hz).

### Example 31:

### 4-[(4-Fluorobenzyl)oxy]-1-{2-[5-(pyrrolidin-1-ylmethyl)pyridin-2-yl]ethyl}pyridin-2(1H)-one:

¹HNMR (400 MHz, CDCl₃, δ ppm): 1.77-1.83 (4H, m), 2.46-2.54 (4H, m), 3.20 (2H, t, J=7.1 Hz), 3.60 (2H, s), 4.29 (2H, t, J=7.1 Hz), 4.92 (2H, s), 5.75 (1H, dd, J=7.7, 2.6 Hz), 5.96 (1H, d, J=2.6 Hz), 6.93 (1H, d, J=7.7 Hz), 7.04-7.11 (3H, m), 7.33-7.39 (2H, m), 7.56 (1H, dd, J=7.8, 2.0 Hz), 8.49 (1H, d, J=2.0 Hz).

### Example 32:

### 1- {2-[5-(Azetidin-1-ylmethyl)pyridin-2-yl]ethyl} -4-[(4-fluorobenzyl)oxy]pyridin-2(1H)-one:

¹HNMR (400 MHz, CDCl₃,δ ppm): 2.06-2.13 (2H, m), 3.17-3.24 (6H, m), 3.54 (2H, s), 4.28 (2H, t, J=6.8 Hz), 4.92 (2H, s), 5.75 (1H, dd, J=7.6, 2.9 Hz), 5.96 (1H, d, J=2.9 Hz), 6.92 (1H, d, J=7.6 Hz), 7.03-7.10 (3H, m), 7.33-7.38 (2H, m), 7.49 (1H, dd, J=7.8, 2.2 Hz), 8.44 (1H, d, J=2.2 Hz).

### Example 33:

### 4-[(4-Fluorobenzyl)oxy]-1-{2-[6-(pyrrolidin-1-ylmethyl)pyridin-3-yl]ethyl}pyridin-2(1H)-one:

¹HNMR (400 MHz, CDCl₃, δ ppm): 1.78-1.84 (4H, m), 2.53-2.62 (4H, m), 3.04 (2H, t, J=7.0 Hz), 3.76 (2H, s), 4.07 (2H, t, J=7.0 Hz), 4.94 (2H, s), 5.79 (1H, dd, J=7.6, 2.7 Hz), 5.98 (1H, d, J=2.7 Hz), 6.78 (1H, d, J=7.6 Hz), 7.06-7.12 (2H, m), 7.32-7.39 (3H, m), 7.46 (1H, dd, J=7.8, 2.2 Hz), 8.34 (1H, d, J=2.2 Hz).

### Example 34:

### 1- {2-[4-(7-Azabicyclo[2.2.1]hept-7-ylmethyl)phenyl]ethyl}-4-[(5-chloropyridin-2-yl)methoxy]pyridin-2(1H)-one:

¹HNMR (400 MHz, CDCl₃, δ ppm): 1.27-1.35 (4H, m), 1.76-1.85 (4H, m), 3.01 (2H, t, J=7.1 Hz), 3.21-3.27 (2H, m), 3.51 (2H, s), 4.07 (2H, t, J=7.1 Hz), 5.09 (2H, s), 5.82 (1H, dd, J=7.8, 2.4 Hz), 5.96 (1H, d, J=2.4 Hz), 6.78 (1H, d, J=7.8 Hz), 7.09 (2H, d, J=8.3 Hz), 7.25-7.32 (2H, m), 7.40 (1H, d, J=8.8 Hz), 7.71 (1H, dd, J=8.8, 2.4 Hz), 8.57 (1H, d, J=2.4 Hz).

### Example 35:

### 4-[(4-chlorobenzyl)oxy]-1-(2-{5-[(dimethylamino)methyl]pyridin-2-yl}ethyl)pyridin-2(1H)-one:

¹HNMR (400 MHz, CDCl₃, δ ppm): 1.03 (6H, t, J=7.1 Hz), 2.50 (4H, q, J=7.1 Hz), 3.20 (2H, t, J=6.8 Hz), 3.54 (2H, s), 4.29 (2H, t, J=6.8 Hz), 4.93 (2H, s), 5.74 (1H, dd, J=7.3, 2.4 Hz), 5.94 (1H, d, J=2.4 Hz), 6.92 (1H, d, J=7.3 Hz), 7.04 (1H, d, J=7.8 Hz), 7.30-7.37 (4H, m), 7.56 (1H, dd, J=7.8, 2.0 Hz), 8.47 (1H, d, J=2.0 Hz).

### Example 36:

### 4-[(4-Chlorobenzyl)oxy]-1-[2-(5-{[methyl(propyl)amino]methyl}pyridin-2-yl)ethyl]pyridin-2(1H)-one:

¹HNMR (400 MHz, CDCl₃, δ ppm): 0.89 (3H, t, J=7.3 Hz), 1.52 (2H, q, J=7.3 Hz), 2.17 (3H, s), 2.32 (2H, t, J=7.3 Hz), 3.20 (2H, t, J=7.1 Hz), 3.46 (2H, s), 4.29 (2H, t, J=7.1 Hz), 4.93 (2H, s), 5.74 (1H, dd, J=7.6, 2.4 Hz), 5.94 (1H, d, J=2.4 Hz), 6.92 (1H, d, J=7.6 Hz), 7.05 (1H, d, J=7.8 Hz), 7.29-7.38 (4H, m), 7.52-7.56 (1H, m), 8.46 (1H, d, J=2.0 Hz).

### Example 37:

### 4-[(4-Chlorobenzyl)oxy]-1-{2-[5-(pyrrolidin-1-ylmethyl)pyridin-2-yl]ethyl}pyridin-2(1H)one:

¹HNMR (400 MHz, CDCl₃, δ ppm): 1.77-1.81 (4H, m), 2.47-2.53 (4H, m), 3.20 (2H, t, J=6.8 Hz), 3.60 (2H, s), 4.29 (2H, t, J=6.8 Hz), 4.93 (2H, s), 5.75 (1H, dd, J=7.8, 2.4 Hz), 5.94 (1H, d, J=2.4 Hz), 6.93 (1H, d, J=7.8 Hz), 7.05 (1H, d, J=7.8 Hz), 7.29-7.38 (4H, m), 7.55 (1H, dd, J=7.8, 2.0 Hz), 8.49 (1H, d, J=2.0 Hz).

### Example 38:

### 1-{2-[5-(Azetidin-1-ylmethyl)pyridin-2-yl]ethyl}-4-[(4-chlorobenzyl)oxy]pyridin-2(1H)-one:

¹HNMR (400 MHz, CDCl₃,δ ppm): 2.05-2.14 (2H, m), 3.17-3.24 (6H, m), 3.54 (2H, s), 4.28 (2H, t, J=6.8 Hz), 4.92 (2H, s), 5.75 (1H, dd, J=7.8, 2.4 Hz), 5.93 (1H, d, J=2.4 Hz), 6.92 (1H, d, J=7.8 Hz), 7.04 (1H, d, J=7.8 Hz), 7.30-7.37 (4H, m), 7.49 (1H, dd, J=7.8, 2.0 Hz), 8.45 (1H, d, J=2.0 Hz).

### Example 39:

### 4-[(4-Chlorobenzyl)oxy]-1-{2-[6-(pyrrolidin-1-ylmethyl)pyridin-3-yl]ethyl}pyridin-2(1H)-one:

¹HNMR (400 MHz, CDCl₃, δ ppm): 1.78-1.85 (4H, m), 2.54-2.64 (4H, m), 3.04 (2H, t, J=6.8 Hz), 3.74-3.80 (2H, m), 4.07 (2H, t, J=6.8 Hz), 4.94 (2H, s), 5.80 (1H, dd, J=7.3, 2.9 Hz), 5.96 (1H, d, J=2.9 Hz), 6.78 (1H, d, J=7.3 Hz), 7.30-7.39 (5H, m), 7.46 (1H, dd, J=8.0, 2.0 Hz), 8.34 (1H, d, J=2.0 Hz).

### Example 40:

### 4-[(4-Chlorobenzyl)oxy]-1-[2-(4-{[methyl(propyl)amino]methyl}phenyl)ethyl]pyridin-2(1H)-one:

¹HNMR (400 MHz, CDCl₃, δ ppm): 0.89 (3H, t, J=7.3 Hz), 1.52-1.55 (2H, m), 2.12 (3H, s), 2.28-2.36 (2H, m), 3.00 (2H, t, J=7.1 Hz), 3.34-3.50 (2H, m), 4.07 (2H, t, J=7.1 Hz), 4.95 (2H, s), 5.75 (1H, dd, J=7.3, 2.4 Hz), 5.96 (1H, d, J=2.4 Hz), 6.74 (1H, d, J=7.3 Hz), 7.09 (1H, d, J=7.8 Hz), 7.22-7.38 (7H, m).

### Example 41:

### 1- {2-[4-(7-Azabicyclo[2.2.1]hept-7-ylmethyl)phenyl]ethyl}-4-[(4-chlorobenzyl)oxy]pyridin-2(1H)-one:

¹HNMR (400 MHz, CDCl₃, δ ppm): 1.25-1.36 (4H, m), 1.74-1.86 (4H, m), 3.01 (2H, t, J=7.1 Hz), 3.21-3.29 (2H, m), 3.50-3.56 (2H, m), 4.07 (2H, t, J=7.1 Hz), 4.95 (2H, s), 5.77 (1H, dd, J=7.3, 2.7 Hz), 5.97 (1H, d, J=2.7 Hz), 6.77 (1H, d, J=7.3 Hz), 7.10 (1H, d, J=8.3 Hz), 7.26-7.38 (7H, m).

### Example 42:

### 4-[(5-Chloropyridin-2-yl)methoxy]-1-(2- {4-[(isopropylamino)methyl]phenyl}ethyl)pyridin-2(1H)-one:

Isopropylamine (0.085 mL, 1.0 mmol) was added to an NMP solution (3 mL) of the compound of Example 14-(3) (40.0 mg, 0.10 mmol), and irradiated with microwaves (150°C, 50 minutes). Aqueous saturated sodium hydrogencarbonate solution was added to the reaction liquid, and extracted with ethyl acetate. The obtained organic layer was washed with aqueous sodium hydrogencarbonate solution and saturated saline, then dried with anhydrous sodium sulfate, and filtered. The obtained filtrate was concentrated under reduced pressure, then the residue was purified by high-performance liquid chromatography (YMC-Pack™ pro C-18, H₂O (0.1 % TFA):CH₃CN (0.1 % TFA) = 90:10 to 50:50) to obtain the entitled compound (26.6 mg, 65 %).
¹HNMR (400 MHz, CDCl₃, δ ppm): 1.11 (6H, d, J=6.2 Hz), 2.84-2.90 (1H, m), 3.00 (2H, t, J=7.0 Hz), 3.77 (2H, s), 4.06 (2H, t, J=7.0 Hz), 5.09 (2H, s), 5.82 (1H, dd, J=7.5, 2.6 Hz), 5.96 (1H, d, J=2.6 Hz), 6.77 (1H, d, J=7.5 Hz), 7.10 (2H, d, J=7.8 Hz), 7.22-7.28 (2H, d, J=7.8 Hz), 7.40 (1H, d, J=8.6 Hz), 7.71 (1H, dd, J=8.6, 2.4 Hz), 8.57 (1H, d, J=2.4 Hz).

### Examples 43 to 53:

The same process as in Example 14-(1) to Example 14-(3) was carried out except that the compound of Reference Example 3 and the compound of Reference Example 21 used in Example 14-(1) were changed to corresponding compounds of Reference Examples, and subsequently, the same process as in Example 42 was carried out except that the obtained compound was used in place of the compound of Example 14-(3) used in Example 42 to obtain the compounds of Examples 43 to 53.

### Example 43:

### 1-(2- {4-[(Tert-butylamino)methyl]phenyl}ethyl)-4-[(5-chloropyridin-2-yl)methoxy]pyridin-2(1H)-one:

¹HNMR (400 MHz, CDCl₃, δ ppm): 1.19 (9H, s), 2.99 (2H, t, J=7.0 Hz), 3.71 (2H, s), 4.06 (2H, t, J=7.0 Hz), 5.09 (2H, s), 5.82 (1H, dd, J=7.5, 2.6 Hz), 5.96 (1H, d, J=2.6 Hz), 6.78 (1H, d, J=7.5 Hz), 7.09 (2H, d, J=7.8 Hz), 7.22-7.30 (2H, m), 7.40 (1H, d, J=8.5 Hz), 7.71 (1H, dd, J=8.5, 2.4 Hz), 8.57 (1H, d, J=2.4 Hz).

### Example 44:

### 4-[(5-Chloropyridin-2-yl)methoxy]-1-(2- {4-[(cyclopropylamino)methyl]phenyl}ethyl)pyridin-2(1H)-one:

¹HNMR (400 MHz, CDCl₃, δ ppm): 0.35-0.46 (4H, m), 2.11-2.16 (1H, m), 3.00 (2H, t, J=7.1 Hz), 3.81 (2H, s), 4.07 (2H, t, J=7.1 Hz), 5.09 (2H, s), 5.81 (1H, d, J=2.9, 7.5 Hz), 5.96 (1H, d, J=2.9 Hz), 6.77 (1H, d, J=7.5 Hz), 7.09 (2H, d, J=7.8 Hz), 7.22 (2H, d, J=7.8 Hz), 7.40 (1H, d, J=8.3 Hz), 7.71 (1H, dd, J=8.3, 2.4 Hz), 8.55-8.60 (1H, m).

### Example 45:

### 4-[(E)-2-(4-Fluorophenyl)vinyl]-1-(2- {4-[(propylamino)methyl]phenyl}ethyl)Pyridin-2(1H)-one:

¹HNMR (400 MHz, CDCl₃, δ ppm): 0.92 (3H, t, J=7.6 Hz), 1.49-1.56 (2H, m), 2.59 (2H, t, J=7.3 Hz), 3.05 (2H, t, J=7.1 Hz), 3.76 (2H, s), 4.12 (2H, t, J=7.1 Hz), 6.22 (1H, dd, J=7.1, 2.0 Hz), 6.56 (1H, d, J=2.0 Hz), 6.77 (1H, d, J=16.6 Hz), 6.86 (1H, d, J=7.1 Hz), 7.05-7.14 (5H, m), 7.22-7.30 (2H, m).7.46-7.52 (2H, m).

### Example 46:

### 4-[(E)-2-(4-Fluorophenyl)vinyl]-1-[2-(4-{[(2-methoxyethyl)amino]methyl}phenyl)ethyl]pyridin-2(1H)-one:

¹HNMR (400 MHz, CDCl₃, δ ppm): 2.80 (2H, t, J=5.3 Hz), 3.05 (2H, t, J=7.1 Hz), 3.35 (3H, s), 3.52 (2H, t, J=5.3 Hz), 3.78 (2H, s), 4.12 (2H, t, J=7.1 Hz), 6.22 (1H, dd, J=7.1, 2.0 Hz), 6.56 (1H, d, J=2.0 Hz), 6.77 (1H, d, J=16.6 Hz), 6.86 (1H, d, J=7.1 Hz), 7.05-7.13 (5H, m), 7.24-7.26 (2H, m), 7.47-7.52 (2H, m).

### Example 47:

### 4-[(E)-2-(4-Fluorophenyl)vinyl]-1-[2-(4-{[(3-methoxypropyl)amino]methyl}phenyl)ethyl]pyridin-2(1H)-one:

¹HNMR (400 MHz, CDCl₃, δ ppm): 1.79 (2H, qui, J=6.4 Hz), 2.72 (2H, t, J=6.4 Hz), 3.05 (2H, t, J=7.1 Hz), 3.32 (3H, s), 3.46 (2H, t, J=6.4 Hz), 3.77 (2H, s), 4.12 (2H, t, J=7.1 Hz), 6.23 (1H, dd, J=7.1, 2.0 Hz), 6.56 (1H, d, J=2.0 Hz), 6.77 (1H, d, J=16.1 Hz), 6.87 (1H, d, J=7.1 Hz), 7.05-7.14 (5H, m), 7.24-7.27 (2H, m), 7.48-7.51 (2H, m).

### Example 48:

### 4-[(4-Fluorobenzyl)oxy]-1-(2- {5-[(propylamino)methyl]pyridin-2-yl}ethyl)pyridin-2(1H)-one:

¹HNMR (400 MHz, CDCl₃, δ ppm): 0.92 (3H, t, J=7.6 Hz), 1.48-1.58 (2H, m), 2.59 (2H, t, J=7.1 Hz), 3.20 (2H, t, J=6.8 Hz), 3.78 (2H, s), 4.29 (2H, t, J=6.8 Hz), 4.92 (2H, s), 5.76 (1H, dd, J=7.3, 2.9 Hz), 5.96 (1H, d, J=2.9 Hz), 6.94 (1H, d, J=7.3 Hz), 7.05-7.10 (3H, m), 7.34-7.37 (2H, m), 7.56 (1H, dd, J=7.8, 2.0 Hz), 8.50 (1H, d, J=2.0 Hz).

### Example 49:

### 4-[(4-Chlorobenzyl)oxy]-1-(2- {5-[(propylamino)methyl]pyridin-2-yl}ethyl)pyridin-2(1H)-one:

¹HNMR (400 MHz, CDCl₃, δ ppm): 0.92 (3H, t, J=7.3 Hz), 1.48-1.58 (2H, m), 2.59 (2H, t, J=7.3 Hz), 3.20 (2H, t, J=6.8 Hz), 3.78 (2H, s), 4.29 (2H, t, J=6.8 Hz), 4.93 (2H, s), 5.76 (1H, dd, J=7.3, 2.9 Hz), 5.94 (1H, d, J=2.9 Hz), 6.94 (1H, d, J=7.3 Hz), 7.06 (1H, d, J=7.8 Hz), 7.30-7.37 (5H, m), 7.56 (1H, dd, J=7.8, 2.0 Hz), 8.50 (1H, d, J=2.0 Hz).

### Example 50:

### 1-(2-{4-[(butylamino)methyl]phenyl}ethyl)-4-[(5-chloropyridin-2-yl)methoxy]pyridin-2(1H)one:

¹HNMR (400 MHz, CDCl₃, δ ppm): 0.91 (3H, t, J=7.3 Hz), 1.30-1.40 (2H, m), 1.45-1.60 (2H, m), 2.63 (2H, t, J=7.3 Hz), 3.00 (3H, t, J=7.1 Hz), 3.77 (2H, s), 4.07 (2H, t, J=7.1 Hz), 5.09 (2H, s), 5.82 (1H, dd, J=7.5, 2.9 Hz), 5.96 (1H, d, J=2.9 Hz), 6.77 (1H, d, J=7.5 Hz), 7.10 (2H, d, J=7.8 Hz), 7.22-7.28 (2H, m), 7.40 (1H, d, J=8.6 Hz), 7.71 (1H, dd, J=8.6, 2.2 Hz), 8.57 (1H, d, J=2.2 Hz).

### Example 51:

### 4-[(5-Chloropyridin-2-yl)methoxy]-1-(2- {4-[(cycloheptylamino)methyl]phenyl}ethyl)pyridin-2(1H)-one:

¹HNMR (400 MHz, CDCl₃, δ ppm): 1.35-1.42 (2H, m), 1.51-1.72 (4H, m), 1.82-1.88 (2H, m), 3.00 (2H, t, J=7.1 Hz), 3.10 (1H, qui, J=6.8 Hz), 3.74 (2H, s), 4.06 (2H, t, J=7.1 Hz), 5.09 (2H, s), 5.81 (1H, dd, J=7.6, 2.9 Hz), 5.96 (1H, d, J=2.9 Hz), 6.77 (1H, d, J=7.6 Hz), 7.09 (2H, d, J=7.8 Hz), 7.20-7.30 (2H, m), 7.40 (1H, d, J=8.3 Hz), 7.71 (1H, dd, J=8.3, 2.4 Hz), 8.57 (1H, d, J=2.4 Hz).

### Example 52:

### 4-[(5-Chloropyridin-2-yl)methoxy]-1-(2-(4-{[(cyclopropylmethyl)amino]methyl}phenyl)ethyl]pyridin-2(1H)-one:

¹HNMR (400 MHz, CDCl₃, δ ppm): 0.10-0.16 (2H, m), 0.46-0.54 (2H, m), 0.95-1.05 (1H, m), 2.51 (2H, d, J=6.8 Hz), 3.00 (2H, t, J=7.1 Hz), 3.82 (2H, s), 4.06 (2H, t, J=7.1 Hz), 5.09 (2H, s), 5.82 (1H, d, J=7.5, 2.9 Hz), 5.97 (1H, d, J=2.9 Hz), 6.77 (1H, d, J=7.5 Hz), 7.11 (2H, d, J=8.3 Hz), 7.24-7.30 (2H, m), 7.40 (1H, d, J=8.3 Hz), 7.71 (1H, dd, J=8.3, 2.4 Hz), 8.57 (1H, d, J=2.4 Hz).

### Example 53:

### 4-[(5-Chloropyridin-2-yl)methoxy]-1-(2-{4-[(propylamino)methyl]phenyl}ethyl)pyridin-2(1H)-one:

¹HNMR (400 MHz, CDCl₃, δ ppm): 0.92 (3H, t, J=7.3 Hz), 1.53-1.65 (2H, m), 2.61 (2H, t, J=7.3 Hz), 3.00 (2H, t, J=7.1 Hz), 3.80 (2H, s), 4.06 (2H, t, J=7.1 Hz), 5.09 (2H, s), 5.82 (1H, dd, J=7.5, 2.8 Hz), 5.97 (1H, d, J=2.8 Hz), 6.78 (1H, d, J=7.5 Hz), 7.11 (2H, d, J=7.8 Hz), 7.24-7.30 (2H, m), 7.40 (1H, d, J=8.6 Hz), 7.71 (1H, dd, J=8.6, 2.2 Hz), 8.57 (1H, d, J=2.2 Hz).

### Example 54:

### 4-[(4-Fluorobenzyl)oxy]-1-[2-(4-pyrrolidin-2-ylphenyl)ethyl]pyridin-2(1H)-one:

### (1) Production of tert-butyl 2-(4- {2-[4-[(4-fluorobenzyl)oxy]-2-oxopyridin-1(2H)yl]ethyl}phenyl)pyrrolidine-1-carboxylate:

In the same manner as in Example 1 but changing the compound of Reference Example 16 used in Example 1 to the compound of Reference Example 23-(2), the compound of Example 54-(1) was obtained.
Mass Spectrum (ESI): 493.3 (M+H).

### (2) Production of 4-[(4-fluorobenzyl)oxy]-1-[2-(4-pyrrolidin-2-ylphenyl)ethyl]pyridin-2(1H)-one:

TFA was added to the compound obtained in the above (1) (341 mg, 0.69 mmol), and stirred at room temperature for 1 hour. The reaction liquid was concentrated under reduced pressure, then aqueous saturated sodium hydrogencarbonate solution was added and extracted with ethyl acetate. The obtained organic layer was washed with saturated saline, then dried with anhydrous sodium sulfate, and filtered. The obtained filtrate was concentrated under reduced pressure, then diisopropyl ether was added to the residue, and the formed solid was collected by filtration and dried to obtain the entitled compound (199 mg, 74 %).
¹HNMR (400 MHz, CDCl₃,δ ppm): 1.85-2.10 (3H, m), 2.20-2.30 (1H, m), 2.96 (2H, t, J=7.2 Hz), 3.08-3.17 (1H, m), 3.20-3.30 (1H, m), 3.98-4.06 (2H, m), 4.25-4.32 (1H, m), 4.92 (2H, s), 5.79 (1H, dd, J=7.4, 2.7 Hz), 5.94 (1H, d, J=2.7 Hz), 6.80 (1H, d, J=7.4 Hz), 7.04-7.10 (4H, m), 7.30 (2H, d, J=8.2 Hz), 7.33-7.39 (2H, m).

### Example 55:

### 4-[(4-Fluorobenzyl)oxy]-1-{2-[4-(1-isopropylpyrrolidin-2-yl)phenyl]ethyl}pyridin-2(1H)-one:

0.2 M Zn[B(CN)H₃]₂ methanol solution (1.0 mL, prepared from zinc chloride and sodium cyanotrihydroborate) was added to a methanol solution (5 mL) of the compound of Example 54 and acetone (0.015 mL, 0.20 mmol), and stirred overnight at room temperature. The reaction liquid was concentrated under reduced pressure, then aqueous saturated sodium hydrogencarbonate solution was added, and extracted with ethyl acetate. The obtained organic layer was dried with anhydrous sodium sulfate, and filtered. The obtained filtrate was concentrated under reduced pressure, then the residue was purified by high-performance liquid chromatography (YMC-Pack™ pro C-18, H₂O (0.1 % TFA):CH₃CN (0.1 % TFA) = 90:10 to 50:50) to obtain the entitled compound (14.6 mg, 25 %).
¹HNMR (400 MHz, CDCl₃, δ ppm): 0.89 (3H, d, J=6.2 Hz), 0.99 (3H, d, J=6.6 Hz), 1.61-1.64 (1H, m), 1.74 (1H, t, J=6.1 Hz), 1.86 (1H, s), 2.11 (1H, q, J=6.0 Hz), 2.56 (1H, q, J=8.5 Hz), 2.70-2.77 (1H, m), 3.00 (2H, t, J=7.0 Hz), 3.07 (1H, td, J=8.3, 3.1 Hz), 3.59 (1H, t, J=7.8 Hz), 4.07 (2H, t, J=7.0 Hz), 4.94 (2H, s), 5.74 (1H, dd, J=7.8, 2.7 Hz), 5.98 (1H, d, J=2.7 Hz), 6.75 (1H, d, J=7.8 Hz), 7.06-7.10 (4H, m), 7.27 (10H, d, J=7.0 Hz), 7.37 (2H, dd, J=8.8, 5.3 Hz).

### Example 56:

### 4-[(4-Fluorobenzyl)oxy]-1-{2-[4-(1-methylpyrrolidin-2-yl)phenyl]ethyl}pyridin-2(1H)-one:

In the same manner as in Example 55 but changing acetone used in Example 55 to aqueous 35 % formaldehyde solution, a racemate of the entitled compound was obtained. Further, this was purified by high-performance liquid chromatography (CHIRALPAK^{™} AD-H, hexane:ethanol:diethylamine = 8:2:0.05) to obtain the entitled compound (fast) and (slow). (fast)
¹HNMR (400 MHz, CDCl₃, δ ppm): 1.70-1.85 (2H, s), 1.90-2.05 (1H, s), 2.10-2.25 (4H, s), 2.25-2.35 (1H, s), 3.01 (3H, t, J=6.9 Hz), 3.20-3.30 (1H, s), 4.07 (2H, t, J=6.9 Hz), 4.94 (2H, s), 5.75 (1H, dd, J=7.5, 2.6 Hz), 5.98 (1H, d, J=2.6 Hz), 6.76 (1H, d, J=7.5 Hz), 7.06-7.14 (4H, m), 7.23-7.29 (2H, m), 7.34-7.40 (2H, m).
(slow)
¹HNMR (400 MHz, CDCl₃,δ ppm): 1.75-1.90 (1H, m), 1.90-2.05 (1H, m), 2.15-2.23 (4H, m), 2.25-2.35 (1H, m), 2.98-3.08 (3H, m), 3.22-3.32 (1H, m), 4.07 (2H, t, J=7.3 Hz), 4.94 (2H, s), 5.76 (1H, dd, J=7.5, 2.8 Hz), 5.98 (1H, d, J=2.8 Hz), 6.76 (1H, d, J=7.5 Hz), 7.06-7.13 (4H, m), 7.22-7.30 (2H, m), 7.35-7.39 (2H, m).

### Example 57:

### 4-[(4-Fluorobenzyl)oxy]-1-(2- {4-[1-(2-hydroxyethyl)pyrrolidin-2-yl]phenyl}ethyl)pyridin-2(1H)-one:

### (1) Production of 1-(2-{4-[1-(2-{[tert-butyl(dimethyl)silyl]oxy}ethyl)pyrrolidin-2-yl]phenyl}ethyl)-4-[(4-fluorobenzyl)oxy]pyridin-2(1H)-one:

In the same manner as in Example 55 but changing acetone used in Example 55 to {[tert-butyl(dimethyl)silyl]oxy}acetaldehyde, the compound of Example 57-(1) was obtained. Mass Spectrum (ESI): 551.5 (M+H).

### (2) Production of 4-[(4-fluorobenzyl)oxy]-1-(2-{4-[1-(2-hydroxyethyl)pyrrolidin-2-yl]phenyl}ethyl)pyridin-2(1H)-one:

In the same manner as in Reference Example 19-(8) but changing the compound used in Reference Example 19-(8) to the compound obtained in the above (1), the compound of Example 57 was obtained.
¹HNMR (400 MHz, CDCl₃, δ ppm): 1.65-2.05 (3H, m), 2.12-2.25 (1H, m), 2.25-2.35 (2H, m), 2.68-2.77 (1H, m), 3.00 (2H, t, J=7.1 Hz), 3.32-3.40 (3H, m), 3.57 (1H, td, J=10.4, 3.6 Hz), 4.00-4.14 (2H, m), 4.94 (2H, s), 5.78 (1H, dd, J=7.5, 2.8 Hz), 5.98 (1H, d, J=2.8 Hz), 6.76 (1H, d, J=7.5 Hz), 7.05-7.13 (4H, m), 7.22-7.28 (2H, m), 7.34-7.40 (2H, m).

### Example 58:

### 4-[(4-Fluorobenzyl)oxy]-1-(2-{4-[1-(2-methoxyethyl)pyrrolidin-2-yl]phenyl}ethyl)pyridin-2(1H)-one:

With cooling with ice, sodium hydride (60 % oily, 2.2 mg, 0.055 mmol) was added to a THF solution (1 mL) of the compound of Example 57 (20 mg, 0.046 mmol), and stirred at 0°C for 30 minutes. Next, methyl iodide (0.006 mL, 0.092 mmol) was added, and stirred at room temperature for 2 days. Aqueous sodium hydrogencarbonate solution was added to the reaction liquid, then extracted with ethyl acetate. The obtained organic layer was washed with aqueous sodium hydrogencarbonate solution, then dried with anhydrous sodium sulfate, and filtered. The obtained filtrate was concentrated under reduced pressure, then the residue was purified by preparative thin-layer chromatography (Kieselgel^{™} 60F254, chloroform:methanol = 19:1) to obtain the entitled compound (7.3 mg, 35 %).
¹HNMR (400 MHz, CDCl₃, δ ppm): 1.62-1.75 (1H, m), 1.76-1.88 (1H, m), 1.88-2.02 (1H, m), 2.08-2.18 (1H, m), 2.20-2.34 (2H, m), 2.72-2.79 (1H, m), 3.00 (2H, t, J=7.1 Hz), 3.18-3.30 (1H, m), 3.26 (3H, s), 3.37-3.45 (3H, m), 4.07 (2H, t, J=7.1 Hz), 4.94 (2H, s), 5.77 (1H, dd, J=7.7, 2.6 Hz), 5.99 (1H, d, J=2.6 Hz), 6.78 (1H, d, J=7.7 Hz), 7.05-7.13 (4H, m), 7.24-7.30 (2H, m), 7.34-7.40 (2H, m).

### Example 59:

### 4-[(4-Fluorobenzyl)oxy]-1-(2-(4-[1-(2-fluoroethyl)pyrrolidin-2-yl]phenyl)ethyl)pyridin-2(1H)-one:

With cooling with ice, 2-fluoroethyl trifluoromethanesulfonate (28.0 mg, 0.14 mmol) was added to an acetonitrile solution (5 mL) of the compound of Example 54 (50.0 mg, 0.13 mmol) and potassium carbonate (27.0 mg, 0.20 mmol), and stirred overnight at 0°C to room temperature. Next, aqueous saturated sodium hydrogencarbonate solution was added to the reaction liquid, and extracted with ethyl acetate. The obtained organic layer was washed with aqueous sodium hydrogencarbonate solution, then dried with anhydrous sodium sulfate, and filtered. The obtained filtrate was concentrated under reduced pressure, and the residue was purified by silica gel column chromatography (chloroform:methanol = 19:1) to obtain the entitled compound (41.5 mg, 73 %).
¹HNMR (400 MHz, CDCl₃, δ ppm): 1.60-1.75 (1H, m), 1.75-1.90 (1H, m), 1.90-2.05 (1H, m), 2.10-2.20 (1H, m), 2.30-2.50 (2H, m), 2.72-2.88 (1H, m), 3.00 (2H, t, J=7.1 Hz), 3.27 (1H, t, J=8.3 Hz), 3.36-3.45 (1H, m), 4.07 (2H, t, J=7.1 Hz), 4.32-4.38 (1H, m), 4.45-4.50 (1H, m), 4.94 (2H, s), 5.76 (1H, dd, J=7.6, 2.9 Hz), 5.98 (1H, d, J=2.9 Hz), 6.77 (1H, d, J=7.6 Hz), 7.05-7.14 (4H, m), 7.24-7.30 (2H, m), 7.34-7.40 (2H, m).

### Example 60:

### 4-[(5-Chloropyridin-2-yl)methoxy]-1-[2-(4-pyrrolidin-2-ylphenyl)ethyl]pyridin-2(1H)-one:

In the same manner as in Example 14-(1) but changing the compound of Reference Example 21 used in Example 14-(1) to the compound of Reference Example 23, obtained was tert-butyl 2-(4-{2-[4-[(5-chloropyridin-2-yl)methoxy]-2-oxopyridin-1(2H)-yl]ethyl}phenyl)pyrrolidine-1-carboxylate. Next, in the same manner as in Example 54-(2) but changing the compound used in Example 54-(2) to the above compound, the compound of Example 60 was obtained.
¹HNMR (400 MHz, CDCl₃, δ ppm): 1.90-2.14 (3H, m), 2.26-2.30 (1H, m), 2.98 (2H, t, J=7.1 Hz), 3.08-3.17 (1H, m), 3.22-3.27 (1H, m), 4.04 (2H, t, J=7.1 Hz), 4.26-4.34 (1H, m), 5.08 (2H, s), 5.86 (1H, dd, J=7.5, 2.8 Hz), 5.93 (1H, d, J=2.8 Hz), 6.83 (1H, d, J=7.5 Hz), 7.10 (2H, d, J=8.3 Hz), 7.30 (2H, d, J=8.3 Hz), 7.40 (1H, d, J=8.3 Hz), 7.71 (1H, dd, J=8.3, 2.4 Hz), 8.56 (1H, d, J=2.4 Hz).

### Example 61:

### 4-[(5-Chloropyridin-2-yl)methoxy]-1-{2-[4-(1-methylpyrrolidin-2-yl)phenyl]ethyl}pyridin-2(1H)-one:

In the same manner as in Example 55 but changing acetone used in Example 55 to aqueous 35 % formaldehyde solution and changing the compound of Example 54 to the compound of Example 60, obtained was the compound of Example 61.
¹HNMR (400 MHz, CDCl₃, δ ppm): 1.70-1.85 (2H, m), 1.90-2.00 (1H, m), 2.10-2.20 (4H, m), 2.23-2.33 (1H, m), 2.95-3.05 (3H, m), 3.23 (1H, t, J=8.3 Hz), 4.07 (2H, t, J=7.1 Hz), 5.09 (2H, s), 5.81 (1H, dd, J=2.4, 7.3 Hz), 5.96 (1H, d, J=2.4 Hz), 6.77 (1H, d, J=7.3 Hz), 7.10 (2H, d, J=8.3 Hz), 7.22-7.30 (2H, m), 7.40 (1H, d, J=8.6 Hz), 7.71 (1H, dd, J=8.6, 2.4 Hz), 8.57 (1H, d, J=2.4 Hz).

### Example 62:

### 4-[(5-Chloropyridin-2-yl)methoxy]-1-(2-[4-(1-ethylpyrrolidin-2-yl)phenyl]ethyl)pyridin-2(1H)-one:

In the same manner as in Example 59 but changing 2-fluoroethyl trifluoromethanesulfonate used in Example 59 to iodoethane and changing the compound of Example 54 to the compound of Example 60, obtained was the compound of Example 62. ¹HNMR (400 MHz, CDCl₃, δ ppm): 1.01 (3H, t, J=7.1 Hz), 1.65-2.20 (6H, m), 2.53-2.63 (1H, m), 3.00 (2H, t, J=7.3 Hz), 3.12-3.20 (1H, m), 3.30-3.40 (1H, m), 4.07 (2H, t, J=7.3 Hz), 5.09 (2H, s), 5.81 (1H, dd, J=7.5, 2.8 Hz), 5.96 (1H, d, J=2.8 Hz), 6.78 (1H, d, J=7.5 Hz), 7.09 (2H, d, J=8.3 Hz), 7.23-7.30 (2H, m), 7.39 (1H, d, J=8.3 Hz), 7.71 (1H, dd, J=8.3, 2.4 Hz), 8.57 (1H, d, J=2.4 Hz).

### Example 63:

### 4-[(4-Fluorobenzyl)oxy]-1-(2- {4-[(methylamino)methyl]phenyl}ethyl)pyridin-2(1H)-one:

In the same manner as in Example 60 but changing the compound of Reference Example 3 used in Example 60 to the compound of Reference Example 2 and changing the compound of Reference Example 23 to the compound of Reference Example 24, the entitled compound was obtained:
¹HNMR (400 MHz, CDCl₃, δ ppm): 2.46 (3H, s), 3.00 (2H, t, J=7.1 Hz), 3.78 (2H, s), 4.06 (2H, t, J=7.1 Hz), 4.94 (2H, s), 5.78 (1H, dd, J=7.7, 2.8 Hz), 5.98 (1H, d, J=2.8 Hz), 6.77 (1H, d, J=7.7 Hz), 7.06-7.13 (4H, m), 7.24-7.30 (2H, m), 7.34-7.40 (2H, m).

### Examples 64 to 69:

In the same manner as in Example 55 but changing acetone used in Example 55 to corresponding carbonyl compounds and changing the compound of Example 54 to the compound of Example 63, obtained were the compounds of Examples 64 to 69:

### Example 64:

### 4-[(4-Fluorobenzyl)oxy]-1-[2-(4- {[isopropyl(methyl)amino]methyl}phenyl)ethyl]pyridin-2(1H)-one:

¹HNMR (400 MHz, CDCl₃, δ ppm): 1.10 (6H, d, J=6.3 Hz), 2.15 (3H, s), 2.82-2.94 (1H, m), 3.00 (2H, t, J=7.1 Hz), 3.47 (2H, s), 4.07 (2H, t, J=7.1 Hz), 4.94 (2H, s), 5.75 (1H, dd, J=7.5, 2.5 Hz), 5.98 (1H, d, J=2.5 Hz), 6.75 (1H, d, J=7.5 Hz), 7.05-7.13 (4H, m), 7.20-7.30 (2H, m), 7.34-7.40 (2H, m).

### Example 65:

### 1-[2-(4-{[Cyclobutyl(methyl)amino]methyl}phenyl)ethyl]-4-[(4-fluorobenzyl)oxy]pyridin-2(1H)-one:

¹HNMR (400 MHz, CDCl₃, δ ppm): 1.56-1.74 (3H, m), 1.86-1.96 (1H, m), 1.98 (3H, s), 2.00-2.07 (2H, m), 2.83 (1H, qui, J=7.6 Hz), 3.01 (2H, t, J=7.1 Hz), 3.32 (2H, s), 4.07 (2H, t, J=7.1 Hz), 4.94 (2H, s), 5.74 (1H, dd, J=7.7, 2.8 Hz), 5.98 (1H, d, J=2.8 Hz), 6.73 (1H, d, J=7.7 Hz), 7.05-7.12 (4H, m), 7.20 (2H, d, J=8.3 Hz), 7.34-7.40 (2H, m).

### Example 66:

### 1-[2-(4-{[Cyclopentyl(methyl)amino]methyl}phenyl)ethyl]-4-[(4-fluorobenzyl)oxy]pyridin-2(1H)-one:

¹HNMR (400 MHz, CDCl₃, δ ppm): 1.45-1.65 (4H, m), 1.66-1.76 (2H, m), 1.82-1.94 (2H, m), 2.14 (3H, s), 2.66-2.78 (1H, m), 3.01 (2H, t, J=7.1 Hz), 3.48 (2H, s), 4.07 (2H, t, J=7.1 Hz), 4.94 (2H, s), 5.74 (1H, q, J=7.8, 2.4 Hz), 5.98 (1H, d, J=2.4 Hz), 6.74 (1H, d, J=7.8 Hz), 7.06-7.10 (4H, m), 7.23 (2H, d, J=7.8 Hz), 7.34-7.39 (2H, m).

### Example 67:

### 4-[(4-Fluorobenzyl)oxy]-1-[2-(4- {[methyl(propyl)amino]methyl}phenyl)ethyl]pyridin-2(1H)-one:

¹HNMR (400 MHz, CDCl₃, δ ppm): 0.89 (3H, t, J=7.3 Hz), 1.48-1.68 (2H, m), 2.18 (3H, s), 2.28-2.35 (2H, m), 3.01 (2H, t, J=7.1 Hz), 3.44 (2H, s), 4.07 (2H, t, J=7.1 Hz), 4.94 (2H, s), 5.75 (1H, dd, J=7.5, 2.8 Hz), 5.98 (1H, d, J=2.8 Hz), 6.74 (1H, d, J=7.5 Hz), 7.05-7.12 (4H, m), 7.22 (2H, d, J=8.3 Hz), 7.35-7.39 (2H, m).

### Example 68:

### 4-[(4-Fluorobenzyl)oxy]-1-[2-(4-{[methyl(3-methylbutyl)amino]methyl}phenyl)ethyl]pyridin-2(1H)-one:

¹HNMR (400 MHz, CDCl₃, δ ppm): 0.87 (6H, d, J=6.8 Hz), 1.35-1.45 (2H, m), 1.55-1.62 (1H, m), 2.16 (3H, s), 2.32-2.39 (2H, m), 3.01 (2H, t, J=6.9 Hz), 3.43 (2H, s), 4.07 (2H, t, J=6.9 Hz), 4.94 (2H, s), 5.74 (1H, dd, J=7.7, 2.5 Hz), 5.98 (1H, d, J=2.5 Hz), 6.73 (1H, d, J=7.7 Hz), 7.05-7.12 (4H, m), 7.22 (2H, d, J=7.8 Hz), 7.34-7.40 (2H, m).

### Example 69:

### 4-[(4-Fluorobenzyl)oxy]-1-[2-(4- {[isobutyl(methyl)amino]methyl}phenyl)ethyl]pyridin-2(1H)-one:

¹HNMR (400 MHz, CDCl₃, δ ppm): 0.89 (6H, d, J=6.7 Hz), 1.80 (1H, qui, J=6.7 Hz), 2.07-2.16 (5H, m), 3.01 (2H, t, J=7.0 Hz), 3.41 (2H, s), 4.07 (2H, t, J=7.0 Hz), 4.94 (2H, s), 5.75 (1H, dd, J=7.8, 2.7 Hz), 5.98 (1H, d, J=2.7 Hz), 6.74 (1H, d, J=7.8 Hz), 7.05-7.11 (4H, m), 7.22-7.29 (2H, m), 7.34-7.40 (2H, m).

### Examples 70 and 71:

In the same manner as in Example 59 but changing 2-fluoroethyl trifluoromethanesulfonate sued in Example 59 to a corresponding alkylating agent and changing the compound of Example 54 to the compound of Example 63, obtained were compounds of Examples 70 and 71.

### Example 70:

### 4-[(4-Fluorobenzyl)oxy]-1-[2-(4-{[(2-fluoroethyl)(methyl)amino]methyl}phenyl)ethyl]pyridin-2(1H)-one:

¹HNMR (400 MHz, CDCl₃, δ ppm): 2.28 (3H, s), 2.68 (1H, t, J=5.1 Hz), 2.74 (1H, t, J=5.1 Hz), 3.01 (2H, t, J=7.1 Hz), 3.55 (2H, s), 4.07 (2H, t, J=7.1 Hz), 4.48 (1H, t, J=5.1 Hz), 4.60 (1H, t, J=5.1 Hz), 4.94 (2H, s), 5.75 (1H, dd, J=7.7, 2.8 Hz), 5.98 (1H, d, J=2.8 Hz), 6.75 (1H, d, J=7.7 Hz), 7.05-7.13 (4H, m), 7.24 (2H, t, J=6.3 Hz), 7.34-7.39 (2H, m).

### Example 71:

### 4-[(4-Fluorobenzyl)oxy]-1-[2-(4- {[(2-methoxyethyl)(methyl)amino]methyl}phenyl)ethyl]pyridin-2(1H)-one:

¹HNMR (400 MHz, CDCl₃,δ ppm): 2.24 (3H, s), 2.58 (2H, t, J=5.9 Hz), 3.01 (2H, t, J=7.1 Hz), 3.34 (3H, s), 3.51 (2H, t, J=5.9 Hz), 3.52 (2H, s), 4.07 (2H, t, J=7.1 Hz), 4.94 (2H, s), 5.75 (1H, dd, J=7.5, 2.6 Hz), 5.98 (1H, d, J=2.6 Hz), 6.74 (1H, d, J=7.5 Hz), 7.05-7.10 (4H, m), 7.23 (2H, d, J=7.8 Hz), 7.34-7.40 (2H, m).

### Example 72:

### 4-[(4-Fluorobenzyl)oxy]-1-(2- {4-[(propylamino)methyl]phenyl}ethyl)pyridin-2(1H)-one:

In the same manner as in Example 63 but changing the compound of Reference Example 24 used in Example 63 to the compound of Reference Example 25, obtained was the compound of Example 72.
¹HNMR (400 MHz, CDCl₃,δ ppm): 0.91 (3H, t, J=7.5 Hz), 1.60 (2H, six, J=7.5 Hz), 2.66 (2H, t, J=7.5 Hz), 2.99 (2H, t, J=7.1 Hz), 3.84 (2H, s), 4.04 (2H, t, J=7.1 Hz), 4.94 (2H, s), 5.78 (1H, dd, J=7.7, 2.6 Hz), 5.99 (1H, d, J=2.6 Hz), 6.78 (1H, d, J=7.7 Hz), 7.04-7.16 (4H, m), 7.24-7.34 (2H, m), 7.34-7.40 (2H, m).

### Examples 73 to 76:

In the same manner as in Example 55 but changing acetone used in Example 55 to corresponding carbonyl compounds and changing the compound of Example 54 to the compound of Example 72, obtained were the compounds of Examples 73 to 76.

### Example 73:

### 4-[(4-Fluorobenzyl)oxy]-1-[2-(4- {[isopropyl(propyl)amino]methyl}phenyl)ethyl]pyridin-2(1H)-one:

¹HNMR (400 MHz, CDCl₃, δ ppm): 0.88 (3H, t, J=6.8 Hz), 0.99 (6H, d, J=6.3 Hz), 1.34-1.42 (2H, m), 2.34 (2H, t, J=6.8 Hz), 2.85-2.95 (1H, m), 3.00 (2H, t, J=7.3 Hz), 3.50 (2H, s), 4.07 (2H, t, J=7.3 Hz), 4.94 (2H, s), 5.75 (1H, dd, J=7.8, 2.4 Hz), 5.98 (1H, d, J=2.4 Hz), 6.74 (1H, d, J=7.8 Hz), 7.04-7.12 (4H, m), 7.22-7.30 (2H, m), 7.34-7.40 (2H, m).

### Example 74:

### 1-(2-{4-[(Dipropylamino)methyl]phenyl}ethyl)-4-[(4-fluorobenzyl)oxy]pyridin-2(1H)-one:

¹HNMR (400 MHz, CDCl₃, δ ppm): 0.84-0.90 (6H, t, J=6.8 Hz), 1.48 (4H, brs), 2.36 (4H, brs), 3.01 (2H, t, J=7.1 Hz), 3.52 (2H, brs), 4.07 (2H, t, J=6.8 Hz), 4.94 (2H, s), 5.74 (1H, dd, J=7.7, 2.8 Hz), 5.98 (1H, d, J=2.8 Hz), 6.73 (1H, d, J=7.7 Hz), 7.04-7.12 (4H, m), 7.22-7.30 (2H, m), 7.34-7.40 (2H, m).

### Example 75:

### 4-[(4-Fluorobenzyl)oxy]-1-[2-(4- {[isobutyl(propyl)amino]methyl}phenyl)ethyl]pyridin-2(1H)-one:

¹HNMR (400 MHz, CDCl₃, δ ppm): 0.83 (3H, t, J=7.6 Hz), 0.86 (6H, d, J=6.6 Hz), 1.40-1.50 (2H, m), 1.75 (1H, qui, J=6.6 Hz), 2.10 (2H, d, J=7.3 Hz), 2.29 (2H, t, J=7.2 Hz), 3.00 (2H, t, J=7.1 Hz), 3.47 (2H, s), 4.07 (2H, t, J=7.1 Hz), 4.94 (2H, s), 5.74 (1H, dd, J=7.7, 2.6 Hz), 5.99 (1H, d, J=2.6 Hz), 6.72 (1H, d, J=7.7 Hz), 7.05-7.10 (4H, m), 7.22-7.28 (2H, m), 7.35-7.38 (2H, m).

### Example 76:

### 1-[2-(4- {[Cyclopentyl(propyl)amino]methyl}phenyl)ethyl]-4-[(4-fluorobenzyl)oxy]pyridin-2(1H)-one:

¹HNMR (400 MHz, CDCl₃, δ ppm): 0.80 (3H, t, J=7.3 Hz), 1.36-1.70 (8H, m), 1.70-1.85 (2H, m), 2.35-2.45 (2H, m), 2.96-3.14 (3H, m), 3.58 (2H, s), 4.07 (2H, t, J=6.8 Hz), 4.94 (2H, s), 5.70-5.78 (1H, m), 5.95-6.00 (1H, m), 6.73 (1H, d, J=7.8 Hz), 7.06-7.10 (4H, m), 7.20-7.30 (2H, m), 7.34-7.40 (2H, m).

### Examples 77 to 79:

In the same manner as in Example 59 but changing 2-fluoroethyl trifluoromethanesulfonate used in Example 59 to corresponding alkylating agents and changing the compound of Example 54 to the compound of Example 72, obtained were the compounds of Examples 77 to 79.

### Example 77:

### 1-[2-(4- {[ethyl(propyl)amino]methyl}phenyl)ethyl]-4-[(4-fluorobenzyl)oxy]pyridin-2(1H)-one:

¹HNMR (400 MHz, CDCl₃, δ ppm): 0.86 (3H, t, J=7.3 Hz), 0.99-1.10 (3H, m), 1.44-1.56 (2H, m), 2.2.30-2.44 (2H, m), 2.44-2.50 (2H, m), 3.01 (2H, t, J=7.1 Hz), 3.54 (2H, brs), 4.07 (2H, t, J=7.1 Hz), 4.94 (2H, s), 5.75 (1H, dd, J=7.7, 2.8 Hz), 5.98 (1H, d, J=2.8 Hz), 6.74 (1H, d, J=7.7 Hz), 7.04-7.14 (4H, m), 7.22-7.32 (2H, m), 7.35-7.39 (2H, m).

### Example 78:

### 4-[(4-Fluorobenzyl)oxy]-1-[2-(4- {[(2-fluoroethyl)(propyl)amino]methyl}phenyl)ethyl]pyridin-2(1H)-one:

¹HNMR (400 MHz, CDCl₃, δ ppm): 0.87 (3H, t, J=7.3 Hz), 1.46-1.58 (2H, m), 2.47 (2H, t, J=7.3 Hz), 2.72 (1H, t, J=5.3 Hz), 2.78 (1H, t, J=5.3 Hz), 3.01 (2H, t, J=7.1 Hz), 3.61 (2H, s), 4.07 (2H, t, J=7.1 Hz), 4.39 (1H, t, J=5.4 Hz), 4.51 (1H, t, J=5.4 Hz), 4.94 (2H, s), 5.75 (1H, dd, J=7.5, 2.6 Hz), 5.98 (1H, d, J=2.6 Hz), 6.73 (1H, d, J=7.5 Hz), 7.06-7.10 (4H, m), 7.22-7.30 (2H, m), 7.34-7.40 (2H, m).

### Example 79:

### 4-[(4-Fluorobenzyl)oxy]-1-[2-(4-[{(2-methoxyethyl)(propyl)amino]methyl}phenyl)ethyl]pyridin-2(1H)-one:

¹HNMR (400 MHz, CDCl₃, δ ppm): 0.88 (3H, t, J=7.3 Hz), 1.44-1.56 (2H, m), 2.41-2.49 (2H, m), 2.67 (2H, t, J=6.1 Hz), 3.03 (2H, t, J=7.1 Hz), 3.34 (3H, s), 3.48 (2H, t, J=6.1 Hz), 3.62 (2H, s), 4.10 (2H, t, J=7.1 Hz), 4.97 (2H, s), 5.78 (1H, dd, J=7.7, 2.8 Hz), 6.01 (1H, d, J=2.8 Hz), 6.77 (1H, d, J=7.7 Hz), 7.08-7.14 (4H, m), 7.24-7.30 (2H, m), 7.38-7.42 (2H, m).

### Example 80:

### 1-(2- {4-[(Diethylamino)methyl]-3-fluorophenyl}ethyl)-4-[(4-fluorobenzyl)oxy]pyridin-2(1H)-one:

### (1) Production of 4-[(4-fluorobenzyl)oxy]-1-[2-(3-fluoro-4- {[(4-methoxybenzyl)oxy]methyl}phenyl)ethyl]pyridin-2(H)-one:

In the same manner as in Example 14-(1) but changing the compound of Reference Example 21 used in Example 14-(1) to the compound of Reference Example 26 and changing the compound of Reference Example 3 to the compound of Reference Example 2, obtained was the compound of Example 80-(1).
Mass Spectrum (ESI): 492.3 (M+H).

### (2) Production of 4-[(4-Fluorobenzyl)oxy]-1-{2-[3-fluoro-4-(hydroxymethyl)phenyl]ethyl}pyridin-2(1H)-one:

2,3-Dichloro-5,6-dicyano-1,4-benzoquinone (54.0 mg, 0.24 mmol) was added to a chloroform/water mixed solution [chloroform (5 mL), water (0.5 mL)]] of the compound obtained in the above (1) (100.0 mg, 0.20 mmol), and stirred at room temperature for 1.5 hours. Next, aqueous saturated sodium hydrogencarbonate solution was added to the reaction liquid, and extracted with chloroform. The obtained organic layer was dried with anhydrous sodium sulfate, and filtered. The obtained filtrate was concentrated under reduced pressure, and the residue was purified by silica gel column chromatography (chloroform:methanol = 49:1) to obtain the entitled compound (67.0 mg, 90 %).
Mass Spectrum (ESI): 372.2 (M+H).

### (3) Production of 1-(2- {4-[(diethylamino)methyl]-3-fluorophenyl}ethyl)-4-[(4-fluorobenzyl)oxy]pyridin-2(1H)-one:

In the same manner as in Example 14-(3) and 14-(4) but changing the compound used in Example 14-(3) to the compound obtained in the above (2), the compound of Example 80 was obtained.
¹HNMR (400 MHz, CDCl₃,δ ppm): 1.05 (6H, t, J=7.3 Hz), 2.52 (4H, q, J=7.3 Hz), 3.00 (2H, t, J=7.0 Hz), 3.59 (2H, s), 4.06 (2H, t, J=7.0 Hz), 4.94 (2H, s), 5.77 (1H, dd, J=7.5, 2.8 Hz), 5.98 (1H, d, J=2.8 Hz), 6.76 (1H, d, J=7.5 Hz), 6.82 (1H, dd, J=10.7, 2.0 Hz), 6.89 (1H, dd, J=7.8, 2.0 Hz), 7.08 (2H, t, J=8.8 Hz), 7.7.28-7.40 (3H, m).

### Example 81:

### 1- {2-[4-(Azetidin-1-ylmethyl)-3-fluorophenyl]ethyl}-4- [(4-fluorobenzyl)oxy]pyridin-2(1H)-one:

In the same manner as in Example 80 but changing diethylamine used in Example 80-(3) to azetidine, the compound of Example 81 was obtained.
¹HNMR (400 MHz, CDCl₃, δ ppm): 2.08 (2H, t, J=6.8 Hz), 3.00 (2H, t, J=7.1 Hz), 3.24 (4H, t, J=6.8 Hz), 3.58 (2H, s), 4.06 (2H, t, J=7.1 Hz), 4.94 (2H, s), 5.77 (1H, dd, J=7.4, 2.5 Hz), 5.98 (1H, d, J=2.5 Hz), 6.75 (1H, d, J=7.4 Hz), 6.80-6.85 (1H, m), 6.88 (1H, dd, J=7.8, 1.5 Hz), 7.08 (2H, t, J=8.8 Hz), 7.20-7.25 (1H, m), 7.34-7.40 (2H, m).

### Example 82:

### 1-(2-{4-[1-(Diethylamino)ethyl]phenyl}ethyl)-4-[(4-fluorobenzyl)oxy]pyridin-2(1H)-one:

### (1) Production of 4-[(4-fluorobenzyl)oxy]-1-(2-{4-[1-(methoxymethoxy)ethyl]phenyl} ethyl)pyridin-2(1H)-one:

In the same manner as in Example 14-(1) but changing the compound of Reference Example 21 used in Example 14-(1) to the compound of Reference Example 27 and changing the compound of Reference Example 3 to the compound of Reference Example 2, obtained was the compound of Example 82-(1).
¹HNMR (400 MHz, CDCl₃, δ ppm): 1.46 (3H, d, J=6.6 Hz), 3.02 (2H, t, J=7.0 Hz), 3.36 (3H, s), 4.07 (2H, t, J=7.0 Hz), 4.52 (1H, d, J=6.6 Hz), 4.57 (1H, d, J=6.6 Hz), 4.72 (1H, q, J=6.6 Hz), 4.94 (2H, s), 5.77 (1H, dd, J=7.2, 3.0 Hz), 5.99 (1H, d, J=3.0 Hz), 6.77 (1H, d, J=7.2 Hz), 7.06-7.16 (4H, m), 7.24-7.30 (2H, m), 7.34-7.40 (2H, m).

### (2) Production of 4-[(4-fluorobenzyl)oxy]-1-(2-{4-[1-(hydroxyethyl)phenyl]ethyl}pyridin-2(1H)-one:

A 10 % hydrogen chloride/methanol solution (5 mL) of the compound obtained in the above (1) was stirred at room temperature for 3 days. The reaction liquid was concentrated under reduced pressure, then aqueous saturated ammonium chloride solution was added, and extracted with ethyl acetate. The obtained organic layer was washed with saturated saline, dried with anhydrous sodium sulfate, and filtered. The obtained filtrate was concentrated under reduced pressure, and the residue was purified by silica gel column chromatography (chloroform:methanol = 10:0 to 19:1) to obtain the entitled compound (189 mg, 46 %).
Mass Spectrum (ESI): 368.2 (M+H).

### (3) Production of 1- {2-{4-[1-(diethylamino)ethyl]phenyl}ethyl)-4-[(4-fluorobenzyl)oxy]pyridin-2(1H)-one:

In the same manner as in Example 14-(3) and 14-(4) but changing the compound used in Example 14-(3) to the compound obtained in the above (2), the compound of Example 82 was obtained.
¹HNMR (400 MHz, CDCl₃, δ ppm): 0.98 (6H, t, J=7.1 Hz), 1.33 (3H, d, J=6.4 Hz), 2.46-2.56 (4H, m), 3.00 (2H, t, J=7.1 Hz), 3.73-3.82 (1H, m), 4.07 (2H, dt, J=1.6, 7.1 Hz), 4.94 (2H, s), 5.74 (1H, dd, J=7.5, 2.6 Hz), 5.98 (1H, d, J=2.6 Hz), 6.74 (1H, d, J=7.5 Hz), 7.06-7.11 (4H, m), 7.25-7.30 (2H, m), 7.34-7.40 (2H, m).

### Example 83:

### 4-[(4-Fluorobenzyl)oxy]-1-[2-(4-{1-[methyl(propyl)amino]ethyl}phenyl)ethyl]pyridin-2(1H)-one:

In the same manner as in Example 82 but changing diethylamine used in Example 82-(3) to N-methylpropylamine, the compound of Example 83 was obtained.
¹HNMR (400 MHz, CDCl₃, δ ppm): 0.83 (3H, t, J=7.3 Hz), 1.33 (3H, d, J=6.8 Hz), 1.40-1.52 (2H, m), 2.15-2.23 (4H, m), 2.28-2.38 (1H, m), 3.01 (2H, t, J=7.1 Hz), 3.53 (1H, q, J=6.7 Hz), 4.07 (2H, td, J=7.1, 3.6 Hz), 4.94 (2H, s), 5.74 (1H, dd, J=2.6, 7.5 Hz), 5.98 (1H, d, J=2.6 Hz), 6.73 (1H, d, J=7.5 Hz), 7.05-7.12 (4H, m), 7.22 (2H, d, J=8.3 Hz), 7.34-7.40 (2H, m).

### Example 84:

### 4-[(4-Fluorobenzyl)oxy]-1-{2-[4-(1-pyrrolidin-1-ylethyl)phenyl]ethyl}pyridin-2(1H)-one:

### (1) Production of 1-{2-[4-(1,3-dioxan-2-yl)phenyl]ethyl}-4-[(4-fluorobenzyl)oxy]pyridin-2(1H)-one:

In the same manner as in Example 14-(1) but changing the compound of Reference Example 21 used in Example 14-(1) to the compound of Reference Example 20 and changing the compound of Reference Example 3 to the compound of Reference Example 2, obtained was the compound of Example 84-(1).
Mass Spectrum (ESI): 410.3 (M+H).

### (2) Production of 4-[(4-fluorobenzyl)oxy]-1-{2-[4-(1-hydroxyethyl)phenyl]ethyl}pyridin-2(1H)-one:

Trifluoroacetic acid (3.7 mL) and water (0.37 mL) were added to a chloroform solution (7.4 mL) of the compound obtained in the above (1) (371 mg, 0.91 mmol), and stirred at room temperature for 3 hours. The reaction liquid was concentrated under reduced pressure, then aqueous saturated ammonium chloride solution was added, and extracted with chloroform. The obtained organic layer was dried with anhydrous sodium sulfate, and filtered. The obtained filtrate was concentrated under reduced pressure, then with cooling with ice, methylmagnesium bromide (3 M ether solution, 1.5 mL) was added to a THF solution (10 mL) of the residue, and stirred overnight at 0°C to room temperature. Next, aqueous saturated ammonium chloride solution was added to the reaction liquid, and extracted with ethyl acetate. The obtained organic layer was dried with anhydrous sodium sulfate, and filtered. The obtained filtrate was concentrated under reduced pressure, then the residue was purified by silica gel column chromatography (chloroform:methanol= 50:1) to obtain the entitled compound (172 mg, 52 %). Mass Spectrum (ESI): 368.3 (M+H).

### (3) Production of 4-[(4-fluorobenzyl)oxy]-1-{2-[4-(1-pyrrolidin-1-ylethyl)phenyl]ethyl}pyridin-2(1H)-one:

In the same manner as in Example 82-(3) but changing diethylamine used in Example 82-(3) to pyrrolidine, the compound of Example 84 was obtained.
¹HNMR (400 MHz, CDCl₃,δ ppm): 1.39 (3H, d, J=6.7 Hz), 1.70-1.80 (4H, m), 2.36 (2H, brs), 2.54 (2H, brs), 3.00 (2H, t, J=7.0 Hz), 3.17 (1H, brs), 4.07 (2H, td, J=7.0, 3.0 Hz), 4.94 (2H, s), 5.75 (1H, dd, J=7.6, 2.5 Hz), 5.98 (1H, d, J=2.5 Hz), 6.76 (1H, d, J=7.6 Hz), 7.04-7.12 (4H, m), 7.22-7.28 (2H, m), 7.34-7.40 (2H, m).

### Example 85:

### 4-[(5-Chloropyridin-2-yl)methoxy]-1-(2-{4-[1-(diethylamino)ethyl]phenyl}ethyl)pyridin-2(1H)-one:

### (1) Production of 4- {2-[4-[(5-chloropyridin-2-yl)methoxy]-2-oxopyridin-1(2H)-yl]ethyl}benzaldehyde:

Manganese dioxide (881.2 mg, 8.62 mmol) was added to a50 % chloroform/acetone (100 ml) solution of the compound of Example 14-(2) (810.0 mg, 2.18 mmol), and stirred at room temperature for 23 hours. Next, the reaction liquid was filtered through Celite, the obtained filtrate was evaporated under reduced pressure. The obtained residue was purified by silica gel column chromatography (chloroform:methanol = 10:0 to 19:1) to obtain the entitled compound (682.9 mg, 85 %).
Mass Spectrum (APCI): 369.1 (M+H).

### (2) Production of 4-[(5-chloropyridin-2-yl)methoxy]-1-{2-[4-(1-hydroxyethyl)phenyl]ethyl}pyridin-2(1H)-one:

At -78°C, methylmagnesium bromide (1.0 M THF solution, 8.0 ml, 8.0 mmol) was dropwise added to a THF (60 ml) solution of the compound obtained in the above (1) (682.9 mg, 1.85 mmol), stirred for 2 hours, then heated up to room temperature, and stirred for 16 hours. Next, aqueous 10 % hydrochloric acid solution was added to the reaction liquid, extracted with ethyl acetate, the obtained organic layer was washed with saturated saline, dried with anhydrous sodium sulfate, and filtered. The obtained filtrate was concentrated under reduced pressure, and the residue was purified by silica gel column chromatography (chloroform:methanol = 10:0 to 19:1) to obtain the entitled compound (780.4 mg, 100 %).
Mass Spectrum (ESI): 385.1 (M+H).

### (3) Production of 4-[(5-chloropyridin-2-yl)methoxy]-1-(2-{4-[1-(diethylamino)ethyl]phenyl} ethyl)pyridin-2(1H)-one:

In the same manner as in Example 82-(3) but changing the compound used in Example 82-(3) to the compound obtained in the above (2), the compound of Example 85 was obtained.
¹HNMR (400 MHz, CDCl₃, δ ppm): 0.91-1.08 (6H, m), 1.25 (3H, s), 2.40-2.60 (4H, m), 3.00 (2H, t, J=7.1 Hz), 3.71-3.82 (1H, m), 4.07 (2H, t, J=7.1 Hz), 5.09 (2H, s), 5.77-5.81 (1H, m), 5.96 (1H, d, J=2.9 Hz), 6.75 (1H, d, J=7.3 Hz), 7.08 (2H, d, J=7.8 Hz), 7.22-7.30 (2H, m), 7.39 (1H, d, J=8.3 Hz), 7.71 (1H, dd, J=8.3, 2.0 Hz), 8.57 (1H, d, J=2.0 Hz).

### Examples 86 and 87:

In the same manner as in Example 85 but changing diethylamine used in Example 85-(3) to corresponding amine compounds, the compounds of Examples 86 and 87 were obtained.

### Example 86:

### 4-[(5-Chloropyridin-2-yl)methoxy]-1-(2-(4-{1-[methyl(propyl)amino]ethyl}phenyl)ethyl]pyridin-2(1H)-one:

¹HNMR (400 MHz, CDCl₃, δ ppm): 0.84 (3H, t, J=7.4 Hz), 1.24-1.62 (5H, m), 2.10-2.41 (5H, m), 3.01 (2H, t, J=7.0 Hz), 3.50-3.59 (1H, m), 4.08 (2H, t, J=7.0 Hz), 5.09 (2H, s), 5.78-5.81 (1H, m), 5.97 (1H, d, J=2.7 Hz), 6.75 (1H, d, J=7.8 Hz), 7.09 (2H, d, J=7.4 Hz), 7.21-7.28 (2H, m), 7.40 (1H, d, J=8.6 Hz), 7.69-7.73 (1H, m), 8.57 (1H, d, J=2.3 Hz).

### Example 87:

### 1-{2-[4-(1-Azetidin-1-ylethyl)phenyl]ethyl}-4-[(5-chloropyridin-2-yl)methoxy]pyridin-2(1H)-one:

¹HNMR (400 MHz, CDCl₃, δ ppm): 1.17-1.26 (3H, m), 1.97-2.09 (3H, m), 2.97-3.23 (6H, m), 4.06 (2H, t, J=7.2 Hz), 5.09 (2H, s), 5.79-5.82 (1H, m), 5.96 (1H, d, J=2.7 Hz), 6.76 (1H, d, J=7.0 Hz), 7.09 (2H, d, J=7.4 Hz), 7.22 (2H, d, J=7.4 Hz), 7.40 (1H, d, J=8.6 Hz), 7.69-7.73 (1H, m), 8.57 (1H, d, J=2.0 Hz).

### Example 88:

### 4-[(5-Chloropyridin-2-yl)methoxy]-1-(2-[4-(1-pyrrolidin-1-ylethyl)phenyl]ethyl)pyridin-2(1H)-one:

In the same manner as in Example 85 but changing diethylamine used in Example 85-(3) to pyrrolidine, a racemate of Example 88 was obtained. Next, this was further purified by high-performance chromatography (CHIRALPAK^{™} OJ, hexane:ethanol:diethylamine = 75:25:0.025) to obtain the entitled compounds (fast) and (slow).
(fast)
¹HNMR (400 MHz, CDCl₃, δ ppm): 1.38 (3H, d, J=6.3 Hz), 1.72-1.79 (4H, m), 2.29-2.37 (2H, m), 2.50-2.56 (2H, m), 3.00 (2H, t, J=7.1 Hz), 3.15 (1H, q, J=6.3 Hz), 4.04-4.09 (2H, m), 5.09 (2H, s), 5.80 (1H, dd, J=7.8, 2.9 Hz), 5.96 (1H, d, J=2.9 Hz), 6.77 (1H, d, J=7.8 Hz), 7.08 (2H, d, J=8.3 Hz), 7.25 (2H, d, J=8.3 Hz), 7.39 (1H, d, J=8.3 Hz), 7.71 (1H, dd, J=8.3, 2.0 Hz), 8.57 (1H, d, J=2.0 Hz).
(slow)
¹HNMR (400 MHz, CDCl₃,δ ppm): 1.38 (3H, d, J=6.3 Hz), 1.72-1.79 (4H, m), 2.30-2.37 (2H, m), 2.50-2.57 (2H, m), 3.00 (2H, t, J=7.1 Hz), 3.12-3.18 (1H, m), 4.04-4.09 (2H, m), 5.09 (2H, s), 5.80 (1H, dd, J=7.8, 2.9 Hz), 5.96 (1H, d, J=2.9 Hz), 6.77 (1H, d, J=7.8 Hz), 7.08 (2H, d, J=8.3 Hz), 7.24 (2H, d, J=8.3 Hz), 7.40 (1H, d, J=8.3 Hz), 7.71 (1H, dd, J=8.3, 2.0 Hz), 8.57 (1H, d, J=2.0 Hz).

### Example 89:

### 4-[(5-Chloropyridin-2-yl)methoxy]-1-(2-{4-[1-(diethylamino)propyl]phenyl}ethyl)pyridin-2(1H)-one:

In the same manner as in Example 85 but changing methylmagnesium bromide used in Example 85-(2) to ethylmagnesium bromide, the compound of Example 89 was obtained. ¹HNMR (400 MHz, CDCl₃, δ ppm): 0.72 (3H, t, J=7.3 Hz), 0.98 (6H, t, J=7.1 Hz), 1.63-1.94 (2H, m), 2.29-2.39 (2H, m), 2.57-2.64 (2H, m), 3.00 (2H, t, J=7.1 Hz), 3.44-3.48 (1H, m), 4.03-4.12 (2H, m), 5.09 (2H, s), 5.76 (1H, dd, J=7.8, 2.9 Hz), 5.96 (1H, d, J=2.9 Hz), 6.69 (1H, d, J=7.3 Hz), 7.06 (2H, d, J=7.8 Hz), 7.15 (2H, d, J=7.8 Hz), 7.39 (1H, d, J=8.3 Hz), 7.71 (1H, dd, J=8.8, 2.4 Hz), 8.57 (1H, d, J=2.4 Hz).

### Example 90:

### 1-(2- {4-[2-(Diethylamino)ethoxy]phenyl}ethyl)-4-[(4-fluorobenzyl)oxy]pyridin-2(1H)-one:

### (1) Production of 1-[2-(4-{[tert-butyl(dimethyl)silyl]oxy}phenyl)ethyl]-4-[(4-fluorobenzyl)oxy]pyridin-2(1H)-one:

In the same manner as in Example 1 but changing the compound of Reference Example 16 used in Example 1 to 2-(4-{[tert-butyl(dimethyl)silyl]oxy}phenyl)ethanol [Org. Lett., 5(2), 121(2003)], the compound of Example 90-(1) was obtained.
Mass Spectrum (ESI): 454.3 (M+H).

### (2) Production of 4-[(4-fluorobenzyl)oxy]-1-[2-(4-hydroxyphenyl)ethyl]pyridin-2(1H)-one:

In the same manner as in Reference Example 19-(8) but changing the compound used in Reference Example 19-(8) to the compound obtained in the above (1), the compound of Example 90-(2) was obtained.
Mass Spectrum (ESI): 340.2 (M+H).

### (3) Production of 1-(2-{4-[2-(diethylamino)ethoxy]phenyl}ethyl)-4-[(4-fluorobenzyl)oxy]pyridin-2(1H)-one:

In the same manner as in Example 1 but changing the compound of Reference Example 16 used in Example 1 to 2-(diethylamino)ethanol and changing the compound of Reference Example 2 to the compound obtained in the above (2), the compound of Example 90 was obtained.
¹HNMR (400 MHz, CDCl₃, δ ppm): 1.07 (6H, t, J=7.1 Hz), 2.64 (4H, q, J=7.1 Hz), 2.86 (2H, t, J=6.3 Hz), 2.95 (2H, t, J=7.1 Hz), 4.00-4.10 (4H, m), 4.94 (2H, s), 5.76 (1H, dd, J=7.5, 2.5 Hz), 5.98 (1H, d, J=2.5 Hz), 6.73 (1H, d, J=7.5 Hz), 6.80-6.83 (2H, m), 7.01-7.11 (4H, m), 7.35-7.40 (2H, m).

### Example 91:

### 4-[(4-Fluorobenzyl)oxy]-1-{2-4-(2-pyrrolidin-1-ylethoxy)phenyl]ethyl}pyridin-2(1H)-one:

In the same manner as in Example 90 but changing 2-(diethylamino)ethanol used in Example 90-(3) to 2-pyrrolidin-1-ylethanol, the compound of Example 91 was obtained.
¹HNMR (400 MHz, CDCl3,δ ppm): 1.79-1.83 (4H, m), 2.60-2.70 (4H, m), 2.89 (2H, t, J=5.8 Hz), 2.96 (2H, t, J=7.0 Hz), 4.04 (2H, t, J=7.0 Hz), 4.08 (2H, t, J=5.8 Hz), 4.94 (2H, s), 5.77 (1H, dd, J=7.7, 2.5 Hz), 5.98 (1H, d, J=2.5 Hz), 6.73 (1H, d, J=7.7 Hz), 6.80-6.85 (2H, m), 7.01-7.12 (4H, m), 7.34-7.40 (2H, m).

### Example 92:

### 1-(2- {4-[2-(Diethylamino)ethyl]phenyl}ethyl)-4-[(4-fluorobenzyl)oxy]pyridin-2(1H)-one:

### (1) Production of 1- {2-[4-(2-{[tert-butyl(dimethyl)silyl]oxy}ethyl)phenyl]ethyl}-4-[(4-fluorobenzyl)oxy]pyridin-2(1H)-one:

In the same manner as in Example 1 but changing the compound of Reference Example 16 used in Example 1 to 2-[4-(2-{[tert-butyl(dimethyl)silyl]oxy}ethyl)phenyl]ethanol (WO98/43956), the compound of Example 92-(1) was obtained.
Mass Spectrum (ESI): 482.1 (M+H).

### (2) Production of 4-[(4-Fluorobenzyl)oxy]-1-{2-[4-(2-hydroxyethyl)phenyl]ethyl)pyridin-2(1H)-one:

In the same manner as in Reference Example 19-(8) but changing the compound used in Reference Example 19-(8) to the compound obtained in the above (1), the compound of Example 92-(2) was obtained.
Mass Spectrum (ESI): 368.3 (M+H).

### (3) Production of 2-(4- {2-[4-[(4-fluorobenzyl)oxy]-2-oxopyridin-1(2H)-yl]ethyl}phenyl)ethyl 4-methylbenzenesulfonate:

In the same manner as in Reference Example 21-(3) but changing the compound used in Reference Example 21-(3) to the compound obtained in the above (2), and without purification of the product, the compound of Example 92-(3) was obtained, and this was used in the next reaction.

### (4) Production of 1-(2-{4-[2-(diethylamino)ethyl]phenyl}ethyl)-4-[(4-fluorobenzyl)oxy]pyridin-2(1H)-one:

In the same manner as in Example 14-(4) but changing the compound used in Example 14-(4) to the compound obtained in the above (3), the compound of Example 92 was obtained.
¹HNMR (400 MHz, CDCl₃, δ ppm): 1.07 (6H, t, J=7.3 Hz), 2.56-2.78 (8H, m), 2.99 (2H, t, J=7.1 Hz), 4.06 (2H, t, J=7.1 Hz), 4.94 (2H, s), 5.78 (1H, dd, J=7.4, 2.8 Hz), 5.98 (1H, d, J=2.8 Hz), 6.77 (1H, d, J=7.4 Hz), 7.06-7.13 (6H, m), 7.37 (2H, dd, J=8.3, 5.4 Hz).

### Example 93:

### 4-[(4-Fluorobenzyl)oxy]-1-{2-4-(2-pyrrolidin-1-ylethyl)phenyl]ethyl}pyridin-2(1H)-one:

In the same manner as in Example 92 but changing diethylamine used in Example 92-(3) to pyrrolidine, the compound of Example 93 was obtained.
¹HNMR (400 MHz, CDCl₃, δ ppm): 1.79-1.85 (4H, s), 2.54-2.64 (4H, s), 2.65-2.74 (2H, m), 2.78-2.86 (2H, m), 2.99 (2H, t, J=7.0 Hz), 4.06 (2H, t, J=7.0 Hz), 4.94 (2H, s), 5.78 (1H, dd, J=7.4, 2.7 Hz), 5.98 (1H, d, J=2.7 Hz), 6.77 (1H, d, J=7.4 Hz), 7.06-7.14 (6H, m), 7.37 (2H, t, J=7.0 Hz).

### Example 94:

### 4-[(4-Fluorobenzyl)oxy]-1-[2-(4-pyrrolidin-1-ylphenyl)ethyl]pyridin-2(1H)-one:

### (1) Production of 1-[2-(4-bromophenyl)ethyl]-4-[(4-fluorobenzyl)oxy]pyridin-2(1H)-one:

In the same manner as in Example 1 but changing the compound of Reference Example 16 used in Example 1 to 2-(4-bromophenyl)ethanol, the compound of Example 94-(1) was obtained.
Mass Spectrum (ESI): 402.1 (M+H).

### (2) Production of 4-[(4-fluorobenzyl)oxy]-1-[2-(4-pyrrolidin-1-ylphenyl)ethyl]pyridin-2(1H)-one:

Tris(dibenzylideneacetone)dipalladium(0) (7 mg, 0.008 mmol), BINAP (14 mg, 0.02 mmol), pyrrolidine (0.025 mL, 0.30 mmol) and sodium tert-butoxide (34 mg, 0.35 mmol) were added to a toluene solution (5 ml) of the compound obtained in the above (1) (100 mg, 0.25 mmol), and stirred overnight at 80°C in the presence of nitrogen. Next, aqueous saturated sodium hydrogencarbonate solution was added to the reaction liquid, and extracted with ethyl acetate. The obtained organic layer was dried with anhydrous sodium sulfate, and filtered. The obtained filtrate was concentrated under reduced pressure, and the residue was purified by silica gel column chromatography (chloroform:methanol = 49:1) to obtain the entitled compound (68 mg, 68 %).
¹HNMR (400 MHz, CDCl₃, δ ppm): 1.98-2.02 (4H, m), 2.90 (2H, t, J=6.9 Hz), 3.23-3.28 (4H, m), 4.03 (2H, t, J=6.9 Hz), 4.94 (2H, s), 5.78 (1H, dd, J=7.6, 2.7 Hz), 5.99 (1H, d, J=2.7 Hz), 6.49 (2H, d, J=8.2 Hz), 6.78 (1H, d, J=7.6 Hz), 6.99 (2H, d, J=8.2 Hz), 7.06-7.12 (2H, m), 7.34-7.40 (2H, m).

### Example 95:

### 1-(2- {4-[3-(dimethylamino)pyrrolidin-1-yl]phenyl}ethyl} -4-[(4-fluorobenzyl)oxy]pyridin-2(1H)-one:

In the same manner as in Example 94 but changing pyrrolidine used in Example 94-(2) to N,N-dimethylpyrrolidine-3-amine, the compound of Example 95 was obtained.
¹HNMR (400 MHz, CDCl₃, δ ppm): 1.85-2.00 (1H, m), 2.15-2.25 (1H, m), 2.31 (6H, s), 2.78-2.90 (1H, m), 2.91 (2H, t, J=7.1 Hz), 3.12 (1H, t, J=8.5 Hz), 3.29 (1H, dt, J=7.2 Hz, 10 Hz), 3.35-3.50 (2H, m), 4.03 (2H, t, J=7.1 Hz), 4.94 (2H, s), 5.78 (1H, dd, J=7.8, 2.8 Hz), 5.99 (1H, d, J=2.8 Hz), 6.47 (2H, d, J=8.3 Hz), 6.78 (1H, d, J=7.8 Hz), 6.97-7.02 (2H, m), 7.04-7.10 (2H, m), 7.35-7.40 (2H, m).

### Example 96:

### 4-[(4-Fluorobenzyl)oxy]-1-[2-(3-methoxy-4- {[methyl(propyl)amino]methyl}phenyl)ethyl]pyridin-2(1H)-one:

### (1) Production of methyl 4- {2-[4-[(4-fluorobenzyl)oxy]-2-oxopyridin-1(2H)-yl]ethyl}-2-methoxybenzoate:

In the same manner as in Example 14-(1) but changing the compound of Reference Example 21 used in Example 14-(1) to the compound of Reference Example 28, and changing the compound of Reference Example 3 to the compound of Reference Example 2, obtained was the compound of Example 95-(1).
Mass Spectrum (ESI): 412.1 (M+H).

### (2) Production of 4-[(4-fluorobenzyl)oxy]-1- {2-[4-(hydroxymethyl)-3-methoxyphenyl]ethyl}pyridin-2(1H)-one:

At -78°C, a THF solution (20 mL) of the compound obtained in the above (1) was gradually added to a THF solution (5 mL) of lithiumaluminium hydride (31 mg, 0.82 mmol), and stirred at -78°C to -10°C for 1.5 hours. Next, sodium sulfate 10-hydrate was added to the reaction liquid, and stirred overnight at room temperature. The reaction liquid was filtered through Celite, the obtained filtrate was concentrated under reduced pressure, and dried to obtain the entitled compound (260 mg, 100 %).
Mass Spectrum (ESI): 384.1 (M+H).

### (3) Production of 4-[(4-fluorobenzyl)oxy]-1-[2-(3-methoxy-4-{[methyl(propyl)amino]methyl}phenyl)ethyl]pyridin-2(1H)-one:

In the same manner as in Example 14-(3) and 14-(4) but changing the compound used in Example 14-(3) to the compound obtained in the above (2) and changing diethylamine used in Example 14-(4) to N-methylpropylamine, obtained was the compound of Example 96. ¹HNMR (400 MHz, CDCl₃, δ ppm): 0.90 (3H, t, J=7.3 Hz), 1.50-1.65 (2H, m), 2.20 (3H, s), 2.36 (2H, t, J=7.8 Hz), 3.01 (2H, t, J=7.0 Hz), 3.47 (2H, s), 3.75 (3H, s), 4.08 (2H, t, J=7.0 Hz), 4.94 (2H, s), 5.76 (1H, dd, J=7.7, 2.6 Hz), 5.99 (1H, d, J=2.6 Hz), 6.61 (1H, s), 6.72 (1H, d, J=7.5 Hz), 6.75 (1H, d, J=7.7 Hz), 7.08 (2H, t, J=8.7 Hz), 7.23 (1H, d, J=7.5 Hz), 7.37 (2H, dd, J=8.7, 5.6 Hz).

### Examples 97 to 99:

In the same manner as in Example 96 but changing N-methylpropylamine used in Example 96-(3) to corresponding amine compounds, obtained were the compounds of Examples 97 to 99.

### Example 97:

### 1-(2- {4-[(Diethylamino)methyl]-3-methoxyphenyl}ethyl)-4-[(4-fluorobenzyl)oxy]pyridin-2(1H)-one:

¹HNMR (400 MHz, CDCl₃,δ ppm): 1.05 (6H, t, J=7.2 Hz), 2.54 (4H, q, J=7.2 Hz), 3.00 (2H, t, J=7.0 Hz), 3.55 (2H, s), 3.74 (3H, s), 4.08 (2H, t, J=7.0 Hz), 4.94 (2H, s), 5.76 (1H, dd, J=7.6, 2.8 Hz), 5.99 (1H, d, J=2.8 Hz), 6.58-6.62 (1H, m), 6.70-6.75 (1H, m), 6.76 (1H, d, J=7.6 Hz), 7.08 (2H, t, J=8.5 Hz), 7.30 (1H, d, J=7.8 Hz), 7.37 (2H, dd, J=8.5, 5.1 Hz).

### Example 98:

### 1-{2-[4-(azetidin-1-ylmethyl)-3-methoxyphenyl]ethyl}-4-[(4-fluorobenzyl)oxy]pyridin-2(1H)-one:

¹HNMR (400 MHz, CDCl₃, δ ppm): 2.09 (2H, qui, J=6.8 Hz), 3.00 (2H, t, J=7.0 Hz), 3.20-3.32 (4H, m), 3.56 (2H, s), 3.75 (3H, s), 4.07 (2H, t, J=7.0 Hz), 4.94 (2H, s), 5.76 (1H, dd, J=7.7, 2.6 Hz), 5.98 (1H, d, J=2.6 Hz), 6.59 (1H, d, J=1.5 Hz), 6.71 (1H, dd, J=7.6, 1.5 Hz), 6.75 (1H, d, J=7.7 Hz), 7.06-7.12 (2H, m), 7.16 (1H, d, J=7.6 Hz), 7.33-7.40 (2H, m).

### Example 99:

### 4-[(4-Fluorobenzyl)oxy]-1-{2-[3-methoxy-4-(pyrrolidin-1-ylmethyl)phenyl]ethyl}-pyridin-2(1H)-one:

¹HNMR (400 MHz, CDCl₃, δ ppm): 1.70-1.85 (4H, m), 2.56 (4H, brs), 3.00 (2H, t, J=7.1 Hz), 3.64 (2H, s), 3.75 (4H, s), 4.08 (2H, t, J=7.1 Hz), 4.94 (2H, s), 5.77 (1H, dd, J=7.7, 2.8 Hz), 5.99 (1H, d, J=2.8 Hz), 6.61 (1H, d, J=1.5 Hz), 6.72 (1H, dd, J=7.6, 1.5 Hz), 6.76 (1H, d, J=7.7 Hz), 7.04-7.12 (2H, m), 7.22-7.30 (2H, m), 7.35-7.39 (2H, m).

### Example 100:

### 1-(2- {4-[(Diethylamino)methyl]-2-methoxyphenyl}ethyl)-4-[(4-fluorobenzyl)oxy]pyridin-2(1H)-one:

In the same manner as in Example 96 but changing the compound of Reference Example 28 used in Example 96-(1) to the compound of Reference Example 29 and changing N-methylpropylamine used in Example 96-(3) to diethylamine, the compound of Example 100 was obtained.
¹HNMR (400 MHz, CDCl₃, δ ppm): 1.04 (6H, t, J=7.1 Hz), 2.52 (4H, q, J=7.1 Hz), 3.00 (2H, t, J=6.8 Hz), 3.53 (2H, s), 3.82 (3H, s), 4.07 (2H, t, J=6.8 Hz), 4.93 (2H, s), 5.72 (1H, dd, J=7.4, 2.9 Hz), 5.97 (1H, d, J=2.9 Hz), 6.73-6.79 (2H, m), 6.88 (1H, s), 6.93 (1H, d, J=7.4 Hz), 7.05-7.11 (2H, m), 7.34-7.40 (2H, m).

### Examples 101 to 104:

In the same manner as in Example 96-(1) and 96-(2) but changing the compound of Reference Example 2 used in Example 96 to the compound of Reference Example 9, subsequently in the same manner as in Example 14-(3) but using the obtained hydroxymethyl compound in place of 4-[(5-chloropyridin-2-yl)methoxy]-1- {2-[4-(hydroxymethyl)phenyl]ethyl}pyridin-2(1H)-one used in Example 14-(3), and then subsequently in the same manner as in Example 42 but using the obtained chloromethyl compound in place of the compound of Example 14-(3) used in Example 42 and further changing isopropylamine to corresponding amine compounds, the compounds of Examples 101 to 104 were obtained.

### Example 101:

### 4-[(E)-2-(4-Chlorophenyl)vinyl]-1-(2- {3-methoxy-4-[(propylamino)methyl]phenyl}ethyl)pyridin-2(1H)-one:

¹HNMR (400 MHz, CDCl₃, δ ppm): 0.92 (3H, t, J=7.3 Hz), 1.70 (2H, m), 2.69 (2H, t, J=7.8 Hz), 3.04 (2H, t, J=7.1 Hz), 3.79 (3H, s), 3.94 (2H, s), 4.11 (2H, t, J=7.1 Hz), 6.26 (1H, dd, J=7.3, 1.8 Hz), 6.57 (1H, d, J=1.8 Hz), 6.65 (1H, s), 6.75-6.80 (1H, m), 6.82 (1H, d, J=16.1 Hz), 6.88 (1H, d, J=6.8 Hz), 7.07 (1H, d, J=16.1 Hz), 7.25-7.30 (2H, m), 7.30-7.40 (2H, m), 7.42-7.47 (2H, m).

### Example 102:

### 4-[(E)-2-(4-Chlorophenyl)vinyl]-1-[2-(4-{[(cyclopropylmethyl)amino]methyl}-3-methoxyphenyl)ethyl]pyridin-2(1H)-one:

¹HNMR (400 MHz, CDCl₃, δ ppm): 0.18-0.25 (2H, m), 0.53-0.62 (2H, m), 1.12 (1H, dd, J=11.5, 4.6 Hz), 2.63 (2H, d, J=7.3 Hz), 3.04 (2H, t, J=7.1 Hz), 3.79 (3H, s), 3.99 (2H, s), 4.11 (2H, t, J=7.1 Hz), 6.26 (1H, dd, J=7.3, 1.8 Hz), 6.56 (1H, d, J=1.8 Hz), 6.65 (1H, d, J=1.0 Hz), 6.75-6.80 (1H, m), 6.82 (1H, d, J=16.1 Hz), 6.88 (1H, d, J=7.3 Hz), 7.07 (1H, d, J=16.1 Hz), 7.25-7.30 (2H, m), 7.30-7.40 (2H, m), 7.40-7.47 (2H, m).

### Example 103:

### 4-[(E)-2-(4-Chlorophenyl)vinyl]-1- {2-[3-methoxy-4-({[(1S)-1-methylpropyl)amino]methyl}phenyl]ethyl}pyridin-2(1H)-one:

¹HNMR (400 MHz, CDCl₃, δ ppm): 0.93 (3H, t, J=7.6 Hz), 1.36 (3H, d, J=6.8 Hz), 1.60-1.70 (1H, m), 1.85-2.00 (1H, m), 2.80-2.95 (1H, m), 3.01 (2H, t, J=7.1 Hz), 3.82 (3H, s), 4.00-4.09 (4H, m), 6.29 (1H, dd, J=7.3, 1.8 Hz), 6.55 (1H, d, J=1.8 Hz), 6.65-6.70 (1H, m), 6.75-6.85 (2H, m), 6.90 (1H, d, J=7.3 Hz), 7.08 (1H, d, J=16.1 Hz), 7.30-7.40 (2H, m), 7.40-7.45 (3H, m).

### Example 104:

### 4-[(E)-2-(4-Chlorophenyl)vinyl]-1-(2-{4-[(cyclopentylamino)methyl]-3-methoxyphenyl}ethyl)pyridin-2(1H)-one:

¹HNMR (400 MHz, CDCl₃, δ ppm): 1.40-2.00 (8H, m), 3.03 (2H, t, J=7.1 Hz), 3.15-3.25 (1H, m), 3.79 (3H, s), 3.92 (2H, s), 4.10 (2.2H, t, J=7.1 Hz), 6.27 (1H, dd, J=7.1, 1.8 Hz), 6.56 (1H, d, J=1.8 Hz), 6.63-6.67 (1H, s), 6.75-6.80 (1H, m), 6.82 (1H, d, J=16.1 Hz), 6.89 (1H, d, J=7.1 Hz), 7.07 (1H, d, J=16.1 Hz), 7.30 (1H, d, J=7.8 Hz), 7.30-7.40 (2H, m), 7.40-7.47 (2H, m). Examples 105 to 107:

In the same manner as in Example 55 but changing acetone used in Example 55 to corresponding carbonyl compounds and changing the compound of Example 54 to the compound of Example 43 or the compound of Example 44, obtained were the compounds of Examples 105 to 107.

### Example 105:

### 1-[2-(4-{[Tert-butyl(methyl)amino]methyl}phenyl)ethyl]-4-[(5-chloropyridin-2-yl)methoxy]pyridin-2(1H)-one:

¹HNMR (400 MHz, CDCl₃, δ ppm): 1.15 (9H, s), 2.06 (3H, s), 3.00 (2H, t, J=6.8 Hz), 3.46 (2H, s), 4.06 (2H, t, J=6.8 Hz), 5.09 (2H, s), 5.78-5.84 (1H, m), 5.94-5.98 (1H, m), 6.76-6.80 (1H, m), 7.04-7.10 (2H, m), 7.23-7.29 (2H, m), 7.40 (1H, d, J=7.8 Hz), 7.70-7.72 (1H, m), 8.55-8.58 (1H, m).

### Example 106:

### 4-[(5-Chloropyridin-2-yl)methoxy]-1-[2-(4-{[cyclopropyl(methyl)amino]methyl}phenyl)ethyl]pyridin-2(1H)-one:

¹HNMR (400 MHz, CDCl₃, δ ppm): 0.38-0.48 (4H, m), 1.65-1.70 (1H, m), 2.23 (3H, s), 3.00 (2H, t, J=7.1 Hz), 3.61 (2H, s), 4.07 (2H, t, J=7.1 Hz), 5.09 (2H, s), 5.80 (1H, dd, J=7.3, 2.9 Hz), 5.96 (1H, d, J=2.9 Hz), 6.74 (1H, d, J=7.3 Hz), 7.07 (2H, d, J=7.8 Hz), 7.18 (2H, d, J=7.8 Hz), 7.39 (1H, d, J=8.3 Hz), 7.71 (1H, dd, J=8.3, 2.4 Hz), 8.57 (1H, d, J=2.4 Hz).

### Example 107:

### 4-[(5-Chloropyridin-2-yl)methoxy]-1-[2-(4-{[cyclopropyl(propyl)amino]methyl}phenyl)ethyl]pyridin-2(1H)-one:

¹HNMR (400 MHz, CDCl₃, δ ppm): 0.35-0.44 (4H, m), 0.82 (3H, t, J=7.3 Hz), 1.49-1.56 (2H, m), 1.66-1.76 (1H, m), 2.40-2.50 (2H, m), 2.95-3.05 (2H, m), 3.68 (2H, s), 4.07 (2H, t, J=7.1 Hz), 5.09 (2H, s), 5.75-5.82 (1H, m), 5.96 (1H, d, J=2.9 Hz), 6.70-6.76 (1H, m), 7.00-7.10 (2H, m), 7.15-7.22 (2H, m), 7.39 (1H, d, J=8.3 Hz), 7.71 (1H, dd, J=8.3, 2.2 Hz), 8.57 (1H, d, J=2.2 Hz).

### Examples 108 and 109:

In the same manner as in Example 59 but changing 2-fluoroethyl trifluoromethanesulfonate used in Example 59 to iodoethane and changing the compound of Example 54 to the compound of Example 43 or the compound of Example 44, obtained were the compounds of Examples 108 and 109.

### Example 108:

### 1-[2-(4-{[Tert-butyl(ethyl)amino]methyl}phenyl)ethyl]-4-[(5-chloropyridin-2-yl)methoxy]pyridin-2(1H)-one:

¹HNMR (400 MHz, CDCl₃, δ ppm): 0.83 (3H, t, J=6.7 Hz), 1.11 (9H, s), 2.59 (2H, q, J=6.7 Hz), 2.99 (2H, t, J=7.0 Hz), 3.63 (2H, s), 4.07 (2H, t, J=7.0 Hz), 5.09 (2H, s), 5.77 (1H, dd, J=7.5, 2.8 Hz), 5.96 (1H, d, J=2.8 Hz), 6.72 (1H, d, J=7.5 Hz), 7.04 (2H, d, J=7.5 Hz), 7.29 (2H, d, J=7.5 Hz), 7.39 (1H, d, J=8.1 Hz), 7.71 (1H, dd, J=8.1, 2.4 Hz), 8.55-8.58 (1H, m).

### Example 109:

### 4-[(5-Chloropyridin-2-yl)methoxy]-1-[2-(4-{[cyclopropyl(ethyl)amino]methyl}phenyl)ethyl]pyridin-2(1H)-one:

¹HNMR (400 MHz, CDCl₃, δ ppm): 0.35-0.47 (4H, m), 1.07 (3H, t, J=7.3 Hz), 1.69-1.73 (1H, m), 2.57 (2H, q, J=7.3 Hz), 3.00 (2H, t, J=6.8 Hz), 3.70 (2H, s), 4.07 (2H, t, J=6.8 Hz), 5.09 (2H, s), 5.79 (1H, dd, J=7.7, 2.6 Hz), 5.96 (1H, d, J=2.6 Hz), 6.73 (1H, d, J=7.7 Hz), 7.06 (2H, d, J=8.1 Hz), 7.19 (2H, d, J=8.1 Hz), 7.39 (1H, d, J=8.3 Hz), 7.71 (1H, dd, J=8.3, 2.4 Hz), 8.57 (1H, d, J=2.4 Hz).

### Example 110:

### 1-(2-{4-[(Diethylamino)methyl]phenyl}-2-fluoroethyl)-4-[(4-fluorobenzyl)oxy]pyridin-2(1H)-one:

### (1) Production of 1-[2-(4-bromophenyl)-2-fluoroethyl]-4-[(4-fluorobenzyl)oxy]pyridin-2(1H)-one:

In the same manner as in Example 1 but changing the compound of Reference Example 16 used in Example 1 to the compound of Reference Example 32-(2), obtained was the compound of Example 110-(1).
Mass Spectrum (ESI): 420.0 (M+H).

### (2) Production of 4-[(4-fluorobenzyl)oxy]-1-{2-fluoro-2-[4-(hydroxymethyl)phenyl]ethyl}pyridin-2(1H)-one:

In the same manner as in Reference Example 19-(5) to 19-(6) but changing [2-(4-bromo-2-fluorophenyl)ethoxy](tert-butyl)dimethylsilane used in Reference Example 19-(5) to the compound obtained in the above (1), the compound of Example 110-(2) was obtained.
Mass Spectrum (ESI): 372.1 (M+H).

### (3) Production of 1-(2- {4-[(diethylamino)methyl]phenyl}-2-fluoroethyl)-4-[(4-fluorobenzyl)oxy]pyridin-2(1H)-one:

In the same manner as in Example 14-(3) and 14-(4) but changing the compound used in Example 14-(3) to the compound obtained in the above (2), the compound of Example 110 was obtained.
¹HNMR (400 MHz, CDCl₃, δ ppm): 1.04 (6H, t, J=7.2 Hz), 2.52 (4H, q, J=7.2 Hz), 3.58 (2H, s), 3.75 (1H, td, J=14.4, 9.1 Hz), 4.63 (1H, ddd, J=33.7, 14.4, 2.4 Hz), 4.97 (2H, s), 5.76 (1H, ddd, J=42.4, 9.1, 2.4 Hz), 5.95 (1H, dd, J=8.0, 2.6 Hz), 6.00 (1H, d, J=2.6 Hz), 7.05-7.15 (2H, m), 7.19-7.25 (1H, m), 7.34-7.40 (6H, m).

### Example 111:

### 4-[(4-Fluorobenzyl)oxy]-1-(2-fluoro-2-{4-[(propylamino)methyl]phenyl}ethyl)pyridin-2(1H)-one:

### (1) Production of 4-[(4-fluorobenzyl)oxy]-1-(2-fluoro-2-{4-[(methoxymethoxy)methyl]phenyl}ethyl)pyridin-2(1H)-one:

In the same manner as in Example 1 but changing the compound of Reference Example 16 used in Example 1 to the compound of Reference Example 32, obtained was the compound of Example 111-(1).
Mass Spectrum (ESI): 416.1 (M+H).

### (2) Production of 1-{2-[4-(chloromethyl)phenyl]-2-fluoroethyl}-4-[(4-fluorobenzyl)oxy]pyridin-2(1H)-one:

A 10 % hydrogen chloride/methanol solution (5 mL) of the compound obtained in the above (1) (47.0 mg, 0.11 mmol) was stirred overnight at room temperature. The reaction liquid was concentrated under reduced pressure, then with cooling with ice, aqueous saturated sodium hydrogencarbonate solution was added to the residue, and extracted with ethyl acetate. The obtained organic layer was washed with saturated saline, dried with anhydrous sodium sulfate, and filtered. The obtained filtrate was concentrated under reduced pressure to obtain a colorless solid residue. Next, in the same manner as in Example 14-(3) but changing the compound used in Example 14-(3) to the obtained residue, the compound of Example 111-(2) was obtained.
Mass Spectrum (ESI): 390.1 (M+H).

### (3) Production of 4-[(4-fluorobenzyl)oxy]-1-(2-fluoro-2-{4-[(propylamino)methyl]phenyl}ethyl)pyridin-2(1H)-one:

In the same manner as in Example 42 but changing the compound of Example 14-(3) used in Example 42 to the compound obtained in the above (2) and changing isopropylamine to propylamine, the compound of Example 111 was obtained.
¹HNMR (400 MHz, CDCl₃, δ ppm): 0.93 (3H, t, J=7.3 Hz), 1.50-1.60 (2H, m), 2.60 (2H, t, J=7.3 Hz), 3.74 (1H, td, J=14.4, 9.2 Hz), 3.82 (2H, s), 4.62 (1H, ddd, J=33.7, 14.4, 2.4 Hz), 4.97 (2H, s), 5.78 (1H, ddd, J=49.3, 9.2, 2.4 Hz), 5.96 (1H, dd, J=7.6, 3.4 Hz), 6.00 (1H, d, J=2.4 Hz), 7.07-7.11 (2H, m), 7.18-7.22 (1H, m), 7.34-7.42 (6H, m).

### Example 112:

### 1-(2-{4-[(Cyclopropylamino)methyl]phenyl}-2-fluoroethyl)-4-[(4-fluorobenzyl)oxy]pyridin-2(1H)one:

In the same manner as in Example 111 but changing propylamine used in Example 111-(3) to cyclopropylamine, the compound of Example 112 was obtained.
¹HNMR (400 MHz, CDCl₃, δ ppm): 0.35-0.47 (4H, m), 2.13-2.16 (1H, m), 3.74 (1H, td, J=14.4, 9.2 Hz), 3.86 (2H, s), 4.62 (1H, ddd, J=33.7, 14.4, 2.4 Hz), 4.97 (2H, s), 5.77 (1H, ddd, J=48.8, 9.2, 2.4 Hz), 5.96 (1H, dd, J=7.3, 2.4 Hz), 6.00 (1H, d, J=2.4 Hz), 7.07-7.11 (2H, m), 7.18-7.23 (1H, m), 7.35-7.40 (6H, m).

### Example 113:

### 1-{2-[4-(Azetidin-1-ylmethyl)phenyl]-2,2-difluoroethyl}-4-[(4-fluorobenzyl)oxy]pyridin-2(1H)-one:

### (1) Production of ethyl 4-{1,1-difluoro-2-[4-[(4-fluorobenzyl)oxy]-2-oxopyridin-1(2H)-yl]ethyl}benzoate:

In the same manner as in Example 14-(1) but changing the compound of Reference Example 21 used in Example 14-(1) to the compound of Reference Example 31 and changing the compound of Reference Example 3 to the compound of Reference Example 2, obtained was the compound of Example 113-(1).
Mass Spectrum (ESI): 432.1 (M+H).

### (2) Production of 1- {2-[4-(azetidin-1-ylmethyl)phenyl]-2,2-difluoroethyl}-4-[(4-fluorobenzyl)oxy]pyridin-2(1H)-one:

In the same manner as in Example 96-(2) and 96-(3) but changing the compound used in Example 96-(2) to the compound obtained in the above (1) and changing N-methylpropylamine used in Example 96-(3) to azetidine, the compound of Example 113 was obtained.
¹HNMR (400 MHz, CDCl₃,δ ppm): 2.09 (2H, qui, J=7.1 Hz), 3.22 (4H, t, J=7.1 Hz), 3.59 (2H, s), 4.52 (2H, t, J=14.1 Hz), 4.93 (2H, s), 5.90 (1H, d, J=2.8 Hz), 5.95 (1H, dd, J=7.6, 2.8 Hz), 7.09 (2H, t, J=8.5 Hz), 7.20-7.30 (1H, m), 7.30-7.40 (4H, m), 7.45 (2H, d, J=8.3 Hz).

### Example 114:

### 1-{[4-(2-Azetidin-1-ylethyl)benzyl]oxy}-4-[(4-fluorobenzyl)oxy]pyridin-2(1H)-one:

### (1) Production of methyl [4-({[4-[(4-fluorobenzyl)oxy]-2-oxopyridin-1(2H)-yl]oxy}methyl)phenyl]acetate:

In the same manner as in Example 14-(1) but changing the compound of Reference Example 3 used in Example 14-(1) to the compound of Reference Example 6 and changing the compound of Reference Example 21 to methyl [4-(bromomethyl)phenyl]acetate [Journal of Medicinal Chemistry (1989), 32(3), 709-15], the compound of Example 114-(1) was obtained. Mass Spectrum (ESI): 398.1 (M+H).

### (2) Production of 4-[(4-fluorobenzyl)oxy]-1-{[4-(2-hydroxyethyl)benzyl]oxy}pyridin-2(1H)-one:

In the same manner as in Reference Example 16-(2) but changing the compound used in Reference Example 16-(2) to the compound obtained in the above (1), the compound of Example 114-(2) was obtained.
Mass Spectrum (ESI): 370.1 (M+H).

### (3) Production of 2-[4-({[4-[(4-fluorobenzyl)oxy]-2-oxopyridin-1(2H)-yl]oxy}methyl)phenyl]ethyl methanesulfonate:

In the same manner as in Reference Example 20 but changing the compound used in Reference Example 20 to the compound obtained in the above (2), the compound of Example 114-(3) was obtained.
Mass Spectrum (ESI): 448.1 (M+H).

### (4) Production of 1-{[4-(2-azetidin-1-ylethyl)benzyl]oxy}-4-[(4-fluorobenzyl)oxy]pyridin-2(1H)-one:

In the same manner as in Example 14-(4) but changing the compound used in Example 14-(4) to the compound obtained in the above (3) and changing diethylamine to azetidine, the compound of Example 114 was obtained.
¹HNMR (400 MHz, CDCl₃, δ ppm): 2.09-2.18 (2H, m), 2.69-2.75 (4H, m), 3.23-3.35 (4H, m), 4.92 (2H, s), 5.21 (2H, s), 5.67 (1H, dd, J=7.8, 3.4 Hz), 6.05 (1H, d, J=3.4 Hz), 6.97 (1H, d, J=7.8 Hz), 7.08 (2H, t, J=8.8 Hz), 7.21 (2H, d, J=7.8 Hz), 7.31-7.38 (4H, m).

### Example 115:

### 4-[(4-Fluorobenzyl)oxy]-1-[{4-(2-pyrrolidin-1-ylethyl)benzyl]oxy}pyridin-2(1H)-one:

In the same manner as in Example 114 but changing azetidine used in Example 114-(4) to pyrrolidine, the compound of Example 115 was obtained.
¹HNMR (400 MHz, CDCl₃, δ ppm): 1.81-1.88 (4H, m), 2.51-2.93 (8H, m), 4.92 (2H, s), 5.21 (2H, s), 5.67 (1H, dd, J=8.0, 3.4 Hz), 6.05 (1H, d, J=3.4 Hz), 6.96 (1H, d, J=8.0 Hz), 7.08 (2H, t, J=8.8 Hz), 7.22 (2H, d, J=8.3 Hz), 7.31-7.38 (4H, m).

### Example 116:

### 4-[(4-Fluorobenzyl)oxy]-1-({4-[2-(methylamino)ethyl]benzyl}oxy)pyridin-2(1H)-one:

The compound of Example 114-(3) (49.0 mg, 0.11 mmol) was dissolved in NMP (1.0 ml), then methylamine (100 µl, 1.20 mmol) was added, and stirred at room temperature for 16 hours. Next, water was added to the reaction liquid, and extracted with ethyl acetate. the obtained organic layer was washed with water and saturated saline, then dried with anhydrous magnesium sulfate, and filtered. The obtained filtrated was concentrated under reduced pressure, then the residue was purified by high-performance liquid chromatography (YMC-Pack™ pro C-18, H₂O (0.1 % TFA):CH₃CN (0.1 % TFA) = 90:10 to 50:50) to obtain the entitled compound (6.9 mg, 16 %).
¹HNMR (400 MHz, CDCl₃, δ ppm): 2.45 (3H, s), 2.85-2.87 (4H, m), 4.92 (2H, s), 5.21 (2H, s), 5.69 (1H, dd, J=7.8, 3.2 Hz), 6.05 (1H, d, J=3.2 Hz), 7.00 (1H, d, J=7.8 Hz), 7.06-7.11 (2H, m), 7.21-7.24 (2H, m), 7.33-7.38 (4H, m).

### Examples 117 to 123:

In the same manner as in Example 116 but changing methylamine used in Example 116 to corresponding amine compounds, the compounds of Examples 117 to 123 were obtained.

### Example 117:

### 1-({4-[2-(Ethylamino)ethyl]benzyl}oxy)-4-[(4-fluorobenzyl)oxy]pyridin-2(1H)-one:

¹HNMR (400 MHz, CDCl₃, δ ppm): 1.11 (3H, t, J=7.2 Hz), 2.68 (2H, q, J=7.2 Hz), 2.82-2.92 (4H, m), 4.92 (2H, s), 5.21 (2H, s), 5.69 (1H, dd, J=7.8, 3.4 Hz), 6.06 (1H, d, J=3.4 Hz), 6.99 (1H, d, J=7.8 Hz), 7.08 (2H, t, J=8.5 Hz), 7.22 (2H, d, J=7.8 Hz), 7.33-7.38 (4H, m).

### Example 118:

### 1-({4-[2-(Cyclopropylamino)ethyl]benzyl}oxy)-4-[(4-fluorobenzyl)oxy]pyridin-2(1H)-one:

¹HNMR (400 MHz, CDCl₃,δ ppm): 0.32-0.36 (2H, m), 0.41-0.46 (2H, m), 2.09-2.15 (1H, m), 2.82 (2H, t, J=7.1 Hz), 2.96 (2H, t, J=7.1 Hz), 4.92 (2H, s), 5.21 (2H, s), 5.68 (1H, dd, J=7.8, 3.4 Hz), 6.06 (1H, d, J=3.4 Hz), 6.99 (1H, d, J=7.8 Hz), 7.08 (2H, t, J=8.8 Hz), 7.22 (2H, d, J=7.8 Hz), 7.32-7.38 (4H, m).

### Example 119:

### 4-[(4-Fluorobenzyl)oxy]-1-({4-[2-(propylamino)ethyl]benzyl}oxy)pyridin-2(1H)-one:

¹HNMR (400 MHz, CDCl₃, δ ppm): 0.89 (3H, t, J=7.6 Hz), 1.45-1.54 (3H, m), 2.60 (2H, t, J=7.3 Hz), 2.81-2.91 (4H, m), 4.92 (2H, s), 5.21 (2H, s), 5.68 (1H, dd, J=8.1, 2.9 Hz), 6.05 (1H, d, J=2.9 Hz), 6.98 (1H, d, J=7.8 Hz), 7.08 (2H, t, J=8.5 Hz), 7.22 (2H, d, J=7.8 Hz), 7.33-7.38 (4H, m).

### Example 120:

### 1-({4-[2-(Tert-butylamino)ethyl]benzyl}oxy)-4-[(4-fluorobenzyl)oxy]pyridin-2(1H)-one:

¹HNMR (400 MHz, CDCl₃, δ ppm): 1.14 (9H, brs), 2.85-2.90 (4H, m), 4.92 (2H, s), 5.21 (2H, s), 5.67 (1H, dd, J=7.8, 3.4 Hz), 6.05 (1H, d, J=3.4 Hz), 6.98 (1H, d, J=7.8 Hz), 7.08 (2H, t, J=8.8 Hz), 7.23 (2H, d, J=7.8 Hz), 7.33-7.37 (4H, m).

### Example 121:

### 4-[(4-Fluorobenzyl)oxy]-1-({4-[2-(isobutylamino)ethyl]benzyl}oxy)pyridin-2(1H)-one:

¹HNMR (400 MHz, CDCl₃, δ ppm): 0.87 (6H, d, J=6.8 Hz), 1.70-1.77 (1H, m), 2.44 (2H, d, J=6.8 Hz), 2.80-2.89 (4H, m), 4.92 (2H, s), 5.21 (2H, s), 5.67 (1H, dd, J=8.0, 3.2 Hz), 6.05 (1H, d, J=3.2 Hz), 6.97 (1H, d, J=8.0 Hz), 7.08 (2H, t, J=8.8 Hz), 7.22 (2H, d, J=7.8 Hz), 7.32-7.38 (4H, m).

### Example 122:

### 1-({4-[2-(sec-butylamino)ethyl]benzyl}oxy)-4-[(4-fluorobenzyl)oxy]pyridin-2(1H)-one:

¹HNMR (400 MHz, CDCl₃, δ ppm): 0.86 (3H, t, J=7.3 Hz), 1.08 (3H, d, J=6.3 Hz), 1.30-1.41 (2H, m), 2.60-2.68 (1H, m), 2.84-2.98 (4H, m), 4.92 (2H, s), 5.21 (2H, s), 5.67 (1H, dd, J=7.8, 3.4 Hz), 6.05 (1H, d, J=3.4 Hz), 6.97 (1H, d, J=7.8 Hz), 7.08 (2H, t, J=8.5 Hz), 7.23 (2H, d, J=8.3 Hz), 7.33-7.37 (4H, m).

### Example 123:

### 1-({4-[2-(cyclohexylamino)ethyl]benzyl}oxy)-4-[(4-fluorobenzyl)oxy]pyridin-2(1H)-one:

¹HNMR (400 MHz, CDCl₃, δ ppm): 1.02-1.32 (5H, m), 1.52-1.92 (5H, m), 2.42-2.53 (1H, m), 2.81-2.96 (4H, m), 4.92 (2H, s), 5.21 (2H, s), 5.67 (1H, dd, J=7.8, 3.4 Hz), 6.05 (1H, d, J=3.4 Hz), 6.97 (1H, d, J=7.8 Hz), 7.08 (2H, t, J=8.8 Hz), 7.22 (2H, d, J=7.8 Hz), 7.32-7.37 (4H, m).

### Example 124:

### 4-[(4-Fluorobenzyl)oxy]-1-({4-[(propylamino)methyl]benzyl}oxy)pyridin-2(1H)-one:

### (1) Production of 1-{[4-(chloromethyl)benzyl]oxy}-4-[(4-fluorobenzyl)oxy]pyridin-2(1H)-one:

In he same manner as in Examples 14-(1) to 14-(3) but changing the compound of Reference Example 3 used in Example 14-(1) to the compound of Reference Example 6 and changing the compound of Reference Example 21 to the compound of Reference Example 34, obtained was the compound of Example 124-(1).
Mass Spectrum (ESI): 374.0 (M+H).

### (2) Production of 4-[(4-fluorobenzyl)oxy]-1-({4-[(propylamino)methyl]benzyl}oxy)pyridin-2(1H)-one:

In the same manner as in Example 116 but changing the compound of Reference Example 114-(3) used in Example 116 to the compound obtained in the above (1) and changing methylamine to propylamine, the compound of Example 124 was obtained.
¹HNMR (400 MHz, CDCl₃,δ ppm): 0.92 (3H, t, J=7.3 Hz), 1.49-1.59 (2H, m), 2.59 (2H, t, J=7.3 Hz), 3.81 (2H, s), 4.92 (2H, s), 5.23 (2H, s), 5.66 (1H, dd, J=7.8, 2.9 Hz), 6.05 (1H, d, J=2.9 Hz), 6.96 (1H, d, J=7.8 Hz), 7.06-7.11 (2H, m), 7.31-7.39 (6H, m).

### Example 125:

### 1-({4-[(Cyclopropylamino)methyl]benzyl}oxy)-4-[(4-fluorobenzyl)oxy]pyridin-2(1H)-one:

In the same manner as in Example 124 but changing propylamine used in Example 124 to cyclopropylamine, the compound of Example 125 was obtained.
¹HNMR (400 MHz, CDCl₃, δ ppm): 0.37-0.47 (4H, m), 2.11-2.17 (1H, m), 3.86 (2H, s), 4.92 (2H, s), 5.23 (2H, s), 5.66 (1H, dd, J=7.8, 2.9 Hz), 6.05 (1H, d, J=2.9 Hz), 6.95 (1H, d, J=7.8 Hz), 7.06-7.11 (2H, m), 7.31-7.38 (6H, m).

### Example 126:

### 1-{[5-(2-Azetidin-1-ylethyl)pyridin-2-yl]methoxy}-4-[(4-fluorobenzyl)oxy]pyridin-2(1H)-one:

### (1) Production of 2-[6-({[4-[(4-fluorobenzyl)oxy]-2-oxopyridin-1(2H)-yl]oxy}methyl)pyridin-3-yl] ethyl methanesulfonate:

In the same manner as in Examples 114-(1) to 114-(3) but changing methyl [4-(bromomethyl)phenyl]acetate used in Example 114-(1) to the compound of Reference Example 33, obtained was the compound of Example 126-(1).

### (2) Production of 1- {[5-(2-azetidin-1-ylethyl)pyridin-2-yl]methoxy}-4-[(4-fluorobenzyl)oxy]pyridin-2(1H)-one:

In the same manner as in Example 114-(4) but changing the compound used in Example 114-(4) to the compound obtained in the above (1), the compound of Example 126 was obtained.
¹HNMR (400 MHz, CDCl₃, δ ppm): 2.04-2.11 (2H, m), 2.61-2.71 (4H, m), 3.15-3.23 (4H, m), 4.92 (2H, s), 5.32 (2H, s), 5.75 (1H, dd, J=7.8, 3.1 Hz), 6.04 (1H, d, J=3.1 Hz), 7.06-7.12 (2H, m), 7.24-7.28 (1H, m), 7.33-7.38 (2H, m), 7.44 (1H, d, J=8.2 Hz), 8.08-8.72 (1H, m), 8.47 (1H, d, J=2.3 Hz).

### Example 127:

### 4-[(4-Fluorobenzyl)oxy]-1-{[5-(2-pyrrolidin-1-ylethyl)pyridin-2-yl]methoxy}pyridin-2(1H)-one:

In the same manner as in Example 126 but changing azetidine used in Example 126-(2) to pyrrolidine, the compound of Example 127 was obtained.
¹HNMR (400 MHz, CDCl₃, δ ppm): 1.77-1.87 (4H, m), 2.53-2.89 (8H, m), 4.92 (2H, s), 5.32 (2H, s), 5.75 (1H, dd, J=7.8, 3.1 Hz), 6.05 (1H, d, J=3.1 Hz), 7.05-7.12 (2H, m), 7.23-7.29 (1H, m), 7.33-7.39 (2H, m), 7.44 (1H, d, J=7.8 Hz), 7.58 (1H, dd, J=7.8, 2.3 Hz), 8.50 (1H, d, J=2.3 Hz).

### Examples 128 and 129:

In the same manner as in Example 116 but changing the compound of Example 114-(3) used in Example 116 to the compound of Example 126-(1) and changing methylamine to corresponding amines, the compounds of Examples 128 and 129 were obtained.

### Example 128:

### 4-[(4-Fluorobenzyl)oxy]-1-({5-[2-(propylamino)ethyl]pyridin-2-yl}methoxy)pyridin-2(1H)-one:

¹HNMR (400 MHz, CDCl₃, δ ppm): 0.90 (3H, t, J=7.4 Hz), 1.46-1.57 (2H, m), 2.62 (2H, t, J=7.2 Hz), 2.83-2.94 (4H, m), 4.93 (2H, s), 5.32 (2H, s), 5.77 (1H, dd, J=7.8, 3.1 Hz), 6.05 (1H, d, J=3.1 Hz), 7.06-7.11 (2H, m), 7.26-7.29 (1H, m), 7.34-7.38 (2H, m), 7.46 (1H, d, J=8.2 Hz), 7.59 (1H, dd, J=8.2, 2.0 Hz), 8.51 (1H, d, J=2.0 Hz).

### Example 129:

### 1-({5-[2-(Cyclopropylamino)ethyl]pyridin-2-yl}methoxy)-4-[(4-fluorobenzyl)oxy]pyridin-2(1H)-one:

¹HNMR (400 MHz, CDCl₃, δ ppm): 0.31-0.36 (2H, m), 0.42-0.48 (2H, m), 2.11-2.16 (1H, m), 2.83 (2H, t, J=7.0 Hz), 2.98 (2H, t, J=7.0 Hz), 4.93 (2H, s), 5.33 (2H, s), 5.77 (1H, dd, J=7.8, 3 Hz), 6.05 (1H, d, J=3.1 Hz), 7.06-7.11 (2H, m), 7.26-7.28 (1H, m), 7.34-7.38 (2H, m), 7.46 (1H, d, J=8.2 Hz), 7.58 (1H, dd, J=8.2, 2.2 Hz), 8.50 (1H, d, J=2.2 Hz).

### Example 130:

### 4-[(4-Chlorobenzyl)oxy]-1-(2-{4-[(2-oxopyrrolidin-1-yl)methyl]phenyl}ethyl)pyridin-2(1H)-one:

### (1) Production of 4-[(4-chlorobenzyl)oxy]-1-{2-[4-(chloromethyl)phenyl]ethyl}pyridin-2(1H)-one:

In the same manner as in Examples 14-(1) to 14-(3) but changing the compound of Reference Example 3 used in Example 14-(1) to the compound of Reference Example 1, obtained was the compound of Example 130-(1).
Mass Spectrum (ESI): 388.1 (M+H).

### (2) Production of 4-[(4-chlorobenzyl)oxy]-1-(2- {4-[(2-oxopyrrolidin-1-yl)methyl]phenyl} ethyl)pyridin-2(1H)-one:

Sodium hydride (60 % oily) was added to a toluene solution or DMF solution of the compound obtained in the above (1) and pyrrolidin-2-one, and stirred overnight at 90°C or room temperature. Next, water was added to the reaction liquid, and extracted with ethyl acetate. The obtained organic layer was washed with water and saturated saline, then dried with anhydrous sodium sulfate, and filtered. The obtained filtrate was concentrated under reduced pressure, then the residue was purified by high-performance liquid chromatography (YMC-Pack^{™} pro C-18, H₂O (0.1% TFA):CH₃CN (0.1 % TFA) = 90:10 to 50:50) to obtain the entitled compound.
¹HNMR (400 MHz, CDCl₃, δ ppm): 1.98-2.05 (2H, m), 2.51 (2H, t, J=8.0 Hz), 3.04 (2H, t, J=7.1 Hz), 3.28 (2H, t, J=7.1 Hz), 4.13 (2H, t, J=7.1 Hz), 4.44 (2H, s), 5.01 (2H, s), 5.98 (1H, dd, J=7.6, 2.7 Hz), 6.41 (1H, d, J=2.4 Hz), 6.86 (1H, d, J=7.3 Hz), 7.08-7.18 (4H, m), 7.32-7.40 (4H, m).

### Example 131:

### 4-[(3,4-Difluorobenzyl)oxy]-1-[2-(4-[{methyl(propyl)amino]methyl}phenyl)ethyl]pyridin-2(1H)-one:

### (1) Production of methyl 4- {2-[4-[(4-fluorobenzyl)oxy]-2-oxopyridin-1(2H)-yl]ethyl}benzoate:

In the same manner as in Example 14-(1) but changing the compound of Reference Example 21 used in Example 14-(1) to methyl 4-(2-bromoethyl)benzoate and changing the compound of Reference Example 3 to the compound of Reference Example 2, obtained was the compound of Example 131-(1).
Mass Spectrum (ESI): 382.2 (M+H).

### (2) methyl 4-[2-(4-hydroxy-2-oxopyridin-1(2H)-yl)ethyl]benzoate:

Palladium (10 wt.% activated charcoal, 10 mg) was added to a methanol (1 mL)/THF (1 mL) mixed solution of the compound obtained in the above (1), and stirred at room temperature for 3 days in the presence of hydrogen. The reaction liquid was filtered, the obtained filtrate was concentrated under reduced pressure, and dried to obtain the entitled compound (53.8 mg, 77 %).
Mass Spectrum (ESI): 274.1 (M+H).

### (3) Production of methyl 4-{2-[4-[(3,4-difluorobenzyl)oxy]-2-oxopyridin-1(2H)-yl]ethyl}benzoate:

4-(bromomethyl)-1,2-difluorobenzene (0.12 mL, 0.90 mmol) and cesium carbonate (489 mg, 1.50 mmol) were added to an NMP solution (5 mL) of the compound obtained in the above (2) (204 mg, 0.75 mmol), and stirred at 80°C for 10 hours. Next, water was added to the reaction liquid, and extracted with ethyl acetate. The obtained organic layer was washed with aqueous saturated sodium hydrogencarbonate solution and saturated saline, dried with anhydrous sodium sulfate, and filtered. The obtained filtrate was concentrated under reduced pressure, then the residue was purified by silica gel column chromatography (chloroform:methanol = 10:0 to 97:3) to obtain the entitled compound (33 mg, 11 %).
Mass Spectrum (ESI): 400.1 (M+H).

### (4) Production of 4-[(3,4-difluorobenzyl)oxy]-1-[2-(4-[{methyl(propyl)amino]methyl}phenyl)ethyl]pyridin-2(1H)-one:

In the same manner as in Examples 96-(2) to 96-(3) but changing the compound used in Example 96-(2) to the compound obtained in the above (3), the compound of Example 131 was obtained.
¹HNMR (400 MHz, CDCl₃, δ ppm): 0.90 (4H, t, J=7.6 Hz), 1.48-1.70 (2H, m), 2.18 (3H, s), 2.28-2.38 (2H, m), 3.01 (2H, t, J=7.1 Hz), 3.46 (2H, s), 4.07 (2H, t, J=7.1 Hz), 4.93 (2H, s), 5.75 (1H, dd, J=7.6, 2.9 Hz), 5.94 (1H, d, J=2.9 Hz), 6.75 (1H, d, J=7.6 Hz), 7.09 (3H, d, J=7.8 Hz), 7.15-7.26 (4H, m).

### Examples 132 and 133:

In the same manner as in Example 42 but changing the compound of Example 14-(3) used in Example 42 to the compound of Example 113-(2) and changing isopropylamine to corresponding amines, the compounds of Examples 132 and 133 were obtained.

### Example 132:

### 1-(2,2-Difluoro-2-{4-[(propylamino)methyl]phenyl}ethyl)-4-[(4-fluorobenzyl)oxy]pyridin-2(1H)-one:

¹HNMR (400 MHz, CDCl₃, δ ppm): 0.93 (3H, t, J=7.3 Hz), 1.50-1.62 (2H, m), 2.62 (2H, t, J=7.3 Hz), 3.85 (2H, s), 4.53 (2H, t, J=14.1 Hz), 4.93 (2H, s), 5.90 (1H, d, J=2.6 Hz), 5.97 (1H, dd, J=7.6, 2.6 Hz), 7.09 (2H, t, J=8.8 Hz), 7.22-7.28 (1H, m), 7.34-7.48 (6H, m).

### Example 133:

### 1-(2-{4-[(cyclopropylamino)methyl]phenyl}-2,2-difluoroethyl)-4-[(4-fluorobenzyl)oxy]pyridin-2(1H)-one:

¹HNMR (400 MHz, CDCl₃, δ ppm): 0.34-0.48 (4H, m), 2.11-2.16 (1H, m), 3.87 (2H, s), 4.53 (2H, t, J=14.1 Hz), 4.92 (2H, s), 5.90 (1H, d, J=2.8 Hz), 5.96 (1H, dd, J=7.6, 2.8 Hz), 7.09 (2H, t, J=8.8 Hz), 7.22-7.30 (1H, m), 7.35-7.38 (4H, m), 7.46 (2H, d, J=8.3 Hz).

### Example 134:

### 4-[(4-Fluorobenzyl)oxy]-1-{[5-(pyrrolidin-1-ylmethyl)pyridin-2-yl]methoxy}pyridin-2(1H)-one:

In the same manner as in Example 114-(1) and 114-(2) but changing the compound of Reference Example 31 used in Example 113-(1) to ethyl 6-{[(methylsulfonyl)oxy]methyl}nicotinate [Journal of Medicinal Chemistry (2003), 46(11), 2216-2226] and changing the compound of Reference Example 2 to the compound of Reference Example 6; then subsequently in the same manner as in Example 14-(3) but using the obtained hydroxymethyl compound in place of the compound used in Example 14-(3); and further subsequently in the same manner as in Example 42 but using the obtained chloromethyl compound in place of the compound of Example 14-(3) used in Example 42 and changing isopropylamine to pyrrolidine, the compound of Example 134 was obtained.
¹HNMR (400 MHz, CDCl₃, δ ppm): 1.75-1.85 (4H, m), 2.45-2.55 (4H, m), 3.66 (2H, s), 4.92 (2H, s), 5.34 (2H, s), 5.75 (1H, dd, J=7.8, 3.2 Hz), 6.05 (1H, d, J=3.2 Hz), 7.06-7.11 (2H, m), 7.20-7.30 (1H, m), 7.34-7.37 (2H, m), 7.48 (1H, d, J=7.8 Hz), 7.70-7.77 (1H, m), 8.57 (1H, d, J=2.0 Hz).

### Examples 135 to 139:

In the same manner as in Example 134 but changing pyrrolidine used in Example 134 to corresponding amines, the compounds of Examples 135 to 139 were obtained.

### Example 135:

### 1-{[5-(Azetidin-1-ylmethyl)pyridin-2-yl]methoxy}-4-[(4-fluorobenzyl)oxy]pyridin-2(1H)-one:

¹HNMR (400 MHz, CDCl₃, δ ppm): 2.07-2.14 (2H, m), 3.23 (4H, t, J=7.1 Hz), 3.60 (2H, s), 4.92 (2H, s), 5.33 (2H, s), 5.74 (1H, dd, J=8.0, 3.3 Hz), 6.04 (1H, d, J=3.3 Hz), 7.05-7.12 (2H, m), 7.22-7.28 (1H, m), 7.32-7.40 (2H, m), 7.47 (1H, d, J=8.2 Hz), 7.66 (1H, dd, J=8.2, 1.9 Hz), 8.52 (1H, d, J=1.9 Hz).

### Example 136:

### 1-({5-[(ethylamino)methyl]pyridin-2-yl}methoxy)-4-[(4-fluorobenzyl)oxy]pyridin-2(1H)-one:

¹HNMR (400 MHz, CDCl₃, δ ppm): 1.16 (3H, t, J=7.3 Hz), 2.70 (2H, q, J=7.3 Hz), 3.85 (2H, s), 4.92 (2H, s), 5.34 (2H, s), 5.76 (1H, dd, J=8.0, 3.3 Hz), 6.05 (1H, d, J=3.3 Hz), 7.05-7.12 (2H, m), 7.23-7.28 (1H, m), 7.33-7.40 (2H, m), 7.49 (1H, d, J=7.9 Hz), 7.74 (1H, dd, J=7.9, 2.4 Hz), 8.58 (1H, d, J=2.4 Hz).

### Example 137:

### 4-[(4-Fluorobenzyl)oxy]-1-({5-[(propylamino)methyl]pyridin-2-yl}methoxy)pyridin-2(1H)-one:

¹HNMR (400 MHz, CDCl₃, δ ppm): 0.93 (3H, t, J=7.3 Hz), 1.51-1.61 (2H, m), 2.60 (2H, t, J=7.1 Hz), 3.83 (2H, s), 4.92 (2H, s), 5.34 (2H, s), 5.75 (1H, dd, J=7.8, 3.2 Hz), 6.05 (1H, d, J=3.2 Hz), 7.06-7.11 (2H, m), 7.22-7.30 (1H, m), 7.32-7.40 (2H, m), 7.49 (1H, d, J=8.1 Hz), 7.73 (1H, dd, J=8.1, 2.0 Hz), 8.58 (1H, d, J=2.0 Hz).

### Example 138:

### 1-[(5-{[(Cyclopropylmethyl)amino]methyl}pyridin-2-yl)methoxy]-4-[(4-fluorobenzyl)oxy]pyridin-2(1H)-one:

¹HNMR (400 MHz, CDCl₃, δ ppm): 0.10-0.15 (2H, m), 0.45-0.55 (2H, m), 0.92-1.02 (1H, m), 2.50 (2H, d, J=6.8 Hz), 3.86 (2H, s), 4.92 (2H, s), 5.34 (2H, s), 5.75 (1H, dd, J=7.8, 2.9 Hz), 6.04 (1H, d, J=2.9 Hz), 7.04-7.12 (2H, m), 7.22-7.28 (1H, m), 7.32-7.40 (2H, m), 7.49 (1H, d, J=7.8 Hz), 7.74 (1H, dd, J=7.8, 2.0 Hz), 8.58 (1H, d, J=2.0 Hz).

### Example 139:

### 4-[(4-Fluorobenzyl)oxy]-1-({5-[(isobutylamino)methyl]pyridin-2-yl}methoxy)pyridin-2(1H)-one:

¹HNMR (400 MHz, CDCl₃, δ ppm): 0.92 (6H, d, J=6.8 Hz), 1.75-1.81 (1H, m), 2.44 (2H, d, J=6.8 Hz), 3.84 (2H, s), 4.92 (2H, s), 5.34 (2H, s), 5.76 (1H, dd, J=8.0, 3.1 Hz), 6.05 (1H, d, J=3.1 Hz), 7.06-7.11 (2H, m), 7.22-7.30 (1H, m), 7.32-7.40 (2H, m), 7.49 (1H, d, J=7.7 Hz), 7.75 (1H, dd, J=7.7, 1.8 Hz), 8.59 (1H, d, J=1.8 Hz).

### Example 140:

### 4-[(4-Fluorobenzyl)oxy]-1-{[6-(pyrrolidin-1-ylmethyl)pyridin-3-yl]methoxy}pyridin-2(1H)-one:

In the same manner as in Reference Example 20 but changing the compound used in Reference Example 20 to [6-({[tert-butyl(dimethyl)silyl]oxy}methyl)pyridin-3-yl]methanol, obtained was [6-({[tert-butyl(dimethyl)silyl]oxy}methyl)pyridin-3-yl]methyl methanesulfonate. Subsequently, in the same manner as in Example 124-(1) but changing the compound of Reference Example 34 used in Example 124-(1) to the obtained [6-({[tert-butyl(dimethyl)silyl]oxy}methyl)pyridin-3-yl]methyl methanesulfonate, obtained was 1-{[6-(chloromethyl)pyridin-3-yl]methoxy}-4-[(4-fluorobenzyl)oxy]pyridin-2(1H)-one. Further, in the same manner as in Example 42 but changing the compound of Example 14-(3) used in Example 42 to the obtained 1-{[6-(chloromethyl)pyridin-3-yl]methoxy}-4-[(4-fluorobenzyl)oxy]pyridin-2(1H)-one and changing isopropylamine to pyrrolidine, obtained was the compound of Example 140.
¹HNMR (400 MHz, CDCl₃, δ ppm): 1.80-1.87 (4H, m), 2.56-2.70 (4H, m), 3.84 (2H, s), 4.92 (2H, s), 5.27 (2H, s), 5.73 (1H, dd, J=7.8, 3.2 Hz), 6.05 (1H, d, J=3.2 Hz), 7.02-7.12 (3H, m), 7.32-7.40 (2H, m), 7.45-7.55 (1H, m), 7.79 (1H, dd, J=8.0, 1.9 Hz), 8.56 (1H, d, J=1.9 Hz). Examples 141 to 145:

In the same manner as in Example 140 but changing pyrrolidine used in Example 140 to corresponding amines, the compounds of Examples 141 to 145 were obtained.

### Example 141:

### 1- {[6-(Azetidin-1-ylmethyl)pyridin-3-yl]methoxy}-4-[(4-fluorobenzyl)oxy]pyridin-2(1H)-one:

¹HNMR (400 MHz, CDCl₃, δ ppm): 2.16 (2H, t, J=7.1 Hz), 3.37 (4H, t, J=7.1 Hz), 3.79 (2H, s), 4.92 (2H, s), 5.26 (2H, s), 5.73 (1H, dd, J=7.8, 3.4 Hz), 6.05 (1H, d, J=3.4 Hz), 7.03-7.11 (3H, m), 7.32-7.42 (3H, m), 7.78 (1H, dd, J=7.8, 2.0 Hz), 8.54 (1H, d, J=2.0 Hz).

### Example 142:

### 1-({6-[(Ethylamino)methyl]pyridin-3-yl}methoxy)-4-[(4-fluorobenzyl)oxy]pyridin-2(1H)-one:

¹HNMR (400 MHz, CDCl₃, δ ppm): 1.20 (3H, t, J=7.2 Hz), 2.76 (2H, q, J=7.2 Hz), 3.99 (2H, s), 4.93 (2H, s), 5.27 (2H, s), 5.74 (1H, dd, J=7.8, 3.2 Hz), 6.05 (1H, d, J=3.2 Hz), 7.05-7.11 (3H, m), 7.34-7.40 (3H, m), 7.79 (1H, dd, J=8.0, 2.1 Hz), 8.56 (1H, d, J=2.1 Hz).

### Example 143:

### 4-[(4-Fluorobenzyl)oxy]-1-({6-[(propylamino)methyl]pyridin-3-yl}methoxy)pyridin-2(1H)-one:

¹HNMR (400 MHz, CDCl₃, δ ppm): 0.95 (3H, t, J=7.3 Hz), 1.55-1.65 (2H, m), 2.67 (2H, t, J=7.3 Hz), 3.98 (2H, s), 4.93 (2H, s), 5.27 (2H, s), 5.74 (1H, dd, J=8.0, 3.3 Hz), 6.05 (1H, d, J=3.3 Hz), 7.05-7.15 (3H, m), 7.33-7.40 (3H, m), 7.79 (1H, dd, J=7.8, 2.2 Hz), 8.56 (1H, d, J=2.2 Hz).

### Example 144:

### 1-[(6-[{(Cyclopropylmethyl)amino]methyl}pyridin-3-yl)methoxy]-4-[(4-fluorobenzyl)oxy]pyridin-2(1H)-one:

¹HNMR (400 MHz, CDCl₃,δ ppm): 0.10-0.20 (2H, m), 0.45-0.55 (2H, m), 0.96-1.06 (1H, m), 2.56 (2H, d, J=6.8 Hz), 4.00 (2H, s), 4.93 (2H, s), 5.27 (2H, s), 5.74 (1H, dd, J=7.8, 2.9 Hz), 6.05 (1H, d, J=2.9 Hz), 7.04-7.11 (3H, m), 7.34-7.40 (3H, m), 7.79 (1H, dd, J=8.0, 2.2 Hz), 8.56 (1H, d, J=2.2 Hz).

### Example 145:

### 4-[(4-Fluorobenzyl)oxy]-1-({6-[(isobutylamino)methyl]pyridin-3-yl}methoxy)pyridin-2(1H)-one:

¹HNMR (400 MHz, CDCl₃, δ ppm): 0.96 (6H, d, J=6.8 Hz), 1.80-1.92 (1H, m), 2.53 (2H, d, J=6.8 Hz), 4.01 (2H, s), 4.93 (2H, s), 5.27 (2H, s), 5.75 (1H, dd, J=8.0, 3.1 Hz), 6.06 (1H, d, J=3.1 Hz), 7.06-7.11 (3H, m), 7.32-7.42 (3H, m), 7.81 (1H, dd, J=8.0, 2.1 Hz), 8.57 (1H, d, J=2.1 Hz).

### INDUSTRIAL APPLICABILITY

The compounds of the invention have an MCH-1R antagonistic effect and are useful, for example, as a preventive or a remedy for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and digestive disorders, respiratory disorders cancer or pigmentation et al.

## Claims

1. A pyridone compound of a formula [I] or a pharmaceutically-acceptable salt thereof: 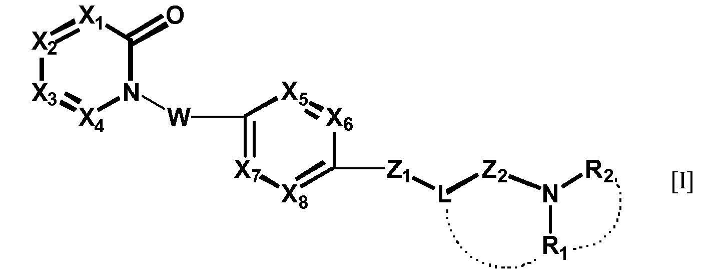 [wherein:
R₁ and R₂ are the same or different, each representing a hydrogen atom, a lower alkyl group optionally having substituent(s), or a lower cycloalkyl group optionally having substituent(s), and R₁ and R₂, taken together with the nitrogen atom to which they bond, may form a 4- to 11-membered, crosslinked, non-crosslinked or spiro-cyclic aliphatic nitrogen-containing hetero ring optionally having substituent(s);
X₁ and X₂ each represent a methine optionally having substituent(s) selected from a group consisting of a group α, a methine substituted with Ar-Y₁-Y₂-Y₃-, or a nitrogen atom, and any one of X₁ and X₂ is a methine substituted with Ar-Y₁-Y₂-Y₃-, and the other is a methine optionally having substituent(s) selected from the group consisting of the group α or a nitrogen atom;
substituent selected from a group consisting of a group α: a halogen atom, a lower alkyl group optionally substituted with halogen atom(s), and a lower alkyloxy group optionally substituted with halogen atom(s);
X₃ and X₄ are the same or different, each representing a methine optionally having substituent(s) or a nitrogen atom;
X₅, X₆, X₇ and X₈ are the same or different, each representing a methine optionally having substituent(s) or a nitrogen atom, however, at least 3 atoms of X₅, X₆, X₇ and X₈ are not nitrogen atoms at the same time;
Y₁ represents a single bond, -O-, -NR-, -S-, -SO- or -SO₂-;
Y₂ represents a single bond, a lower alkylene group optionally having substituent(s), a lower alkenylene group optionally having substituent(s), or a lower cycloalkylene group optionally having substituent(s);
Y₃ represents a single bond, -O-, -NR-, -S-, -SO- or -SO₂-;
R represents a hydrogen atom, or a lower alkyl group optionally having substituent(s);
W represents -(O)m₁-(CH₂)n-(O)m₂-, in which -(CH₂)n- may optionally have substituent(s);
m₁ and m₂ are the same or different, each indicating 0 or 1; n indicates an integer of from 1 to 4; and they satisfy 2 ≤ m₁+m₂+n ≤ 4, but m₁, m₂ and n are not 1 at the same time;
L represents a single bond or a methylene group optionally having substituent(s), and L, taken together with Z₂ and R₁ and with the nitrogen atom adjacent to R₁, may form an aliphatic nitrogen-containing hetero ring optionally having substituent(s);
Z₁ represents a single bond, -O-, or a C₁₋₄ alkylene group optionally having substituent(s);
Z₂ represents a single bond or a C₁₋₄ alkylene group optionally having substituent(s);
however, Y₁, Y₂, Y₃, Z₁, L and Z₂ are not all single bonds at the same time;
Ar represents an aromatic carbocyclic group optionally having substituent(s), an aromatic heterocyclic group optionally having substituent(s), or an aliphatic carbocyclic group optionally having substituent(s)].

2. The compound or the pharmaceutically-acceptable salt thereof as claimed in claim 1, wherein X₁ is an unsubstituted methine and X₂ is a methine substituted with Ar-Y₁-Y₂-Y₃-.

3. The compound or the pharmaceutically-acceptable salt thereof as claimed in claim 1, wherein X₁ is a methine substituted with Ar-Y₁-Y₂-Y₃-, and X₂ is an unsubstituted methine.

4. The compound or the pharmaceutically-acceptable salt thereof as claimed in any of claims 1 to 3, wherein X₃ and X₄ are both methines optionally having substituent(s), or any one of these is a nitrogen atom and the other is an unsubstituted methine.

5. The compound or the pharmaceutically-acceptable salt thereof as claimed in any of claims 1 to 4, wherein X₅, X₆, X₇ and X₈ are all methines optionally having substituent(s).

6. The compound or the pharmaceutically-acceptable salt thereof as claimed in any of claims 1 to 5, wherein Y₁ is a single bond.

7. The compound or the pharmaceutically-acceptable salt thereof as claimed in any of claims 1 to 6, wherein Y₂ is a single bond, a methylene group optionally having substituent(s), a vinylene group optionally having substituent(s), or an ethylene group optionally having substituent(s).

8. The compound or the pharmaceutically-acceptable salt thereof as claimed in any of claims 1 to 7, wherein Y₃ is a single bond or -O-.

9. The compound or the pharmaceutically-acceptable salt thereof as claimed in any of claims 1 to 8, wherein m1=1, n=1 and m2=0 in W.

10. The compound or the pharmaceutically-acceptable salt thereof as claimed in any of claims 1 to 8, wherein m1=0, n=2 and m2=0 in W.

11. The compound or the pharmaceutically-acceptable salt thereof as claimed in any of claims 1 to 10, wherein Z₁ is a single bond, a methylene group optionally having substituent(s), or -O-.

12. The compound or the pharmaceutically-acceptable salt thereof as claimed in claims 1 to 11, wherein L is a single bond, or a methylene group optionally having substituent(s).

13. The compound or the pharmaceutically-acceptable salt thereof as claimed in claims 1 to 12, wherein Z₂ is a single bond or a methylene group optionally having substituent(s).

14. The compound or the pharmaceutically-acceptable salt thereof as claimed in claims 1 to 11, wherein L, taken together with Z₂ and R₁ and with the nitrogen atom adjacent to R₁, forms a pyrrolidine ring optionally having substituent(s).

15. The compound or the pharmaceutically-acceptable salt thereof as claimed in any of claims 1 to 13, wherein R₁ and R₂ are the same or different, each representing a hydrogen, a C₁₋₄ alkyl group optionally having substituent(s), or a C₃₋₆ cycloalkyl group optionally having substituent(s).

16. The compound or the pharmaceutically-acceptable salt thereof as claimed in any of claims 1 to 13, wherein R₁ and R₂, taken together with the nitrogen atom to which they bond, form an azetidine ring optionally having substituent(s), a pyrrolidine ring optionally having substituent(s), or a piperidine ring optionally having substituent(s).

17. The compound or the pharmaceutically-acceptable salt thereof as claimed in any of claims 1 to 16, wherein Ar is a phenyl group optionally having substituent(s), or a pyridinyl group optionally having substituent(s).

18. The compound or the pharmaceutically-acceptable salt thereof as claimed in claim 17, wherein the substituent(s) in Ar is selected from a group consisting of a fluorine atom, a chlorine atom, a methyl group, an ethyl group, a trifluoromethyl group, a difluoromethyl group and a trifluoromethoxy group.

19. The compound or the pharmaceutically-acceptable salt thereof as claimed in claim 1, wherein the compound of formula [I] is selected from a group consisting of:
1) 4- [(5 -chloropyridin-2-yl)methoxy]-1-(2-{4-[(diethylamino)methyl]phenyl}ethyl)pyridin-2(1H)-one,
2) 1- {2-[4-(azetidin-1-ylmethyl)-3-fluorophenyl]ethyl}-4- [(4-fluorobenzyl)oxy]pyridin-2(1H)-one,
3) 4- [(5 -chloropyridin-2-yl)methoxy]-1-(2-{4-[(cyclopropylamino)methyl]phenyl}ethyl)pyridin-2(1H)-one,
4) 4-[(5-chloropyridin-2-yl)methoxy]-1-{2-[4-(1-pyrrolidin-1-ylethyl)phenyl]ethyl}pyridin-2(1H)-one,
5) 1- {2-[4-(1-azetidin-1-ylethyl)phenyl]ethyl}-4- [(5-chloropyridin-2-yl)methoxy]pyridin-2(1H)-one,
6) 1-{2-[4-(azetidin-1-ylmethyl)phenyl]ethyl}-4-[(5-chloropyridin-2-yl)methoxy]pyridin-2(1H)-one,
7) 4-[(5-chloropyridin-2-yl)methoxy]-1-{2-[4-(pyrrolidin-1-ylmethyl)phenyl]ethyl}pyridin-2(1H)-one,
8) 4- [(4-fluorobenzyl)oxy] -1 - {2-[5-(pyrrolidin-1-ylmethyl)pyridin-2-yl]ethyl}pyridin-2(1H)-one,
9) 1- {2-[5-(azetidin-1-ylmethyl)pyridin-2-yl]ethyl}-4- [(4-fluorobenzyl)oxy]pyridin-2(1H)-one,
10) 1- {2-[5-(azetidin-1-ylmethyl)pyridin-2-yl]ethyl}-4- [(4-chlorobenzyl)oxy]pyridin-2(1H)-one,
11) 4-[(4-fluorobenzyl)oxy]-1-(2-fluoro-2-{4-[(propylamino)methyl]phenyl}ethyl)pyridin-2(1H)-one,
12) 4-[(5-chloropyridin-2-yl)methoxy]-1-(2-{4-[(propylamino)methyl]phenyl}ethyl)pyridin-2(1H)-one, and
13) 1-(2,2-difluoro-2-{4-[(propylamino)methyl]phenyl}ethyl)-4-[(4-fluorobenzyl)oxy]pyridin-2(1H)-one.

20. The compound or the pharmaceutically-acceptable salt thereof as claimed in claim 1, wherein the compound of formula [I] is 4-[(5-chloropyridin-2-yl)methoxy]-1-(2- {4-[(diethylamino)methyl]phenyl}ethyl)pyridin-2(1H)-one.

21. The compound or the pharmaceutically-acceptable salt thereof as claimed in claim 1, wherein the compound of formula [I] is 1-{2-[4-(1-azetidin-1-ylethyl)phenyl]ethyl}-4-[(5-chloropyridin-2-yl)methoxy]pyridin-2(1H)-one.

22. The compound or the pharmaceutically-acceptable salt thereof as claimed in claim 1, wherein the compound of formula [I] is 1- {2-[4-(azetidin-1-ylmethyl)phenyl]ethyl}-4-[(5-chloropyridin-2-yl)methoxy]pyridin-2(1H)-one.

23. The compound or the pharmaceutically-acceptable salt thereof as claimed in claim 1, wherein the compound of formula [I] is 1-{2-[5-(azetidin-l-ylmethyl)pyridin-2-yl]ethyl}-4-[(4-fluorobenzyl)oxy]pyridin-2(1H)-one.

24. The compound or the pharmaceutically-acceptable salt thereof as claimed in claim 1, wherein the compound of formula [I] is 1-{2-[5-(azetidin-1-ylmethyl)pyridin-2-yl]ethyl}-4-[(4-chlorobenzyl)oxy]pyridin-2(1H)-one.

25. A melanin concentrating hormone receptor antagonist comprising a compound of any of claims 1 to 24 or a pharmaceutically-acceptable salt thereof as the active ingredient.

26. A pharmaceutical composition comprising a pharmaceutically-acceptable carrier and a compound of any of claims 1 to 24 or a pharmaceutically-acceptable salt thereof.

27. A preventive, treating or remedial agent comprising a compound of any of claims 1 to 24 or a pharmaceutically-acceptable salt thereof as the active ingredient, for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; digestive disorders; respiratory disorders; cancer or pigmentation.
